# EUROPEAN PATENT SPECIFICATION

(11) **EP 3 417 076 B1**
(45) Date of publication and mention of the grant of the patent: **09.11.2022**
(21) Application number: 17753596.0
(22) Date of filing: 16.02.2017
(51) Int. Cl.: C12Q 1/68

(54) **CANCER EPIGENETIC PROFILING**
EPIGENETISCHE KREBSPROFILIERUNG
ÉTABLISSEMENT DE PROFIL ÉPIGÉNÉTIQUE DE CANCER

(30) Priority: 16.02.2016 SG 10201601141X; 16.08.2016 SG 10201606828P
(43) Date of publication of application: 26.12.2018
(73) Proprietor: Agency for Science, Technology and Research, Singapore 138632 (SG)
(72) Inventor: TAN, Patrick, Singapore 138672 (SG); OOI, Wen Fong, Singapore 138672 (SG)
(74) Representative: Mewburn Ellis LLP
(86) International application number: PCT/SG2017/050073
(87) International publication number: WO 2017/142485

(56) References cited:
- WO-A1-2014/066848
- WO-A2-2011/031474
- WO-A2-2017/011710
- MANSOUR MARC R ET AL: "Oncogene regulation. An oncogenic super-enhancer formed through somatic mutation of a noncoding intergenic element.", SCIENCE (NEW YORK, N.Y.) 12 DEC 2014, vol. 346, no. 6215, 12 December 2014 (2014-12-12), pages 1373-1377, XP002794197, ISSN: 1095-9203
- NICHOLAS KWIATKOWSKI ET AL: "Targeting transcription regulation in cancer with a covalent CDK7 inhibitor", NATURE, vol. 511, no. 7511, 22 June 2014 (2014-06-22), pages 616-620, XP055193048, ISSN: 0028-0836, DOI: 10.1038/nature13393
- WHYTE WARREN A ET AL: "Master Transcription Factors and Mediator Establish Super-Enhancers at Key Cell Identity Genes", CELL, ELSEVIER, AMSTERDAM, NL, vol. 153, no. 2, 11 April 2013 (2013-04-11), pages 307-319, XP028547941, ISSN: 0092-8674, DOI: 10.1016/J.CELL.2013.03.035
- M. P. CREYGHTON ET AL: "From the Cover: Histone H3K27ac separates active from poised enhancers and predicts developmental state", PROCEEDINGS OF THE NATIONAL ACADEMY OF SCIENCES, vol. 107, no. 50, 14 December 2010 (2010-12-14), pages 21931-21936, XP055074651, ISSN: 0027-8424, DOI: 10.1073/pnas.1016071107
- AZIZ KHAN ET AL: "dbSUPER: a database of super-enhancers in mouse and human genome", NUCLEIC ACIDS RESEARCH, vol. 44, no. D1, 4 October 2015 (2015-10-04), pages D164-D171, XP055265583, ISSN: 0305-1048, DOI: 10.1093/nar/gkv1002
- HNISZ D. ET AL.: 'Super-Enhancers in the Control of Cell Identity and Disease.' CELL vol. 155, no. 4, 10 October 2013, pages 934 - 947, XP055410112
- CHANG H. R. ET AL.: 'HNF4a is a therapeutic target that links AMPK to WNT signalling in early-stage gastric cancer . Gut' MATERIALS AND METHODS vol. 65, no. 1, 19 November 2014, pages 19 - 32, XP055410131
- NIEDERRITER A. R. ET AL.: 'Super Enhancers in Cancers, Complex Disease, and Developmental Disorders' GENES vol. 6, no. 4, 09 November 2015, pages 1183 - 1200, XP055410120
- OOI W. F. ET AL.: 'Epigenomic profiling of primary gastric adenocarcinoma reveals super-enhancer heterogeneity' NAT COMMUN vol. 7, no. 12983, 28 September 2016, pages 1 - 17, XP055410124
- CHANG H. ET AL.: 'Synergistic action of master transcription factors controls epithelial-to-mesenchymal transition' NUCLEIC ACIDS RES vol. 44, no. 6, 28 February 2016, pages 2514 - 2527, XP055410127

## Description

### FIELD OF THE INVENTION

The invention relates to cancer, in particular, regulatory elements in cancer.

### BACKGROUND OF THE INVENTION

Aberrant gene expression patterns are a universal hallmark of human malignancy, driving clinically important traits such as proliferation, invasion and metastasis. DNA sequence-based alterations including somatic mutations, copy number alterations, and structural variations, have the capacity to reprogram cancer transcriptomes by altering the activity and expression of signaling molecules and transcription factors (TFs). Besides protein-coding genes, *cis*-regulatory elements in noncoding genomic regions such as enhancers can also influence transcriptional programs by facilitating or restricting TF accessibility.

Enhancers are regulatory elements localized distal to promoters and transcription start sites (TSSs). Occupying 10-15% of the human genome, enhancers have been shown to play important roles in cell identity and tissue-specific expression by regulating one or more genes at large distances (>1 Mb). Enhancers play an important role in human disease and their importance raises a need for catalogues of enhancers in different cell types and disease conditions. Whilst there have been studies to profile the regulatory elements in cancer, most of these studies to date have relied on *in vitro* cultured cancer cell lines, which have two limitations. First, *in vitro* cell lines are known to experience substantial epigenomic alterations after repeated passaging. Second, for many cancer cell lines, matched normal counterparts are frequently not available, complicating the ability to identify true somatic alterations. Accordingly, there is a need for a method of profiling regulatory elements in cancer that overcomes, or at least ameliorates, one or more of the disadvantages described above.

Mansour, M. et. Al., Science (2014), 346(6215): 1373-1377 discloses that the expression of critical oncogenes is driven from large regulatory elements, called super-enhancers, which recruit the cells transcriptional apparatus and are defined by extensive acetylation of histone H3 lysine 27 (H3K27ac).

Kwiatkowski, N. et. al., Nature (2014), 511(7511):616-620 discloses correlation between increasing number of enhancers found and H3K27ac signal increase.

Whyte, W. et. al., Cell (2013), 153(2):307-319 details the comparison between different histone modifications to distinguish super-enhancers from typical enhancers.

WO 2014/066848 A1 discloses super-enhancers identified in several cancer cells based on the H3K27Ac signal in a chromatin immunoprecipitation (ChIP-seq) method.

Creyghton, M. et. al., PNAS (2010), 107(50):21931-21936 teaches that H3K27ac modification is a mark that can distinguish active from inactive enhancer regions.

WO 2017/011710 A2 discloses identification of super-enhancers in regions enriched for H3K27ac signal.

Khan, A. and Zhang X., Nucleic Acids Res (2016), 44(D1): 164-171 discloses a database of super-enhancers in the mouse and human genome.

### SUMMARY

The invention herein is as described in the claims.

In one aspect, there is provided a method for determining the presence or absence of at least one super-enhancer in a cancerous biological sample relative to a non-cancerous biological sample, comprising;
a) contacting a cancerous biological sample obtained from the subject with at least one antibody specific for histone modification H3K27ac;
b) isolating nucleic acid from the cancerous biological sample, wherein the isolated nucleic acid comprises at least one region specific to the histone modification H3K27ac;
c) mapping at least one enhancer using an annotated genome sequence based on a signal intensity of the histone modification H3K27ac, wherein the at least one enhancer is at least 2.5 kb from an annotated transcription start site;
d) mapping the at least one enhancer in the isolated nucleic acid against at least one enhancer in at least one reference nucleic acid sequence to identify at least one super-enhancer in the cancerous biological sample;
e) comparing the signal intensity of the at least one super-enhancer in the cancerous biological sample against a reference signal intensity of the at least one super-enhancer obtained from a non-cancerous biological sample; and
f) determining the presence or absence of the at least one super-enhancer in the cancerous biological sample based on the change in signal intensity of the at least one super-enhancer.

In one aspect, there is provided a method for determining the presence of at least one cancer-associated super-enhancer in a subject, comprising:
a) contacting a cancerous biological sample obtained from the subject with at least one antibody specific for histone modification H3K27ac;
b) isolating nucleic acid from the cancerous biological sample, wherein the isolated nucleic acid comprises at least one region specific to the histone modification H3K27ac;
c) mapping at least one enhancer using an annotated genome sequence based on a signal intensity of the histone modification H3K27ac, wherein the at least one enhancer is at least 2.5 kb from an annotated transcription start site;
d) mapping the at least one enhancer in the isolated nucleic acid against at least one enhancer in at least one reference nucleic acid sequence to identify at least one super-enhancer in the cancerous biological sample;
e) comparing the signal intensity of the at least one super-enhancer in the cancerous biological sample against a reference signal intensity of the at least one super-enhancer obtained from a non-cancerous biological sample; and
f) determining the presence of at least one cancer-associated super-enhancer in a subject based on the change in signal intensity of the at least one super-enhancer,
wherein an increased signal intensity of the at least one super-enhancer in the cancerous biological sample relative to the non-cancerous biological sample is indicative of the presence of at least one cancer-associated super-enhancer.

In one aspect, there is provided a biomarker for detecting cancer in a subject, the biomarker comprising at least one super-enhancer having increased signal intensity of H3K27ac in a cancerous biological sample relative to a normal non-cancerous biological sample, or at least one super-enhancer associated with an increase in cancer-associated transcription factor binding sites relative to unaltered super-enhancers, or both.

In one aspect, there is provided a method for determining the prognosis of cancer in a subject, comprising:
a) contacting a cancerous biological sample obtained from the subject with at least one antibody specific for histone modification H3K27ac;
b) isolating nucleic acid from the cancerous biological sample, wherein the isolated nucleic acid comprises at least one region specific to the histone modification H3K27ac;
c) mapping at least one enhancer using an annotated genome sequence based on a signal of the histone modification H3K27ac, wherein the at least one enhancer is at least 2.5 kb from an annotated transcription start site;
d) mapping the at least one enhancer in the isolated nucleic acid against at least one enhancer in at least one reference nucleic acid sequence to identify at least one super-enhancer in the cancerous biological sample;
e) comparing the signal intensity of the at least one super-enhancer in the cancerous biological sample against a reference signal intensity of the at least one super-enhancer obtained from a non-cancerous biological sample; and
f) determining the presence or absence of at least one cancer-associated super-enhancer in a subject based on the change in signal intensity of the at least one super-enhancer in the cancerous biological sample relative to the non-cancerous biological sample,
wherein the presence or absence of at least one cancer-associated super-enhancer is indicative of the prognosis of the cancer in the subject.

In one aspect, there is provided a method of determining the susceptibility of a subject to cancer or a gastrointestinal disease, comprising:
a) contacting a biological sample obtained from the subject with at least one antibody specific for histone modification H3K27ac;
b) isolating nucleic acid from the biological sample, wherein the isolated nucleic acid comprises at least one region specific to the histone modification H3K27ac;
c) mapping at least one enhancer using an annotated genome sequence based on a signal intensity of the histone modification H3K27ac, wherein the at least one enhancer is at least 2.5 kb from an annotated transcription start site;
d) mapping the at least one enhancer in the isolated nucleic acid against at least one enhancer in at least one reference nucleic acid sequence to identify at least one super-enhancer in the biological sample;
e) comparing the signal intensity of the at least one super-enhancer in the biological sample against a reference signal of the at least one super-enhancer obtained from a control biological sample; and
f) determining the presence or absence of the at least one super-enhancer based on the change in signal intensity of the at least one super-enhancer;
g) mapping the presence or absence of the at least one super-enhancer against a reference genome sequence comprising cancer or gastrointestinal disease associated SNPs,
wherein the presence or absence of the at least one super-enhancer associated with one or more cancer or gastrointestinal disease associated SNPs is indicative of the subjects susceptibility to cancer or a gastrointestinal disease.

In one aspect, there is provided a method for modulating the activity of at least one cancer-associated super-enhancer in a cell, comprising administering an inhibitor of CDX2 and/or HNF4α to the cell.

In one aspect, there is provided a biomarker comprising at least one super-enhancer having increased signal intensity of H3K27ac in a cancerous biological sample relative to a normal non-cancerous biological sample, or at least one super-enhancer associated with an increase in cancer-associated transcription factor binding sites relative to unaltered super-enhancers, or both, for use in detecting cancer in a subject.

In one aspect, there is provided a use of a biomarker comprising at least one super-enhancer having increased signal intensity of H3K27ac in a cancerous biological sample relative to a normal non-cancerous biological sample, or at least one super-enhancer associated with an increase in cancer-associated transcription factor binding sites relative to unaltered super-enhancers, or both in the manufacture of a medicament for detecting cancer in a subject.

In one aspect, there is provided an inhibitor of CDX2 and/or HNF4α for use in modulating the activity of at least one cancer-associated super-enhancer in a cell.

In one aspect, there is provided a use of an inhibitor of CDX2 and/or HNF4α in the manufacture of a medicament for modulating the activity of at least one cancer-associated super-enhancer in a cell.

In one aspect, there is provided a method of predicting cancer cell survival or cancer cell viability in a cancerous biological sample obtained from a subject comprising:
a) contacting the cancerous biological sample with at least one antibody specific for histone modification H3K27ac;
b) isolating nucleic acid from the cancerous biological sample, wherein the isolated nucleic acid comprises at least one region specific to the histone modification H3K27ac;
c) mapping at least one enhancer using an annotated genome sequence based on a signal intensity of the histone modification H3K27ac, wherein the at least one enhancer is at least 2.5 kb from an annotated transcription start site;
d) mapping the at least one enhancer in the isolated nucleic acid against at least one enhancer in at least one reference nucleic acid sequence to identify at least one super-enhancer in the cancerous biological sample;
e) comparing the signal intensity of the at least one super-enhancer in the cancerous biological sample against a reference signal intensity of the at least one super-enhancer obtained from a non-cancerous biological sample; and
f) determining the presence of at least one cancer-associated super-enhancer in a subject based on the change in signal intensity of the at least one super-enhancer,
wherein an increased signal intensity of the at least one super-enhancer in the cancerous biological sample relative to the non-cancerous biological sample is predictive of cancer cell survival or cancer cell viability.

### DEFINITIONS

The following words and terms used herein shall have the meaning indicated:

The term "super-enhancer" refers to a cluster of DNA enhancer elements that occur in proximity to each other. A DNA enhancer element is a region of DNA that is capable of integrating diverse cellular and signaling inputs to regulate effector gene expression programs. Compared to typical enhancers, a super enhancer may be larger in size, may exhibit higher transcription factor binding densities and may be more strongly associated with key cell identity regulators, similar to locus control regions (LCRs), DNA methylation valleys, transcription initiation platforms and stretch enhancers. Super-enhancers may also be enriched in disease-associated genetic variants, and may be acquired by cancer cells at key oncogenes and be more sensitive to therapeutic perturbation.

The term "histone modification" refers to covalent modification of histone proteins. Histone modification includes but is not limited to methylation, phosphorylation, acetylation, ubiquitination and sumoylation. Modification of histones may alter chromatin structure and affect gene expression. It is generally understood that modification of histones may occur at one or more amino acids in one or more histones.

The term "annotated genomic sequence" refers to a genomic sequence for which information, including but not limited to coding and non-coding regions, regulatory regions or motifs, transcription start sites and genes has been identified. The term "annotated transcription start site" refers to an identified transcription start site.

The terms "reference", "control" or "standard" as used herein refer to samples or subjects on which comparisons may be performed. Examples of a "reference", "control" or "standard" include a non-cancerous sample obtained from the same subject, a sample obtained from a non-metastatic tumour, a sample obtained from a subject that does not have cancer or a sample obtained from a subject that has a different cancer subtype. The terms "reference", "control" or "standard" as used herein may also refer to the average signal intensity of chromatin modification. The terms "reference", "control" or "standard" as used herein may also refer to a subject who is not suffering from cancer or who is suffering from a different type of cancer. The terms "reference", "control" or "standard" as used herein may also refer to a nucleic acid sequence on which comparisons may be performed. For example, a reference or control or standard may be an untransfected cell.

The term "cancerous" as used herein relates to being affected by or showing abnormalities characteristic of cancer.

The term "antibody" or "antibodies" as used herein refers to molecules with an immunoglobulin-like domain and includes antigen binding fragments, monoclonal, recombinant, polyclonal, chimeric, fully human, humanised, bispecific and heteroconjugate antibodies; a single variable domain, single chain Fv, a domain antibody, immunologically effective fragments and diabodies.

The terms "isolated" or "isolating" as used herein relate to a biological component (such as a nucleic acid molecule, protein or organelle) that has been substantially separated or purified away from other biological components in the cell of the organism in which the component naturally occurs, i.e., other chromosomal and extra-chromosomal DNA and RNA, proteins and organelles. Nucleic acids and proteins that have been "isolated" include nucleic acids and proteins purified by standard purification methods. The term also embraces nucleic acids and proteins prepared by recombinant expression in a host cell as well as chemically synthesized nucleic acids.

The term "nucleic acid" as used herein refers to a deoxyribonucleotide or ribonucleotide polymer in either single or double stranded form, and unless otherwise limited, encompassing known analogues of natural nucleotides that hybridize to nucleic acids in a manner similar to naturally occurring nucleotides. "Nucleotide" includes, but is not limited to, a monomer that includes a base linked to a sugar, such as a pyrimidine, purine or synthetic analogs thereof, or a base linked to an amino acid, as in a peptide nucleic acid (PNA). A nucleotide is one monomer in a polynucleotide. A nucleotide sequence refers to the sequence of bases in a polynucleotide.

The term "biomarker" as used herein refers to an indicator of a biological state or condition.

The term "sample" or "biological sample" as used herein refers to one or more cells, fragments of cells, tissue or fluid that has been obtained from, removed or isolated from a subject. The term "obtained or derived from" as used herein is meant to be used inclusively. That is, it is intended to encompass any nucleotide sequence directly isolated from a biological sample or any nucleotide sequence derived from the sample. An example of a sample is a tumour tissue biopsy. Samples may be frozen fresh tissue, paraffin embedded tissue or formalin fixed paraffin embedded (FFPE) tissue. An example of a biological sample or a fluid sample includes but is not limited to blood, stool, serum, saliva, urine, cerebrospinal fluid and bone marrow fluid.

The term "prognosis", or grammatical variants thereof, as used herein refers to a prediction of the probable course and outcome of a clinical condition or disease. A prognosis of a patient is usually made by evaluating factors or symptoms of a disease that are indicative of a favorable or unfavorable course or outcome of the disease. The term "prognosis" does not refer to the ability to predict the course or outcome of a condition with 100% accuracy. Instead, the term "prognosis" refers to an increased probability that a certain course or outcome will occur; that is, that a course or outcome is more likely to occur in a patient exhibiting a given condition, when compared to those individuals not exhibiting the condition.

The term "susceptibility to cancer" as used herein refers to the likelihood or probability that a subject will develop cancer. A subject that is susceptible to cancer may or may not already be suffering from cancer, or may be suffering from a different type of cancer.

The term "inhibitor" as used herein refers to an agent that decreases or suppresses a biological activity. For example, an inhibitor may decrease or silence the expression of a gene. An inhibitor may also decrease the activity of a protein, enzyme or transcription factor. Examples of inhibitors include but are not limited to an oligonucleotide, a small molecule or a compound. The oligonucleotide may be an interfering RNA (iRNA), including but not limited to small interfering RNA (siRNA) or short hairpin RNA (shRNA). A small molecule will generally be understood in the art as a compound that has a low molecular weight. Another example of an inhibitor may be the clustered regularly interspaced short palindromic repeats (CRISPR) genome editing system. The CRISPR genome editing system may be a CRISPR/Cas system. The CRISPR/Cas system may inhibit gene expression by modifying the genome. Modification of the genome includes but is not limited to deletion, insertion or substitution of nucleotides. The CRISPR/Cas system may also inhibit gene expression by posttranslational modification of one or more histones. In some aspects of the disclosure, the CRISPR/Cas system may be CRISPR/Cas9.

Throughout this disclosure, certain aspects may be disclosed in a range format. It should be understood that the description in range format is merely for convenience and brevity and should not be construed as an inflexible limitation on the scope of the disclosed ranges. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Certain aspects of the disclosure may also be described broadly and generically herein. Each of the narrower species and subgeneric groupings falling within the generic disclosure also form part of the disclosure. This includes the generic description of the aspects with a proviso or negative limitation removing any subject matter from the genus, regardless of whether or not the excised material is specifically recited herein.

Unless the context requires otherwise or specifically stated to the contrary, integers, steps, or elements of the invention recited herein as singular integers, steps or elements clearly encompass both singular and plural forms of the recited integers, steps or elements.

The word "substantially" does not exclude "completely" e.g. a composition which is "substantially free" from Y may be completely free from Y. Where necessary, the word "substantially" may be omitted from the definition of the invention.

The invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non- limiting examples. In addition, where features or aspects of the invention are described in terms of Markush groups, those skilled in the art will recognize that the invention is also thereby described in terms of any individual member or subgroup of members of the Markush group.

### BRIEF DESCRIPTION OF THE DRAWINGS

The invention will be better understood with reference to the detailed description when considered in conjunction with the non-limiting examples and the accompanying drawings, in which:

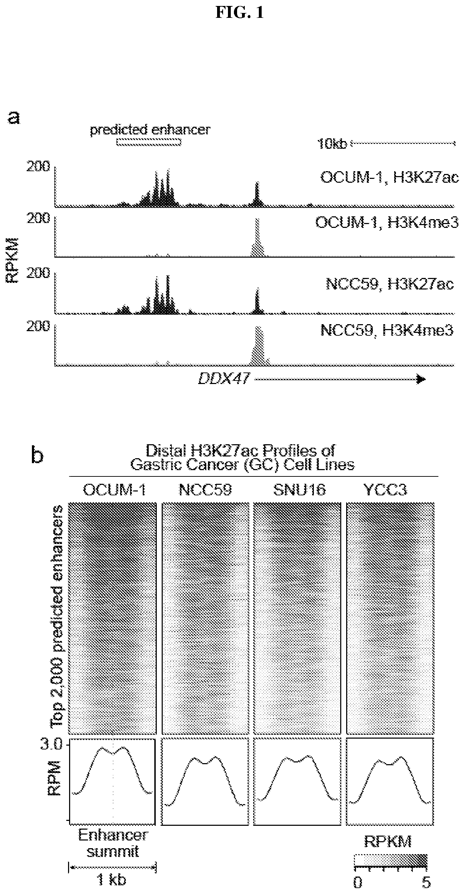

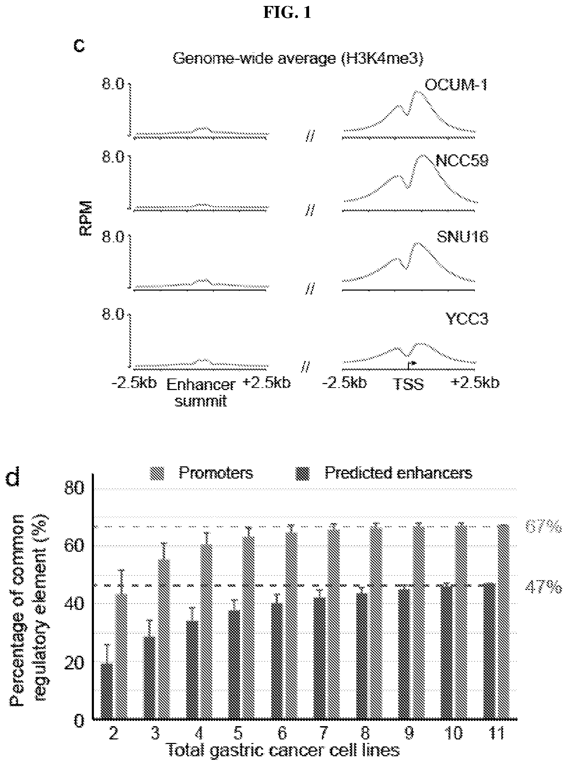

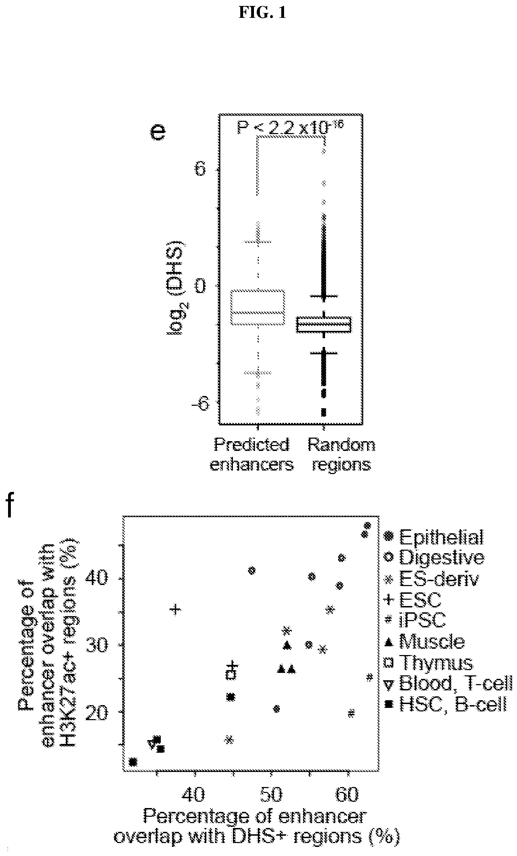

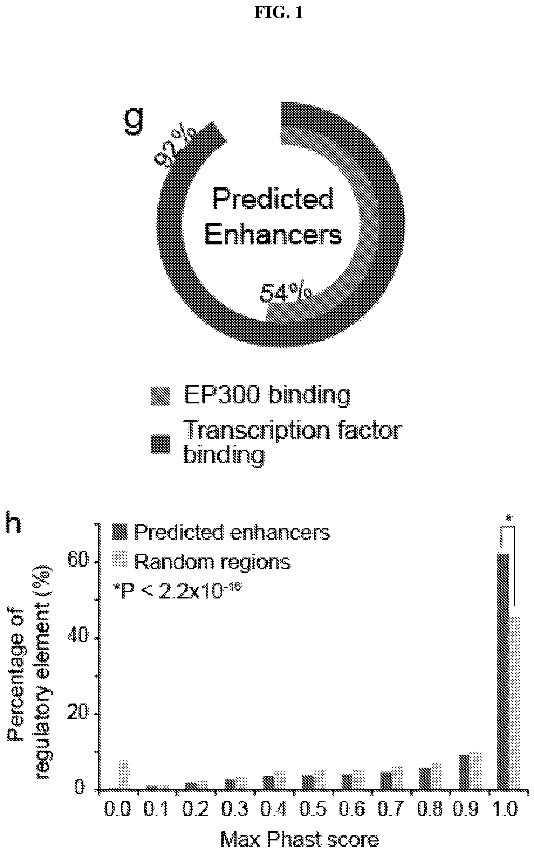

**Fig. 1** **Distal Predicted Enhancer landscapes of GC cell lines.**
   a. Histone profiles of OCUM-1 and NCC59 GC cells show enrichment of H3K27ac and H3K4me3 around the *DDX47* transcription start site (TSS). A predicted enhancer element exhibiting H3K27ac enrichment and >2.5Kb away from the *DDX47* TSS was identified.
   b. Snapshot of distal H3K27ac profiles in 4 of the 11 GC cell lines, visualizing the activity of the top 2,000 predicted enhancers and the genome-wide average H3K27ac signal around the predicted enhancers.
   c. Genome-wide average H3K4me3 signals around predicted enhancers and active TSSs in GC cell lines.
   d. Percentage of common regulatory elements (enhancer - dark grey; promoter - light grey) found in two or more gastric cancer cell lines, as a function of number of cell lines.
   e. Chromatin accessibility of predicted enhancers versus randomly selected regions. DNase I hypersensitivity (DHS) data from normal gastric tissues⁴² was used as a surrogate. The distribution of DHS signals was tested using a one-side Welch's t-test for statistical significance.
   f. Percentage of overlap between predicted enhancers, chromatin accessible regions (denoted as DHS+, x-axis) and active regulatory elements (denoted as H3K27ac+, y-axis) from 50 epigenomic profiles originating from 9 different tissue/cell categories.
   g. Percentage of predicted enhancers overlapping with EP300 and transcription factor binding sites.
   h. Distribution of maximum Phast scores (a measure of DNA sequence conservation) in predicted enhancers and randomly selected regions.

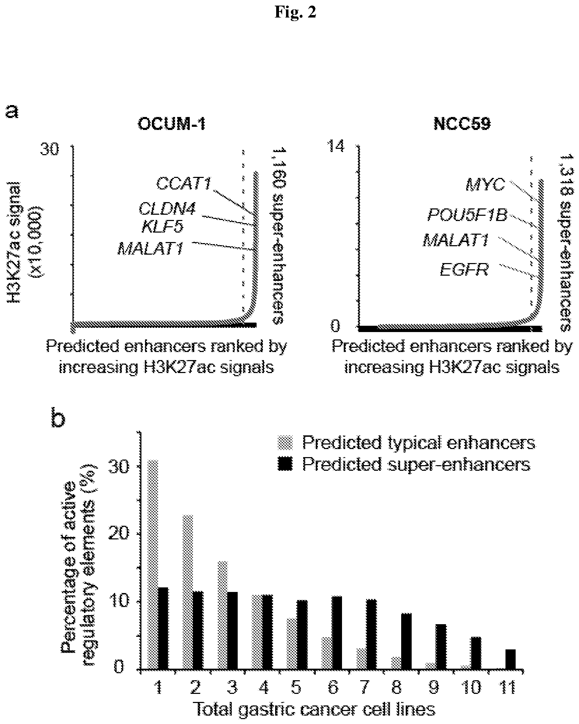

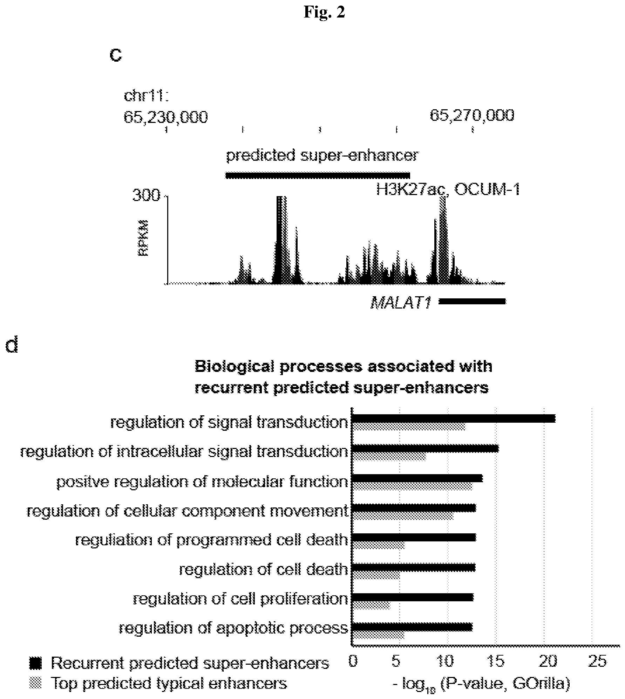

**Fig. 2** **GC cell line derived predicted super-enhancers**
   a. Distribution of H3K27ac ChIP-seq signals reveal locations of predicted super-enhancers showing unevenly high H3K27ac signals. Known cancer-associated genes proximal to predicted super-enhancers are indicated. Two cell lines are shown.
   b. Percentage of distal regulatory elements (predicted typical enhancers - light grey, predicted super-enhancers - dark grey) showing H3K27ac enrichment above randomly selected regions (>99%) across increasing numbers of GC cell lines.
   c. H3K27ac ChIP-seq signals at the *MALAT1* locus shows stretches of predicted enhancers, corresponding to a predicted super-enhancer (in filled box) with high H3K27ac signals.
   d. Examples of top significantly associated biological processes associated with recurrent distal regulatory elements (predicted super-enhancers and top predicted typical enhancers). Negative log-transformed raw p-values from GOrilla were used.

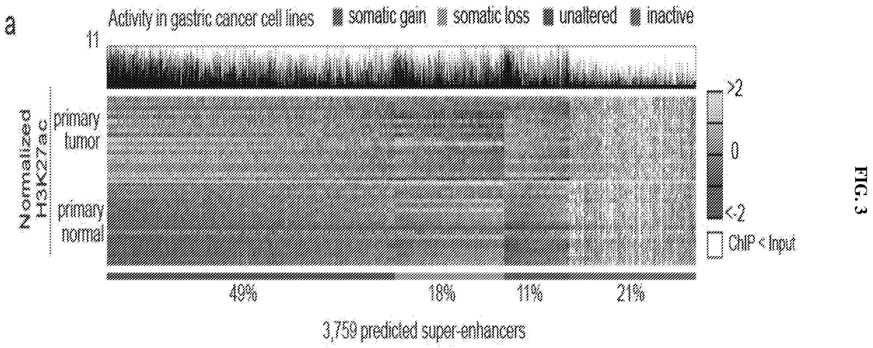

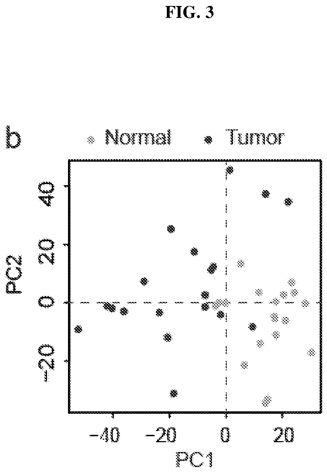

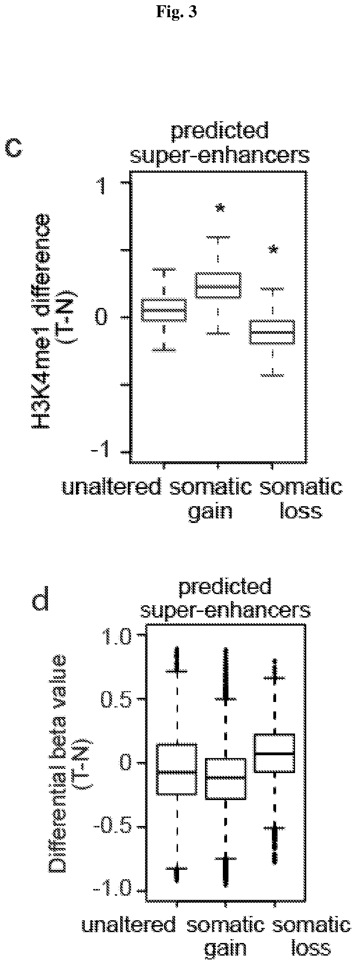

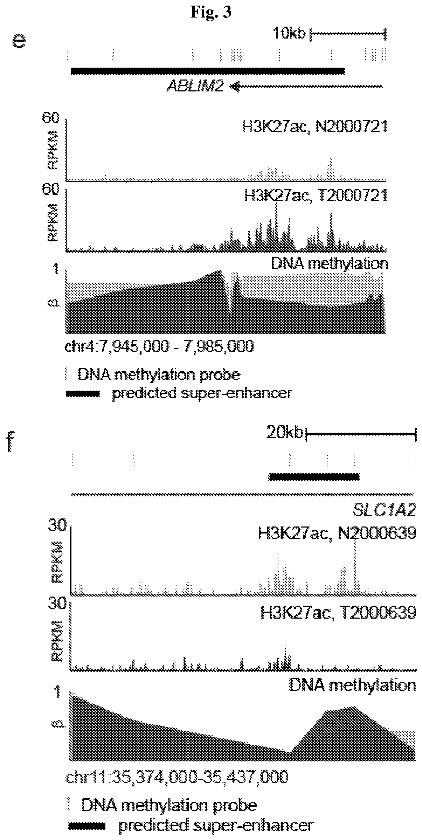

**Fig. 3** **Somatic predicted super-enhancers in primary GCs and matched normal samples.**
   a. Activity of cell-line derived predicted super-enhancers in 19 primary tumor and matched normal samples. H3K27ac predicted super-enhancer signals in units of column-transformed RPKM values (z-score) were visualized. The frequency of active predicted super-enhancers in GC lines *in vitro* is presented as the top histogram (black, above the heatmap). Predicted super-enhancers were categorized into somatic gain, somatic loss, unaltered and inactive. In each category, the predicted super-enhancers were ordered (left to right) by their decreasing mean difference between the tumor and the normal samples.
   b. Principal component analysis using recurrent somatic gain predicted super-enhancer signals establish a separation between tumor and normal samples.
   c. Differences in H3K4me1 (T-N) signals (RPKM) using H3K4me1 profiles from 5 tumor and matched normal samples in three predicted super-enhancer categories: somatic gain, somatic loss, and unaltered. ^{∗}P<2.2×10⁻¹⁶, one-sided Welch t-test.
   d. Differential β values in predicted super-enhancers indicate the state of methylation: hypermethylation (>0) or hypomethylation (<0) between tumors and matched normal samples.
   e. DNA hypomethylation in a somatic gain predicted super-enhancer at the *ABLIM2* locus.
   f. DNA hypermethylation in a somatic loss predicted super-enhancer at the *SLC1A2* locus.

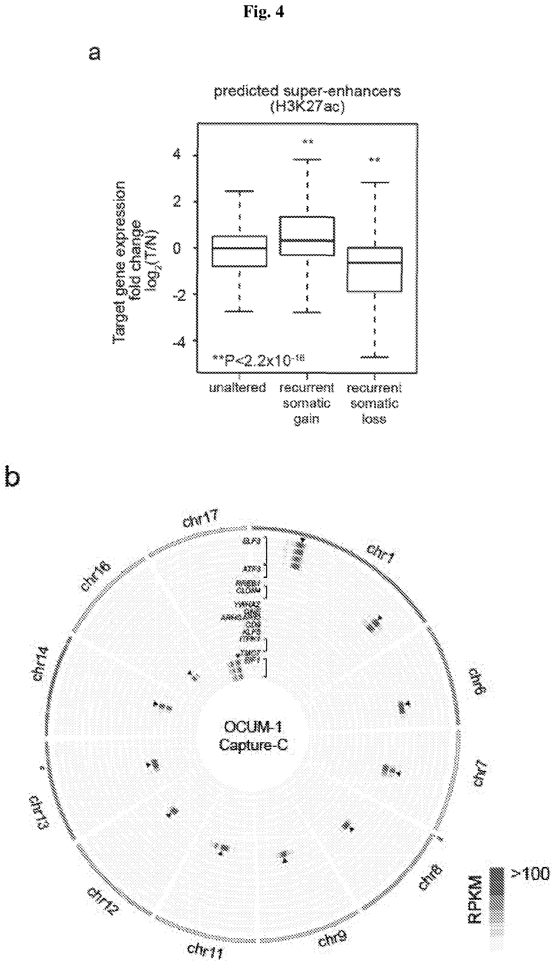

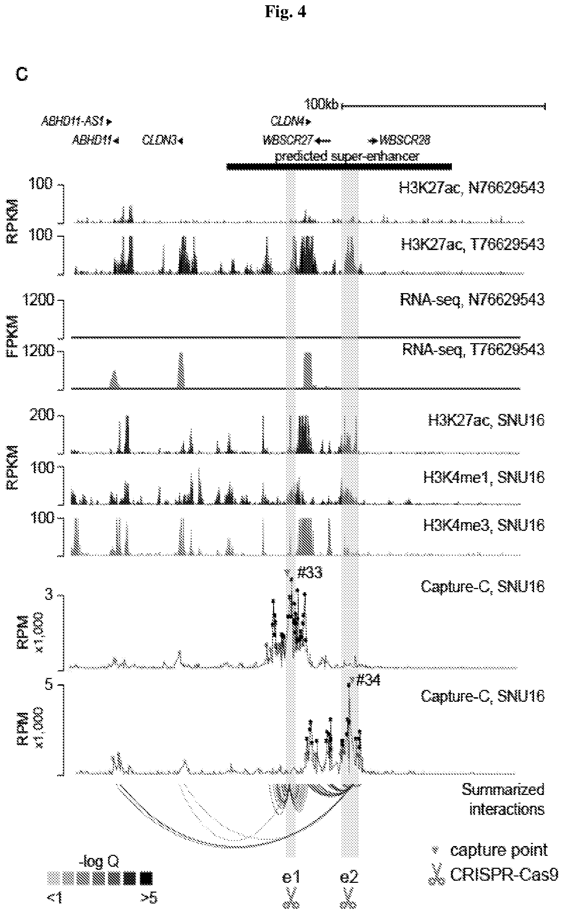

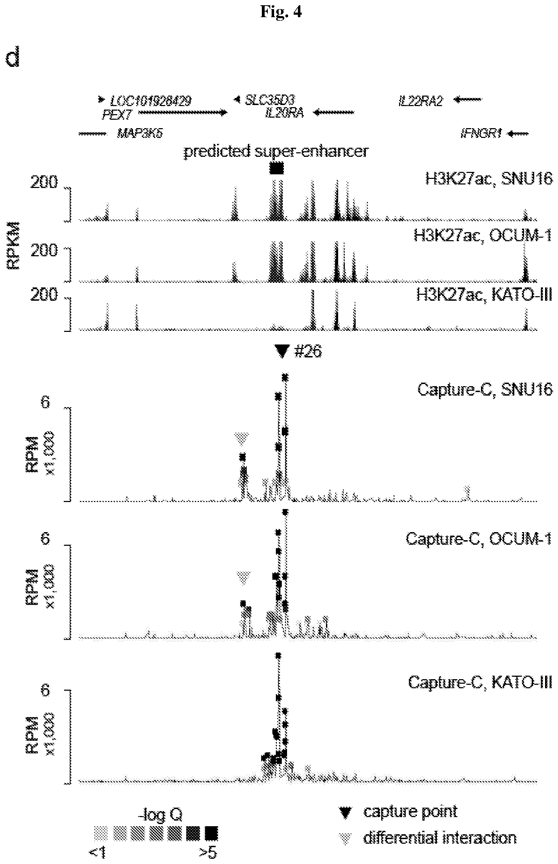

**Fig. 4** **Associations between somatic predicted super-enhancers with gene expression and chromatin interactions**
   a. Correlation between log-transformed fold changes in gene expression between different classes of predicted super-enhancers (unaltered, somatic gain, somatic loss) and predicted target gene expression.
   b. Interaction heat map from 20 capture points covering 12 somatic gain predicted super-enhancers. Each ring represents a profile from a single capture point, denoted by a black arrowhead. Locations of the predicted super-enhancers are indicated by the gene loci in each ring. Genome wide interaction signals were computed across the genome in 100kb bins. Signals at regions within 2 million bases flanking the capture points were visualized.
   c. Example of a somatic gain predicted super-enhancer at the *CLDN4* locus and interactions with neighbouring genes. Somatic gain activity is associated with up-regulation of *CLDN4* and neighbouring genes (*CLDN3* and *ABHD11*) in primary GCs. Interactions were detected in SNU16 cells using two capture points, #33 and #34 by Capture-C. Summarized interactions (Q<0.05, r3Cseq) are presented as the last track. Two constituent predicted enhancers, e1 and e2, were deleted independently in SNU16 cells using CRISPR/Cas9 genome editing.
   d. Correlation between predicted super-enhancer activity and long-range interactions. Long-range interactions (light grey triangle) to the *SLC35D3* promoter were detected with a predicted super-enhancer active in SNU16 and OCUM-1 cells. Such interactions were not observed in KATO-III cells where the predicted super-enhancer was also not detected.

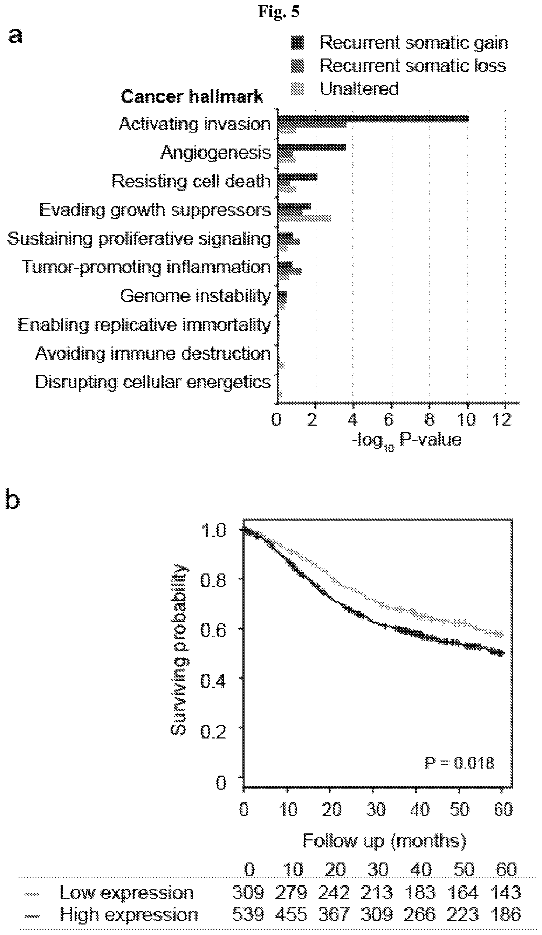

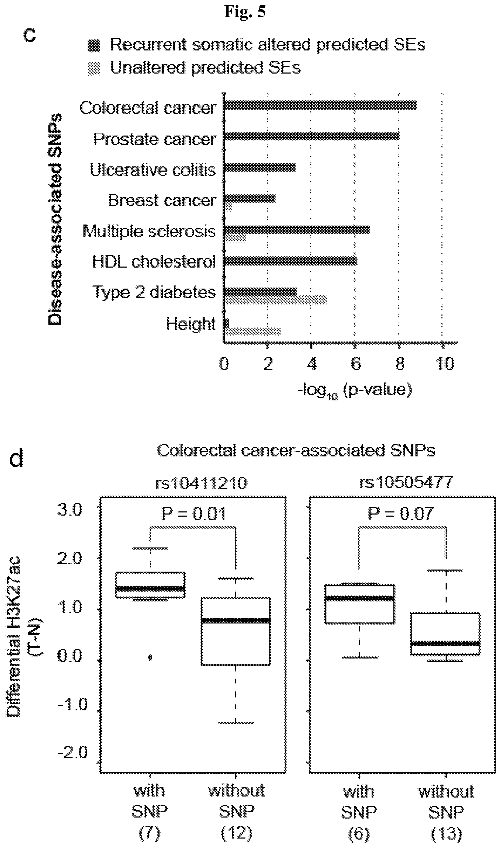

**Fig. 5** **Somatic predicted super-enhancers inform patient survival and disease risk.**
   a. Cancer hallmark analysis using predicted super-enhancers showing recurrent somatic gain, recurrent somatic loss and unaltered H3K27ac signals. Negative log-transformed p-values from the one-sided Fisher's exact test were used.
   b. Survival analysis comparing patient groups with samples exhibiting low (light grey) and high (dark grey) expression from genes associated with top recurrent somatic gain predicted super-enhancers. The signature is prognostic in the compilation of 848 GC patients (P = 1.8×10⁻², log-rank test), with worse prognosis observed for patients with tumors having high signature expression (hazard ratio, 95% confidence interval: 1.30 (1.05 - 1.61); Cox regression p-value after correcting for stage, age, patient locality and Lauren's histological subtypes = 4.4×10⁻²). Survival data is indicated for every 10 months.
   c. Enrichment of disease-associated SNPs in predicted super-enhancers. Enrichments were tested on two classes of predicted super-enhancers: recurrent somatic altered and unaltered predicted super-enhancers using chi-square test. Only diseases/traits with at least 10 SNPs found in all predicted super-enhancers were analysed.
   d. Differential H3K27ac signals in predicted super-enhancers with and without colorectal cancer associated SNPs. The total number of patients with or without the SNPs is indicated in brackets. The difference between the two groups was tested using one-sided Welch t-test.

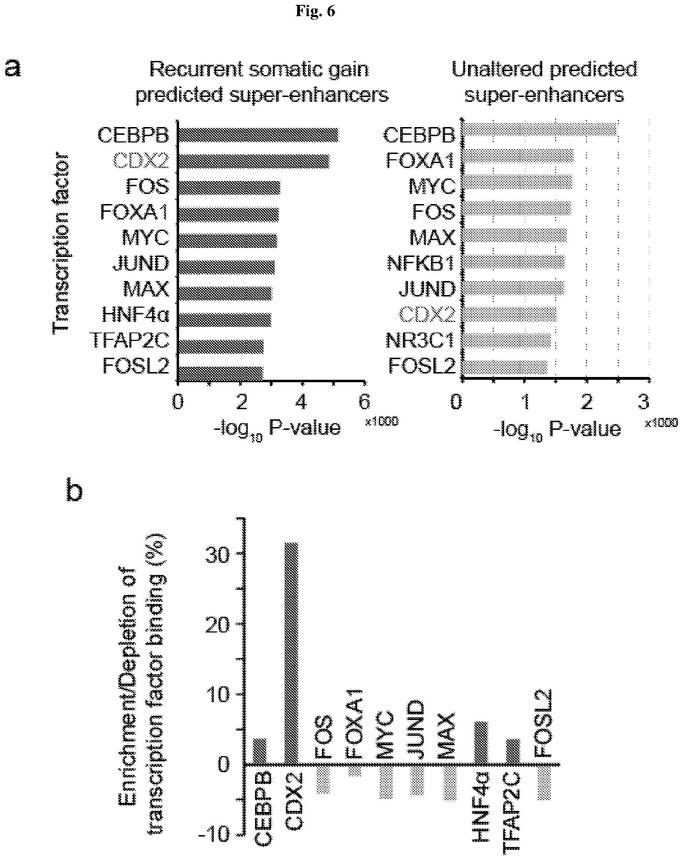

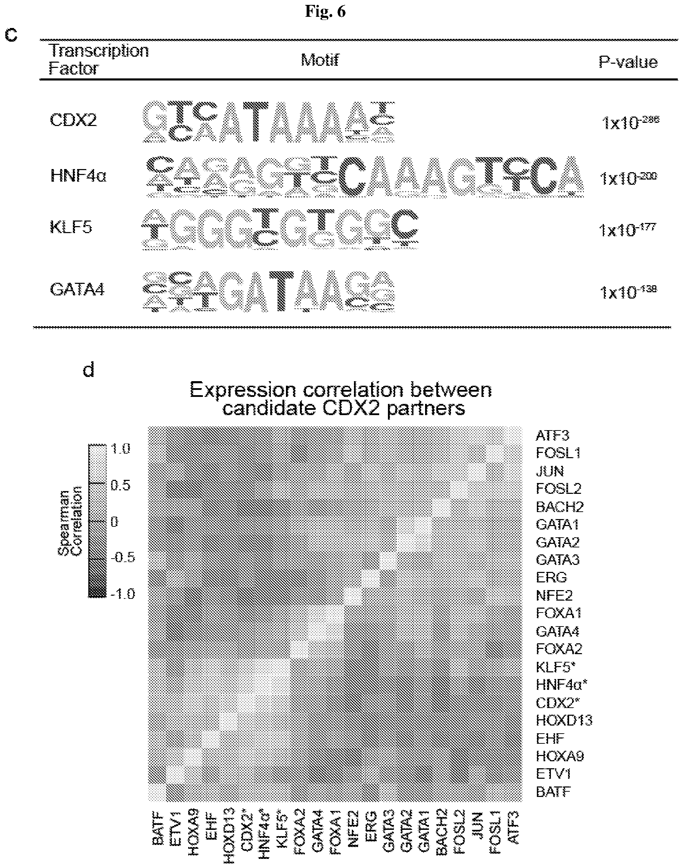

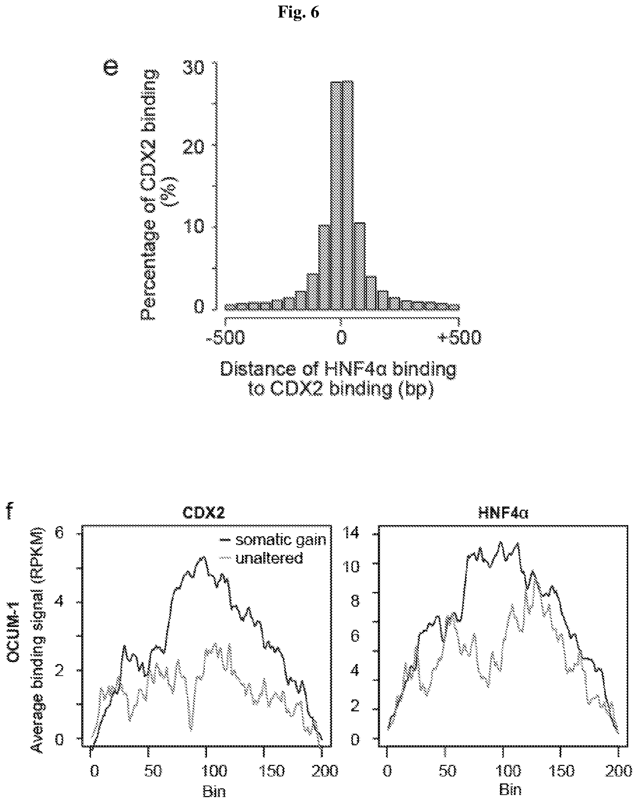

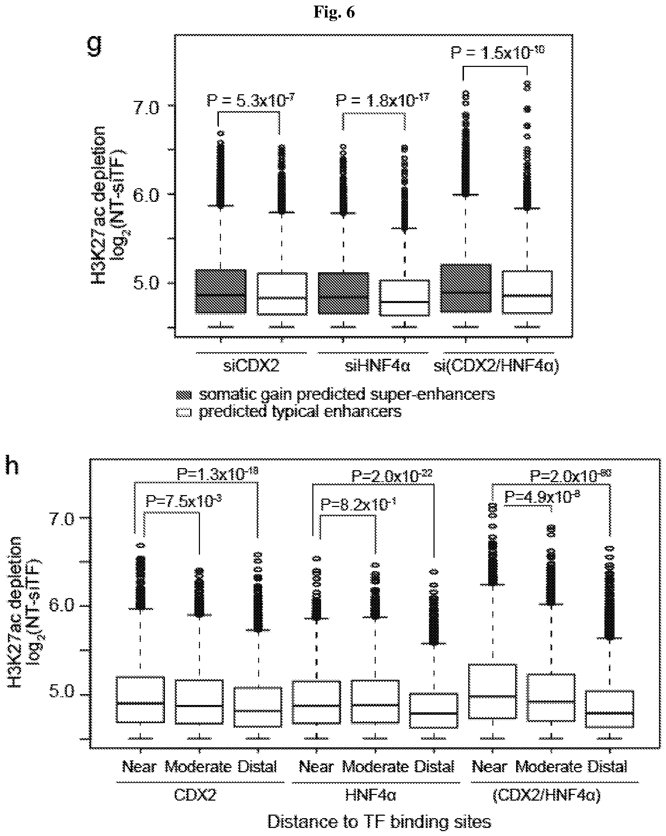

**Fig. 6** **Somatic gain predicted super-enhancers in GC are associated with CDX2 and HNF4α occupancy.**
   a. Top 10 transcription factor binding enrichments at recurrent somatic gain predicted super-enhancers and unaltered predicted super-enhancers using the ReMap database.
   b. Enrichment or depletion of ReMap transcription factors at recurrent somatic gain predicted super-enhancers compared to unaltered predicted super-enhancers.
   c. Detection of candidate CDX2 binding partners using CDX2 binding sites and *de novo* HOMER motif identification.
   d. Pairwise expression correlations of CDX2 and top 20 CDX2 candidate binding partners using RNA-seq from 19 primary tumor and matched normal samples.
   e. Percentage of CDX2 binding sites co-occurring with HNF4α binding sites within a 500bp window in OCUM-1 cells.
   f. Differential CDX2 (left) and HNF4α (right) average binding signal analysis between recurrent somatic gain predicted super-enhancers and unaltered predicted super-enhancers. The predicted super-enhancers were also active in OCUM-1.
   g. Distribution of H3K27ac depletion magnitude in between somatic gain predicted super-enhancers and predicted typical enhancers in OCUM-1 cells, for single and double TF silencing. Statistical significance was evaluated using the one-sided Wilcoxon rank sum test.
   h. Association between H3K27ac sub-regional depletion in somatic gain predicted super-enhancers relative to CDX2, HNF4α or CDX2/HNF4α co-binding sites. Distances were uniformly distributed into three categories: near, moderate and distal to the binding sites. Statistical significance was evaluated using one-sided Wilcoxon rank sum test.
**Fig. 7** **Comparisons between different mapping quality filters (MAPQ*≥*10 and MAPQ≥20).**
   a. Percentage of mapped reads detected using MAPQ≥20 compared to the total mapped reads using MAPQ≥ 10.
   b. Percentage of ChIP-enriched peaks discovered using MAPQ≥20 compared to the total number of ChIP-enriched peaks using MAPQ≥10.

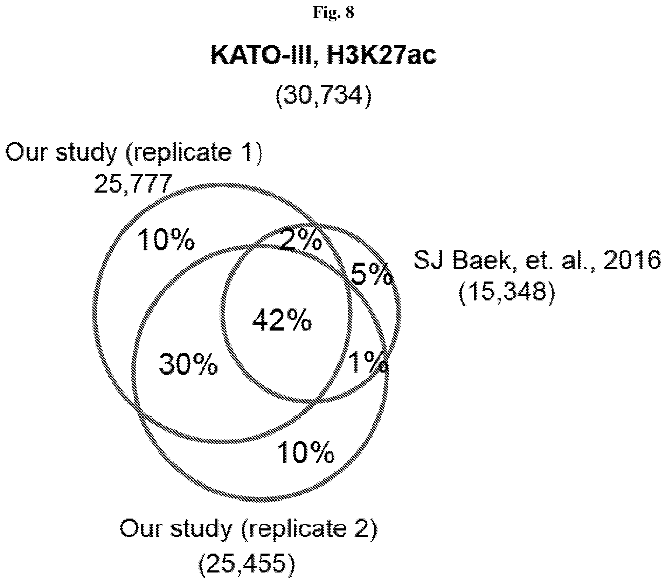

**Fig. 8** **Concordance of H3K27ac-enriched peaks among biological replicates from KATO-III cells.** Replicate 1 and 2 were generated using Nano-ChIPseq, while data from Baek et al. *Oncotarget* (2016) was created using conventional ChIPseq methods. The total number of mapped reads from replicate 1 and 2 is >10x more than the Baek et al. data, and therefore more peaks were detected in our replicates. Peaks from replicates were merged using BEDTools. Using this approach, 30,734 unique peaks were identified. Percentage of overlapping peaks found in replicates compared to the total number unique peaks was computed.
**Fig. 9** **Genome-wide H3K4me1 signals flanking distal predicted enhancers and active TSSs in gastric cancer cell lines.**
**Fig. 10** **Predicted super-enhancers in GC cell lines.**
   a. KLF5- and MYC-associated predicted super-enhancers in OCUM-1 and NCC59, respectively.
   b. Expression levels of genes (in percentile units, across the cell lines) linked to the top recurrent predicted super-enhancers (dark grey) and predicted typical enhancers (light grey). An identical number of randomly chosen genes (black) was used as the reference. Genes were sorted by percentiles in the order from highest to smallest.

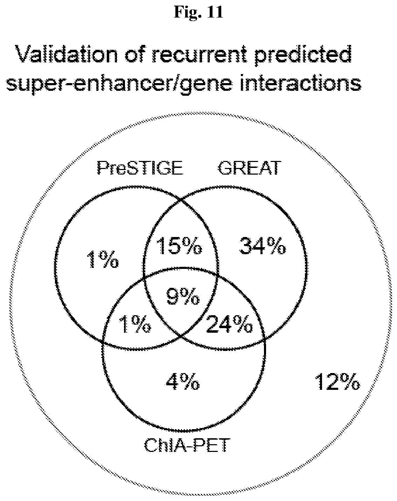

**Fig. 11** **Validation of recurrent predicted super-enhancer/gene interactions using public data sets.** Percentage values reflect the original predicted super-enhancer/gene assignments (see Results and Methods).

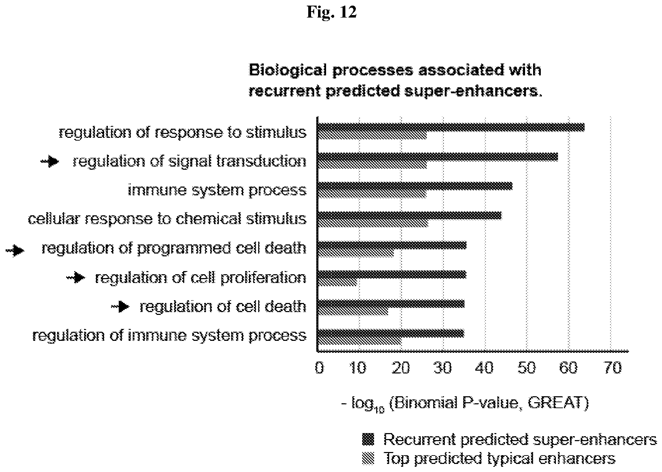

**Fig. 12** **Biological processes associated with recurrent predicted super-enhancers using GREAT analysis tool.** Processes highlighted by black arrows refer to processes observed by both GOrilla (see Results) and GREAT.

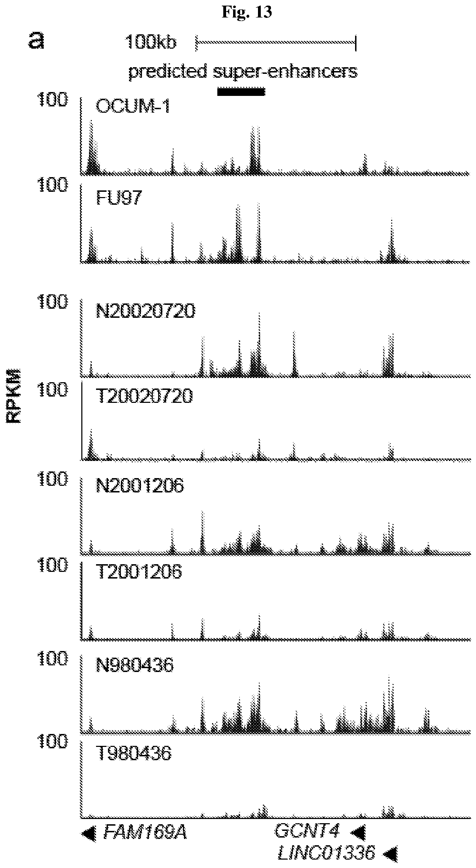

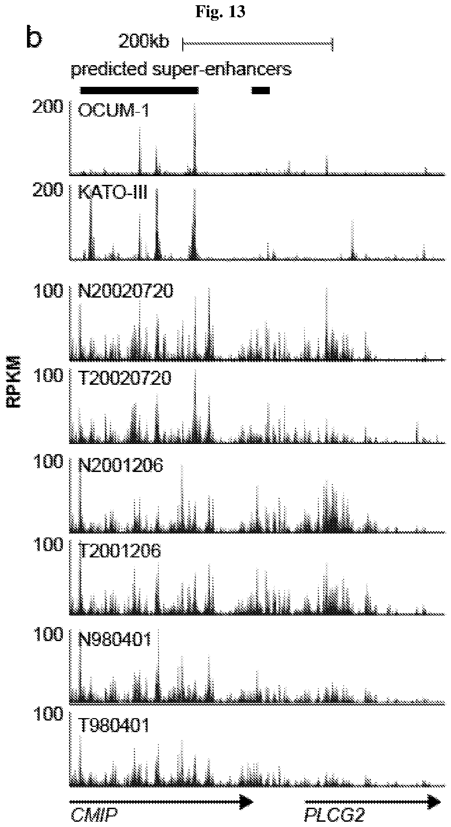

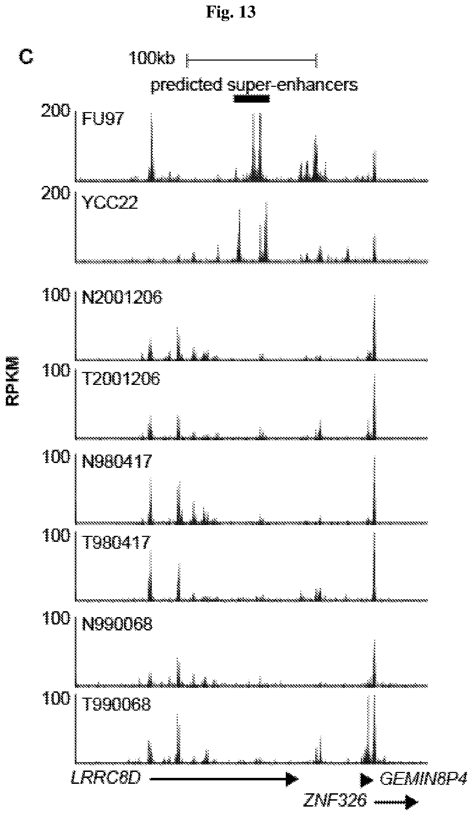

**Fig. 13** **Categorization of cell-line derived predicted super-enhancers using histone H3K27ac profiles from primary samples**.
   a. A somatic loss predicted super-enhancer in three tumor(T)/matched normal(N) pairs at the GCNT4 locus.
   b. An unaltered predicted super-enhancer in T/N20020720, T/N2001206 and T/N980401 at the CMIP locus.
   c. A predicted super-enhancer detected in FU97 and YCC22 GC cells shows an inactive state in three T/N pairs at the ZNF326 locus.

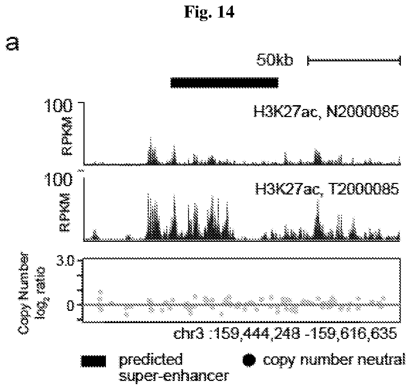

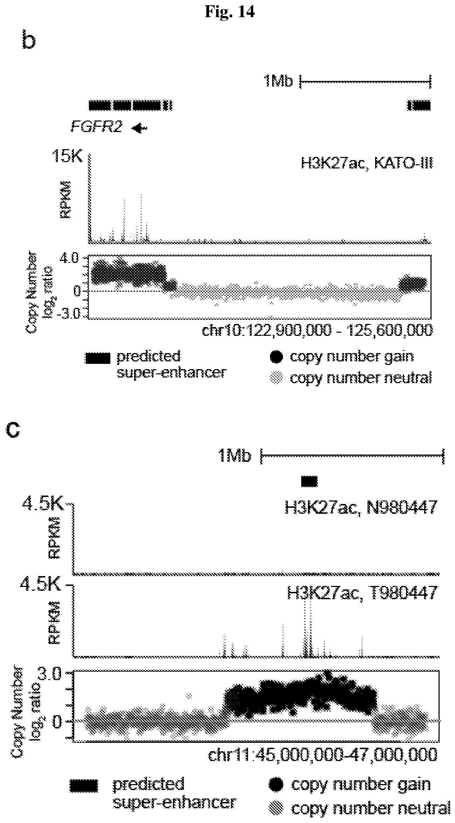

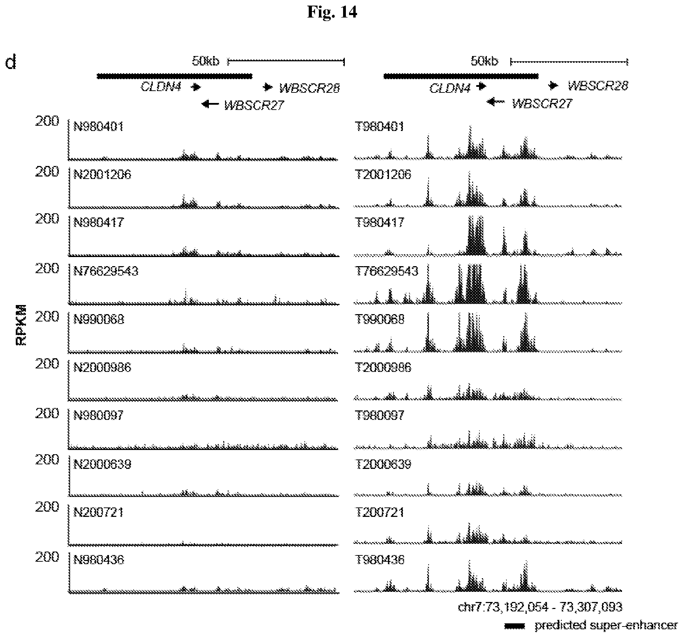

**Fig. 14** **Association between copy number alterations and predicted super-enhancers.**
   a. An example of a somatic gain predicted super-enhancer detected in a copy number neutral region.
   b. FGFR2-associated predicted super-enhancers detected at regions of somatic copy number gain in KATO-III cells.
   c. A somatic gain predicted super-enhancer detected in a region with copy number gain in T/N980447.
   d. A highly recurrent somatic gain (H3K27ac) predicted super-enhancer was detected at the CLDN4 locus. This region was not associated with copy number gain.

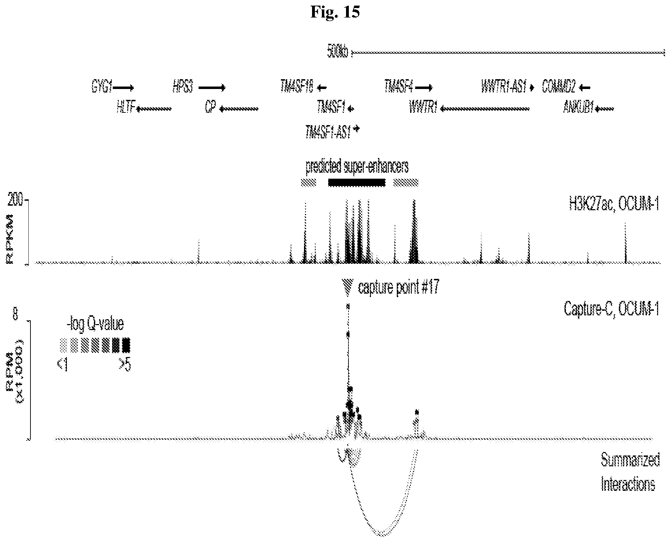

**Fig. 15** **Long-range interactions between a predicted super-enhancer (black rectangle) at TM4SF1 locus and the TM4SF4 promoter detected in OCUM-1 cells using Capture-C technology**. The bottom track indicates the summarized interactions from the capture point #17.

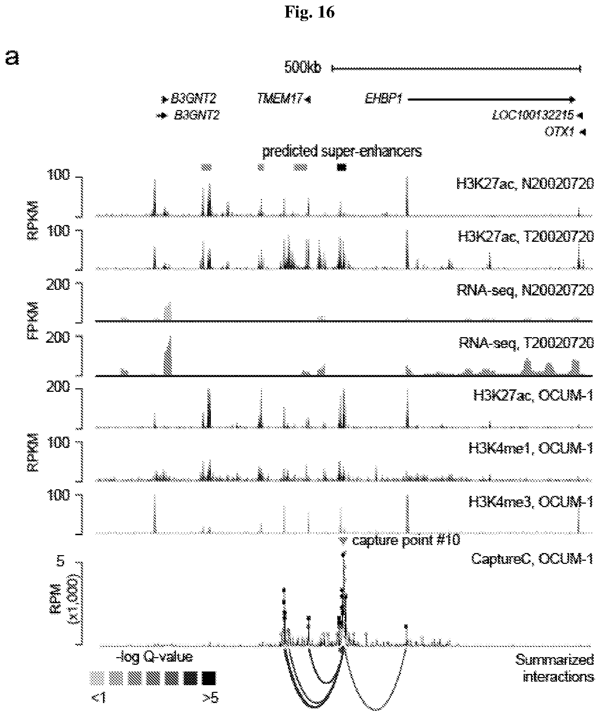

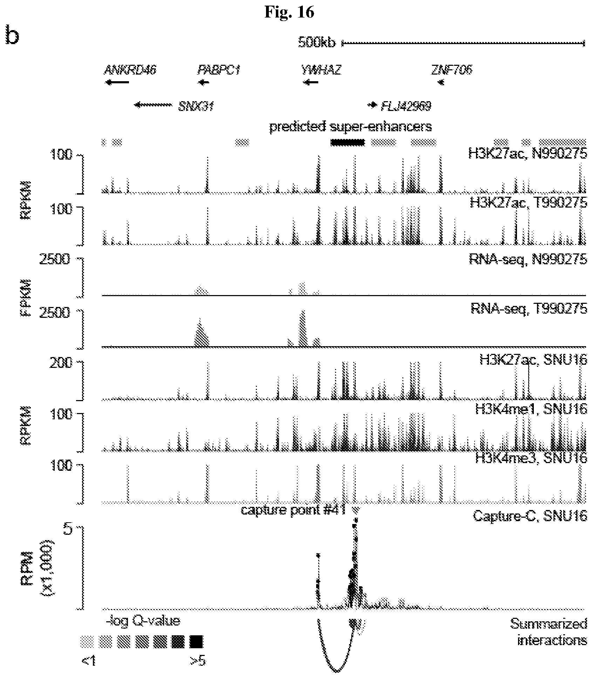

**Fig. 16** **Capture-C interaction profiles.**
   a. Interactions from the EHBP1 predicted super-enhancer (black rectangle) to promoters of TMEM1 and EHBP1 genes. The predicted super-enhancer was detected in OCUM-1 cells, showed somatic gain in primary tumor T20020720 and is associated with up-regulated expression of TMEM1 and EBHP1.
   b. Interactions from a predicted super-enhancer (black rectangle) at the YWHAZ locus to the promoter of YWHAZ. The predicted super-enhancer was detected in SNU16 cells, showed somatic gain in the primary tumor sample T990275 and is associated with up-regulated expression of YWHAZ.

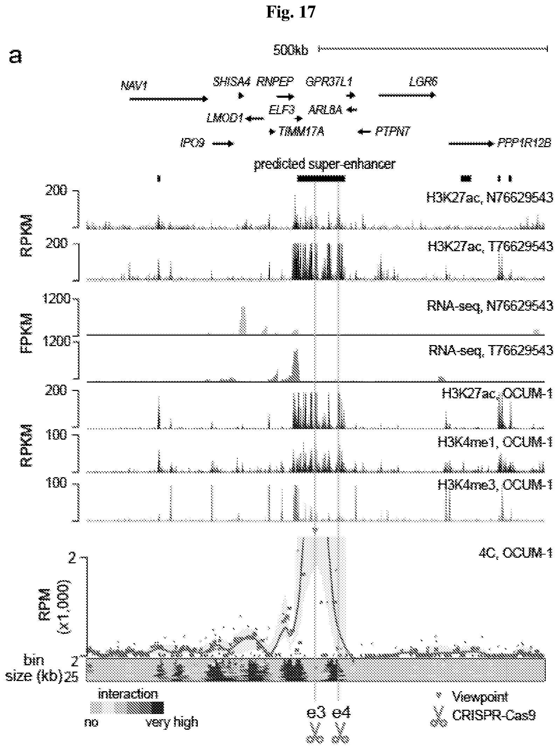

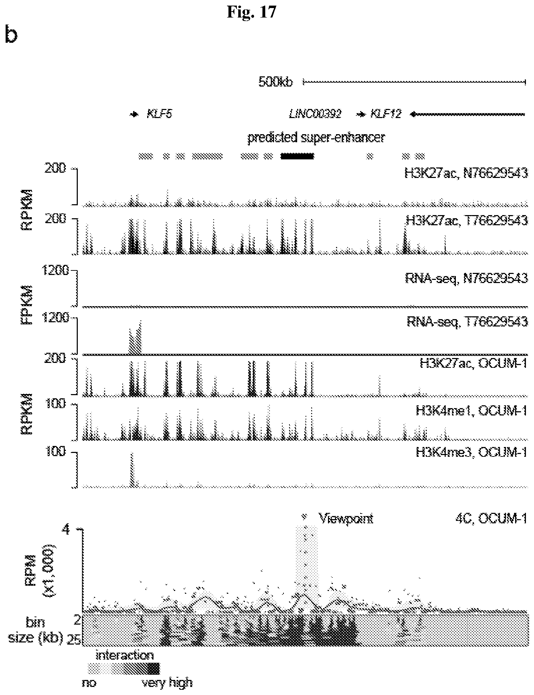

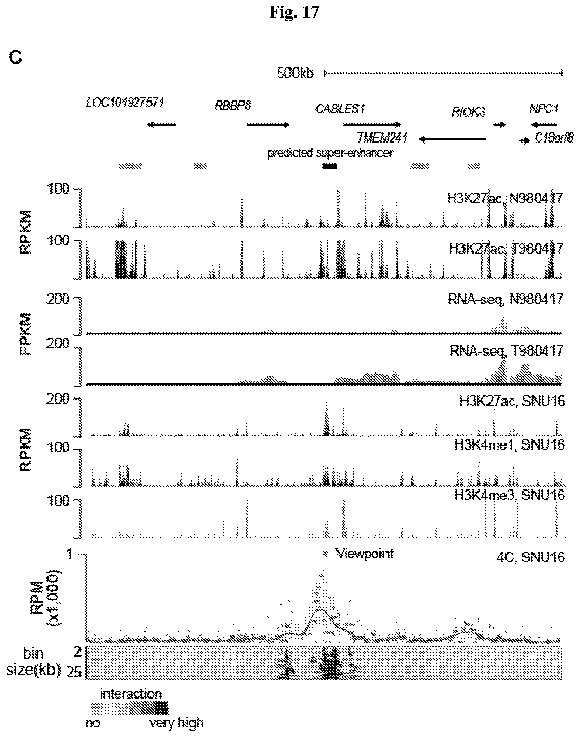

**Fig. 17** **4C** **interaction profiles.**
   a. Example of a somatic gain predicted super-enhancer at the ELF3 locus and interactions with neighbouring genes, such as ELF3, RNPEP, ARL8A and LMOD1. Somatic gain activity is associated with up-regulation of ELF3 in primary GCs. Interactions (Q<0.05, r3Cseq) were detected in OCUM-1 cells using 4C. The 4C signal plot (in units of RPM) was generated using the Basic4CSeq package. Two constituent enhancers, e3 and e4 were deleted independently in OCUM-1 cells using CRISPR/Cas9 genome editing technology.
   b. Long-range interactions between a predicted super-enhancer at KLF5 locus and the KLF5 promoter were detected in OCUM-1 cells. Somatic gain activity in the primary tumor (T76629543) is associated with up-regulation of KLF5 expression in the matched sample. c. Interactions of a predicted super-enhancer at the CABLES 1 locus to neighbouring non-coding regions and promoters of genes, including CABLES 1 and RIOK3.

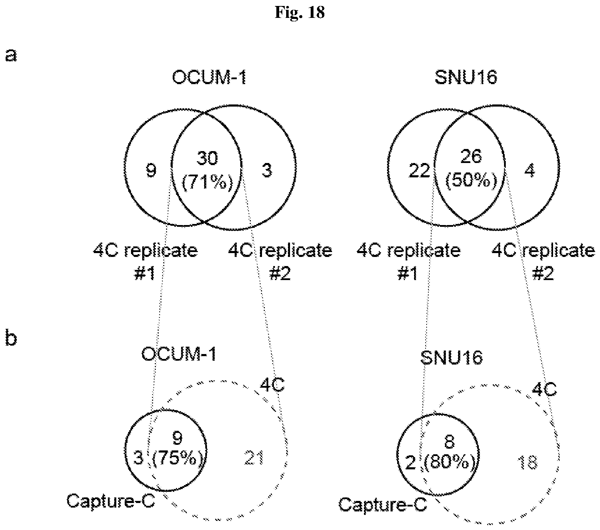

**Fig. 18** **Comparing interaction profiles from Capture-C and 4C.**
   a. Venn diagrams show the overlap of predicted super-enhancer/gene interactions (from 4C) between two biological replicates from OCUM-1 and SNU16 cells. The concordance between replicates was computed (percentage in brackets) with respect to all identified interactions.
   b. Venn diagrams show the overlap of predicted super-enhancer/gene interactions (from Capture-C) with the concordant set of interactions from 4C in the same cells. 75%-80% of the interactions identified by using Capture-C were rediscovered in the results using 4C.

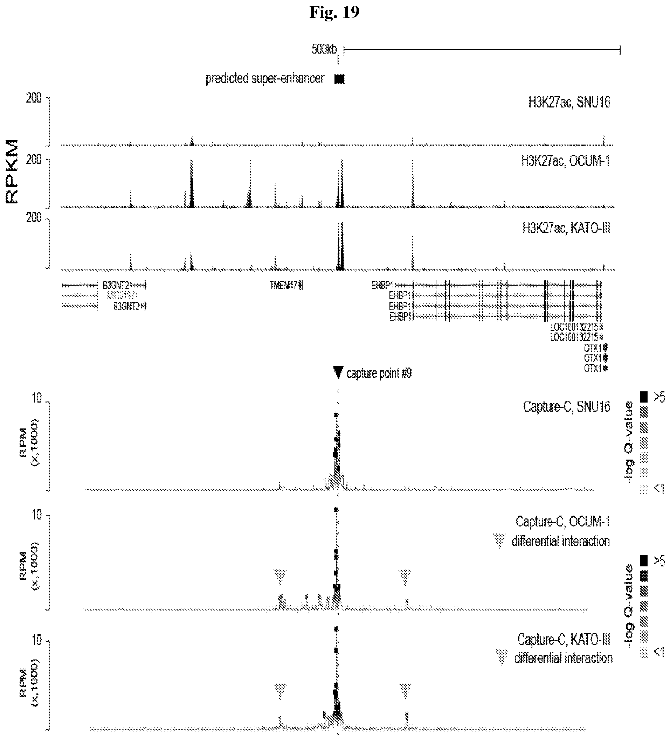

**Fig. 19** **An example of correlation between predicted super-enhancer activity and the presence of long-range interactions.** Long-range interactions (light grey triangle) to the *EHBP1* promoter were detected with a predicted super-enhancer (black rectangle) active in OCUM-1 and KATO-III cells. Such interactions were not observed in SNU16 cells where the predicted super-enhancer was also not detected.

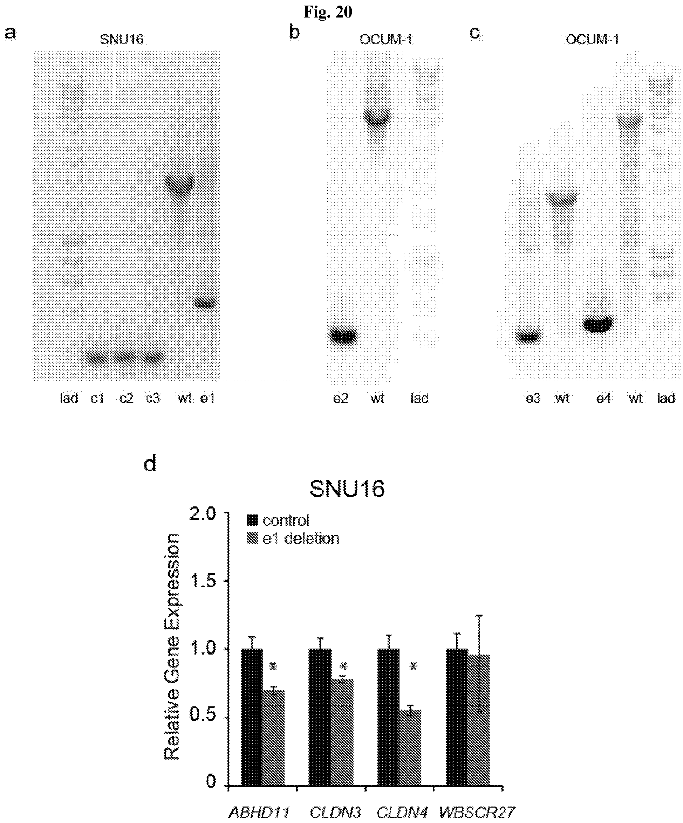

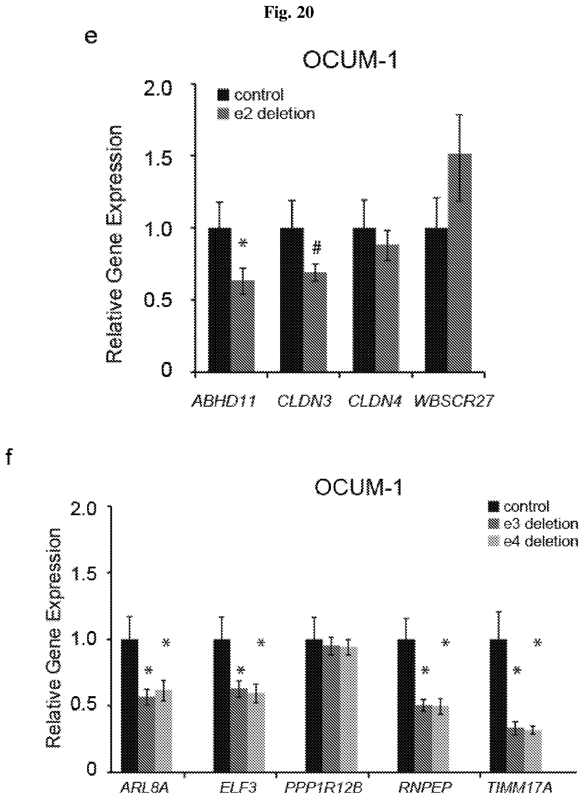

**Fig. 20** **Predicted enhancer deletion using CRISPR/Cas9 deletion.** PCR analysis of CRISPR/Cas9 deletion of a) the constituent enhancer, e1 in SNU16, b) the constituent enhancer, e2 in OCUM-1, c) the constituent enhancers, e3 and e4 in OCUM-1. (e-f) Differential gene expression between mutant (with one predicted enhancer deletion) and wild type cells was performed using RT-qPCR in OCUM-1 and SNU16 cells. Pooled cells were analysed. ^{∗}P<0.05, # P = 0.055, one-sided t-test; wt: wild type; lad: DNA ladder (Bioline HyperLadder I); c1-c3: wild type cells using GAPDH primers.

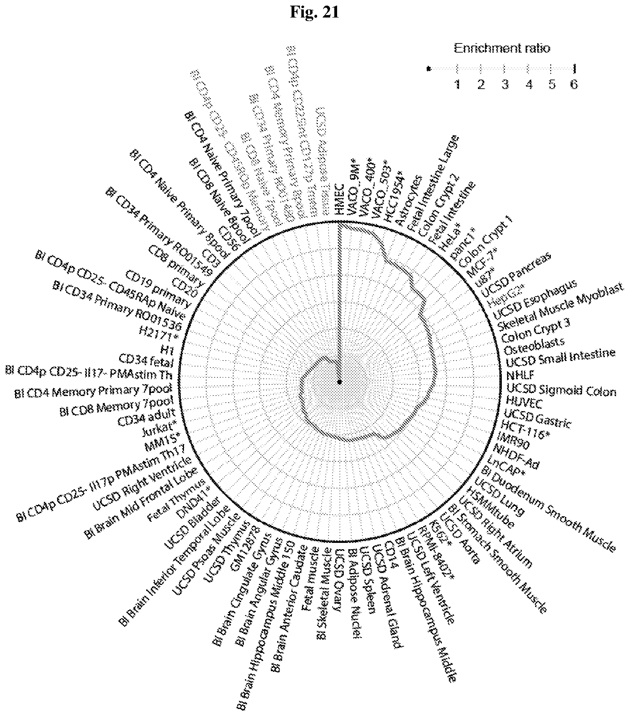

**Fig. 21** **Landscape of GC-associated predicted super-enhancers in other cell and tissue types.** Enrichment ratios of recurrent somatic gain predicted super-enhancers identified in GC overlapping with super-enhancers detected in 86 cell and tissue samples compared to randomly selected regions. Cancer cell lines are labelled in with asterisk; Samples with statistically insignificant (P > 0.001) enrichment ratios are in grey.

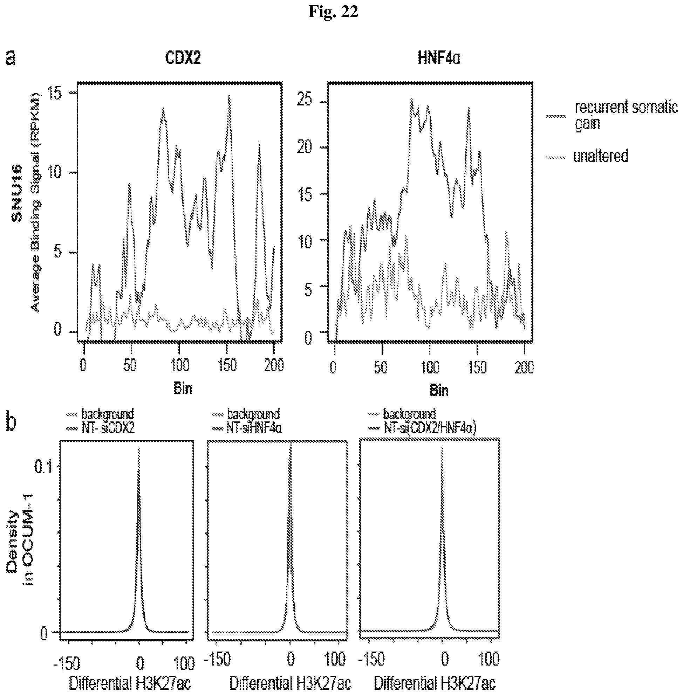

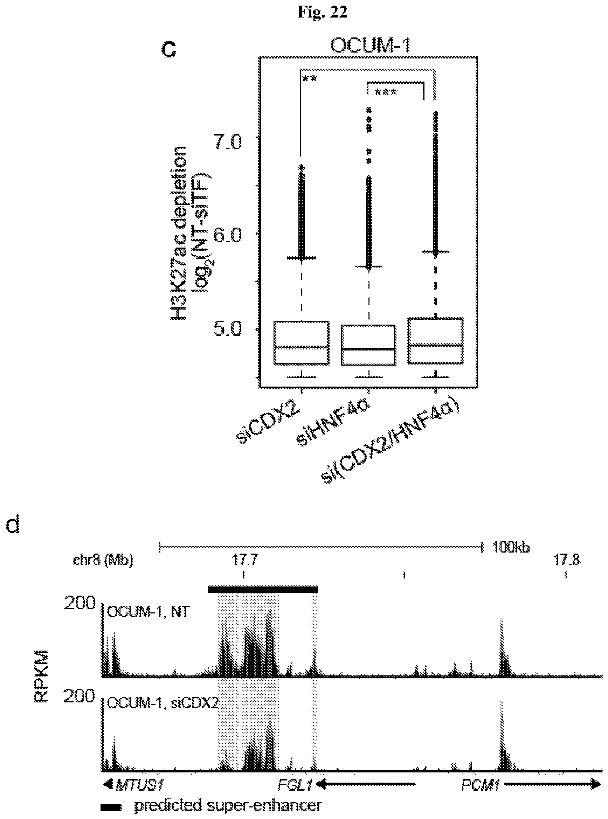

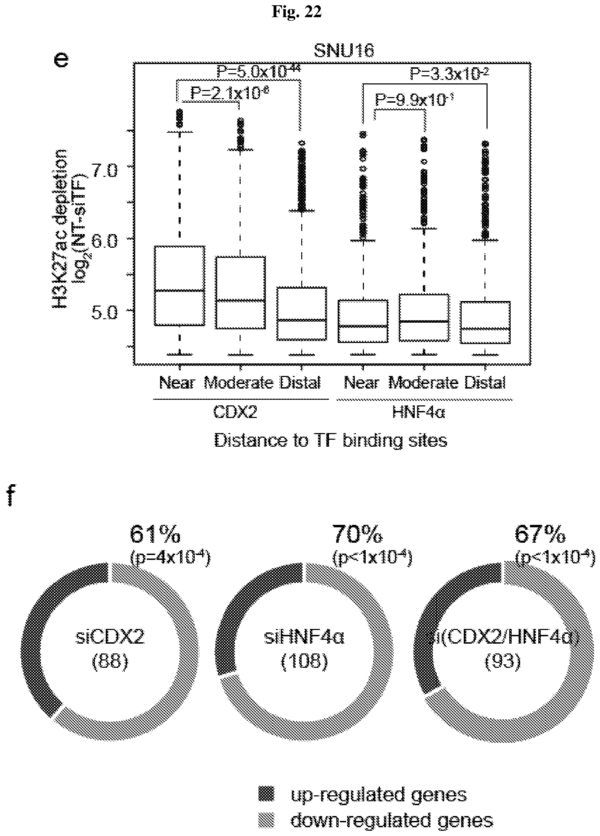

**Fig. 22** **Consequences of transcription factor-silencing on histone modifications and gene expression.**
   a. Differential CDX2 (left) and HNF4α (right) average binding signal analysis between recurrent somatic gain predicted super-enhancers and unaltered predicted super-enhancers. The predicted super-enhancers were also active in SNU16.
   b. Global changes in H3K27ac after silencing one or two transcription factors simultaneously (dark grey). Background changes are created from the difference between two controls (NT_{CDX2} and NT_{HNF4α}).
   c. Magnitude of H3K27ac depletion after silencing of transcription factor(s) in OCUM-1 cells.
   d. Visual example showing H3K27ac depletion in a predicted super-enhancer at the FGL1 locus after CDX2 silencing in OCUM-1 cells.
   e. Association between H3K27ac depletion in somatic gain predicted super-enhancers relative to CDX2 or HNF4α binding sites in SNU16 cells. Distances were uniformly distributed classified into three categories: near, moderate and distal to the binding sites. Statistical significance was evaluated using a one-sided Wilcoxon rank sum test.
   f. Gene expression associated with somatic gain predicted super-enhancers in OCUM-1 was examined after the silencing of single or double transcription factors simultaneously (NT-siTF). The percentage of genes showing changes in expression (FPKM difference > 0 as down-regulation; < 0 as up-regulation) is indicated. The proportion of down-regulated genes was tested using an empirical approach (see Methods).

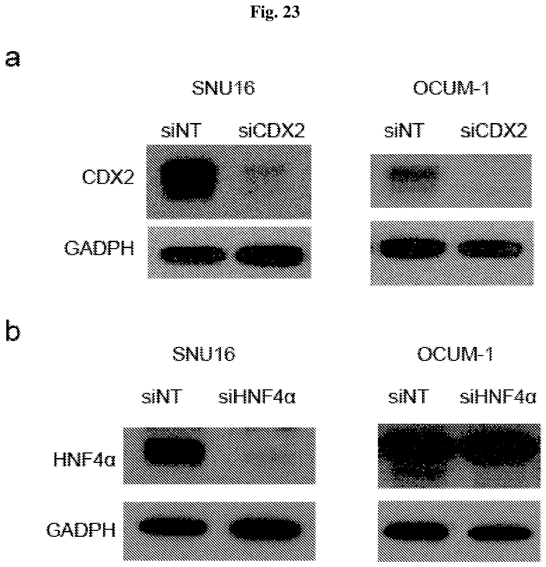

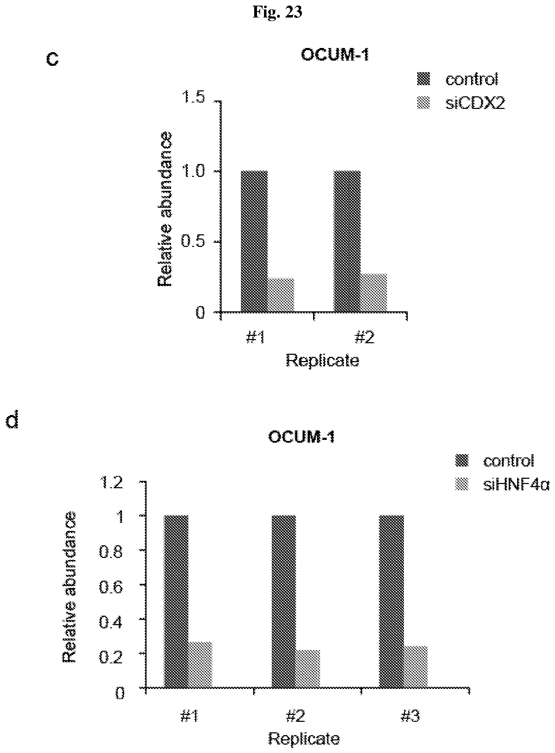

**Fig. 23** **CDX2, HNF4α knockdown efficiency by Western blotting and real time (RT) PCR.**
   a. Western blot measuring CDX2 protein abundance before (siNT) and after CDX2 knockdown (siCDX2) in SNU16 and OCUM-1 cells. GADPH protein abundance was used as a control.
   b. Western blot measuring HNF4α protein abundance before (siNT) and after HNF4α knockdown (siHNF4α) in SNU16 and OCUM-1 cells. GADPH protein abundance was used as a control.
   c. Relative RNA abundance of CDX2 to control was measured using RT-PCR in two replicates in OCUM-1 cells.
   d. Relative RNA abundance of HNF4α to control was measured using RT-PCR in three replicates in OCUM-1 cells.

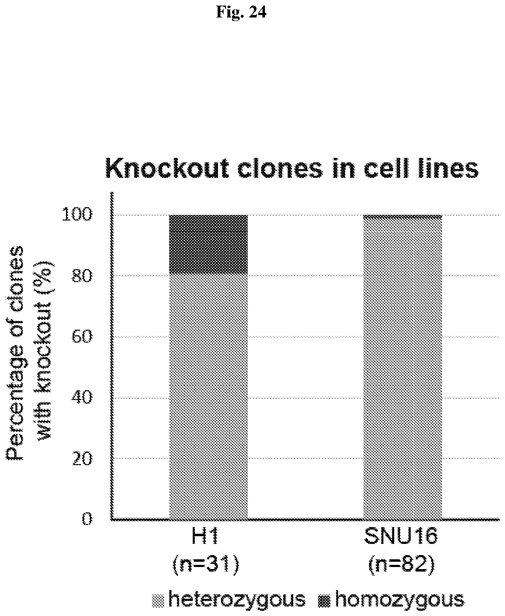

**Fig. 24** **Resistance of GC cells to CLDN4 e1 CRISPR deletion**. Higher rates of e1 homozygous deletion are observed in H1 ES vs SNU16 cells (20% vs 1%). The CLDN4 e1 subregion has been confirmed to be diploid in SNU16.

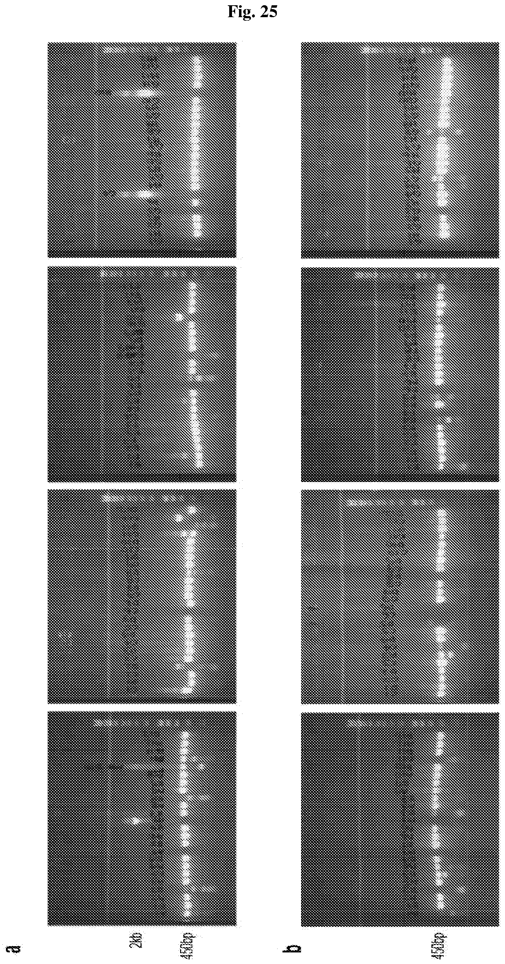

**Fig. 25** **Enhancer e1 deletion confirmation in 91 clones from SNU16 cells using PCR.**
   a. PCR bands resulting from using external primers.
   b. PCR bands resulting from using internal primers. Clones with homozygous deletion show ~450bp band using external primers, and no band using internal primer; Clones with heterozygous deletion show 450bp band using external and internal primers.

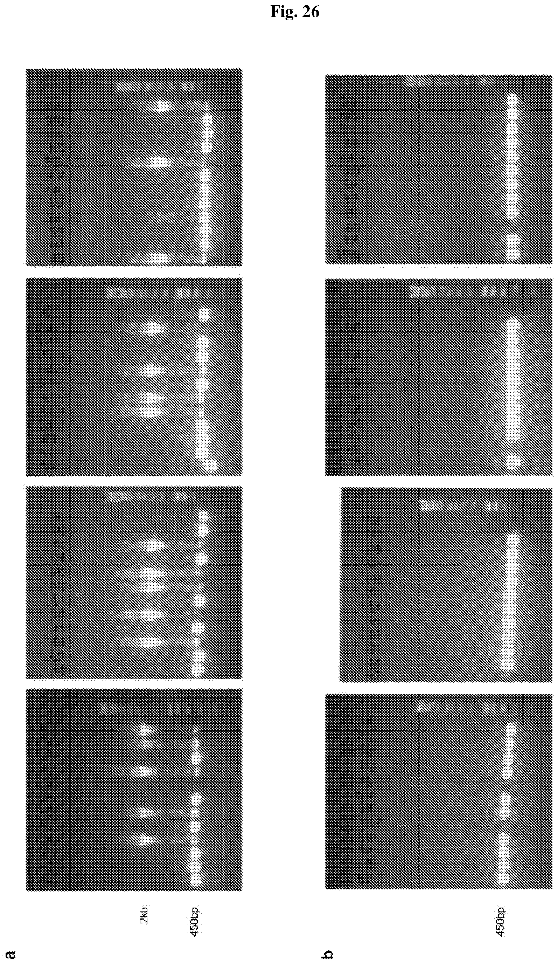

**Fig. 26** **Enhancer e1 deletion confirmation in 48 clones from H1 cells using PCR.**
   a. PCR bands resulting from using external primers.
   b. PCR bands resulting from using internal primers. Clones with homozygous deletion show ~450bp band using external primers, and no band using internal primer; Clones with heterozygous deletion show 450bp band using external and internal primers.

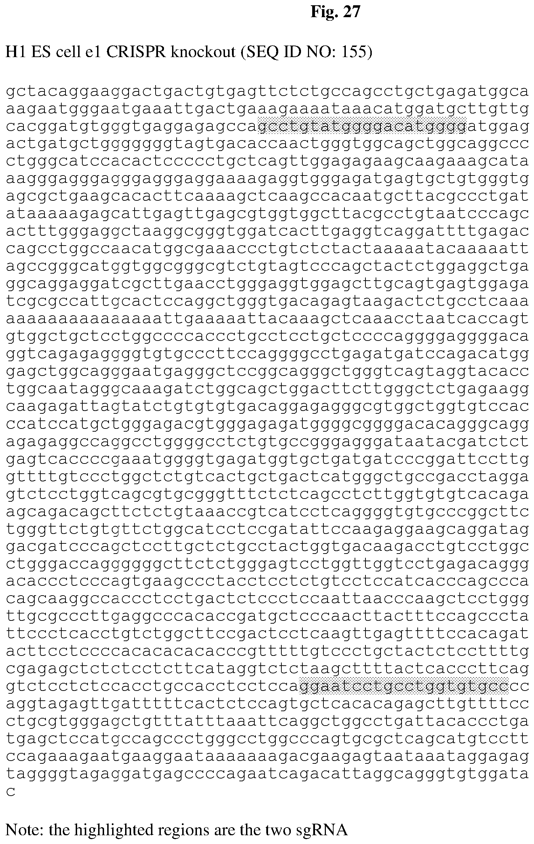

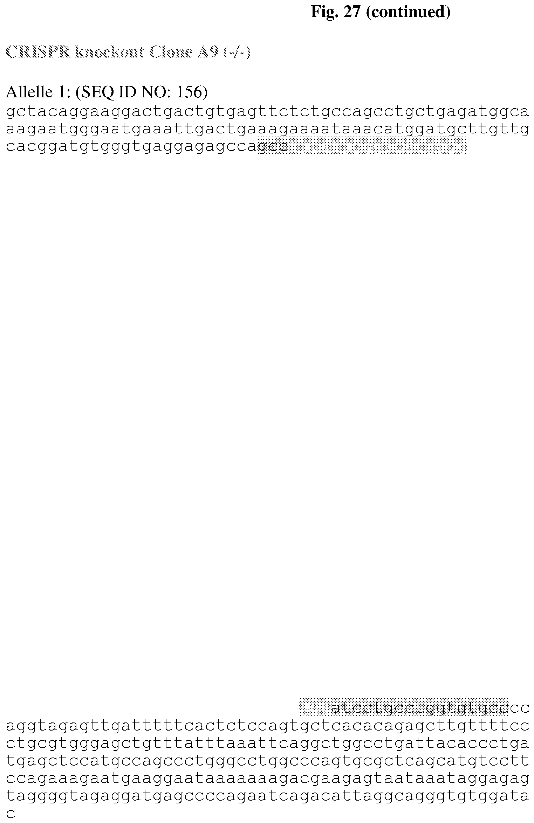

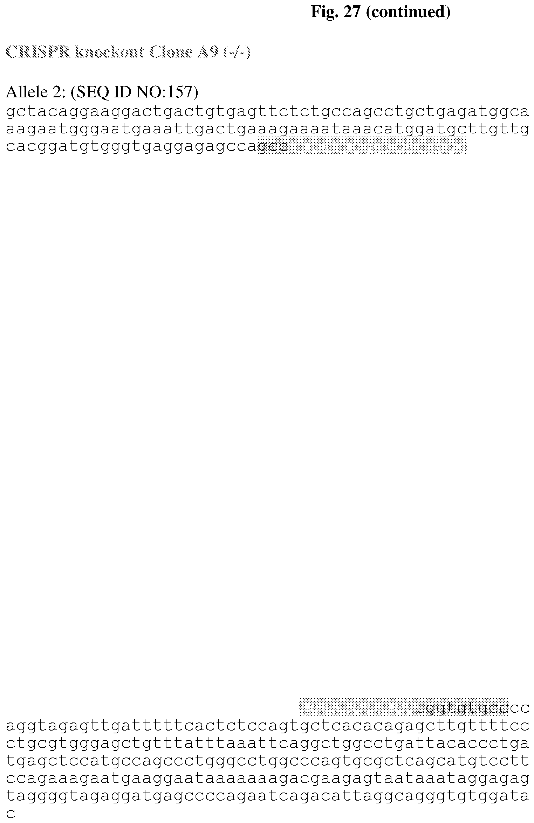

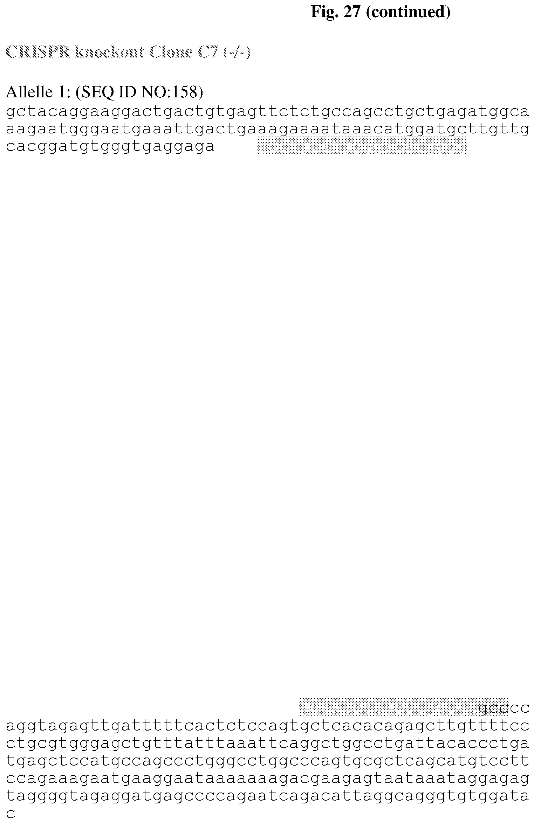

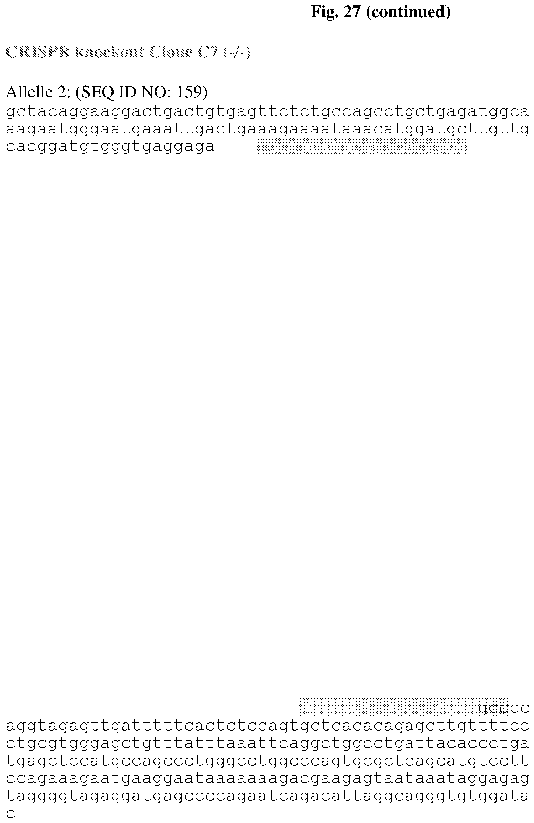

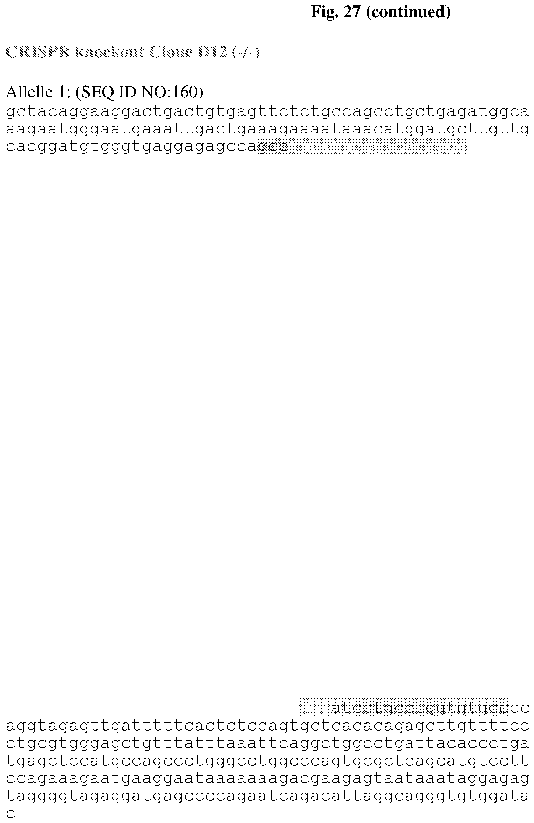

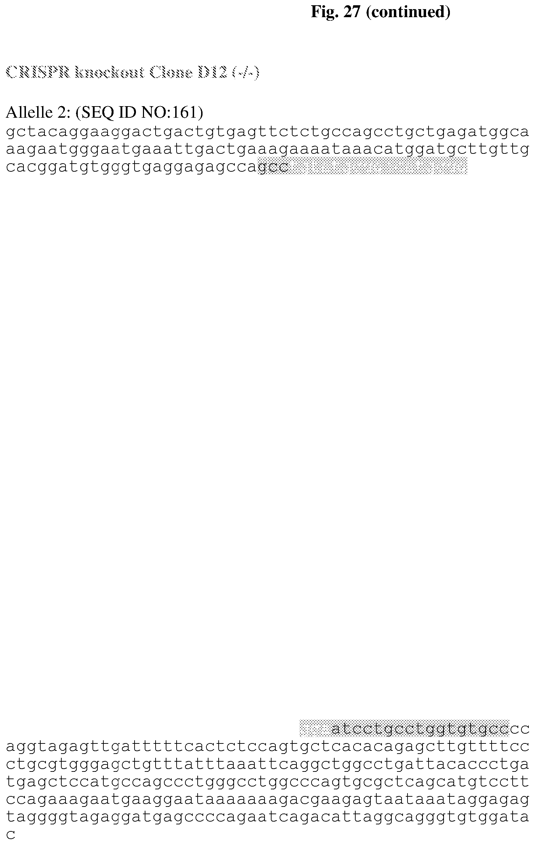

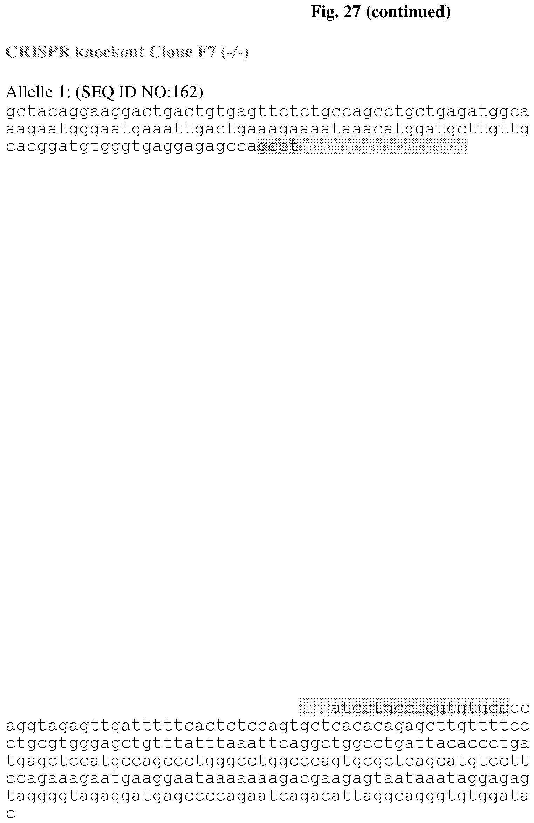

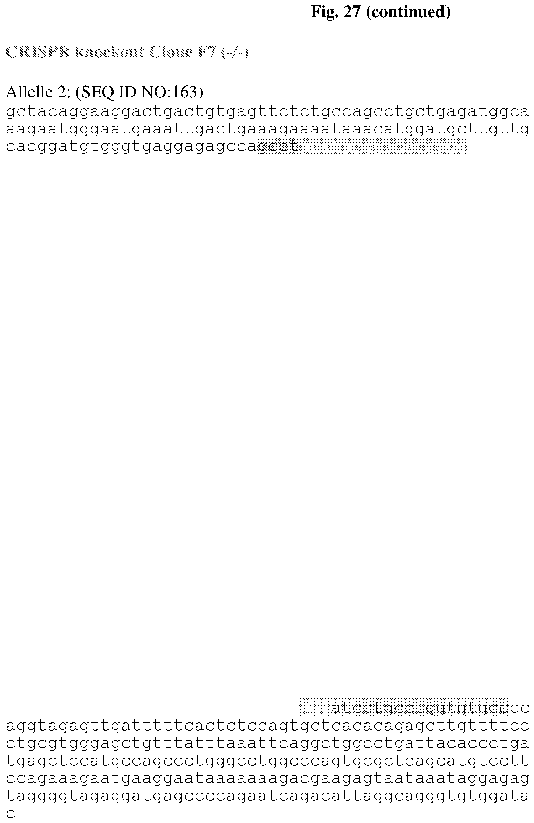

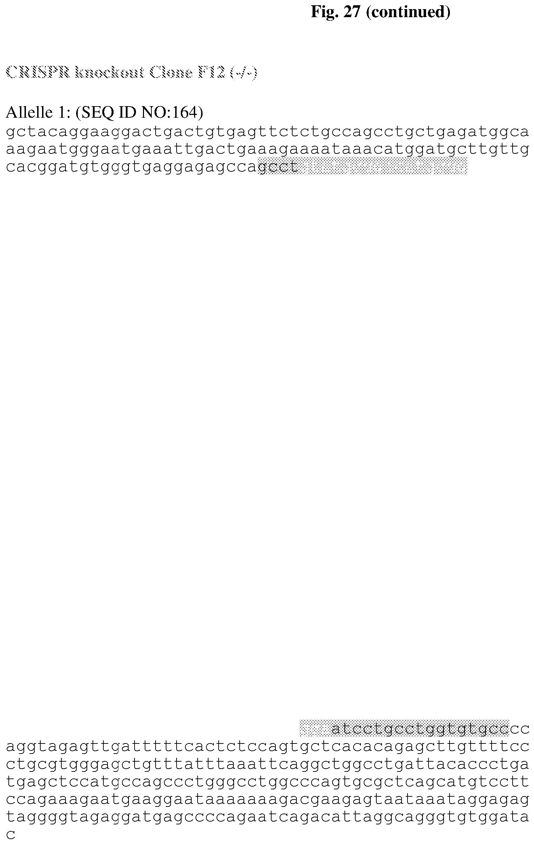

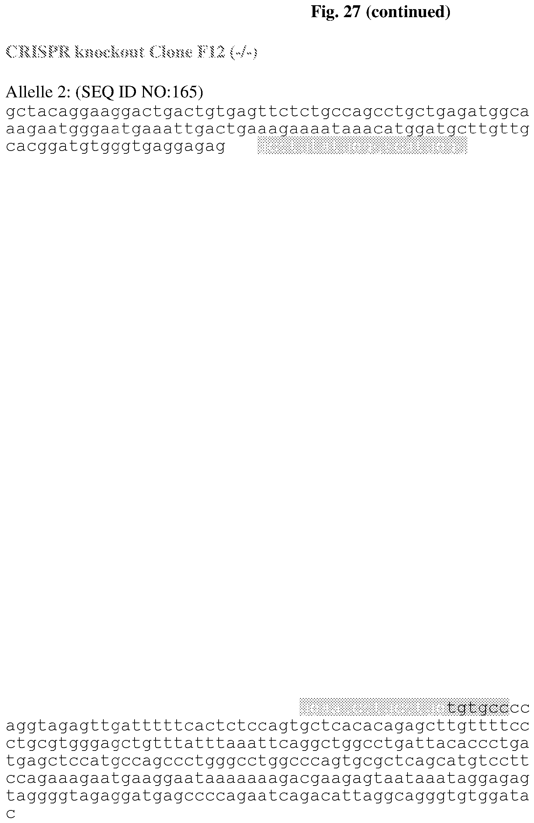

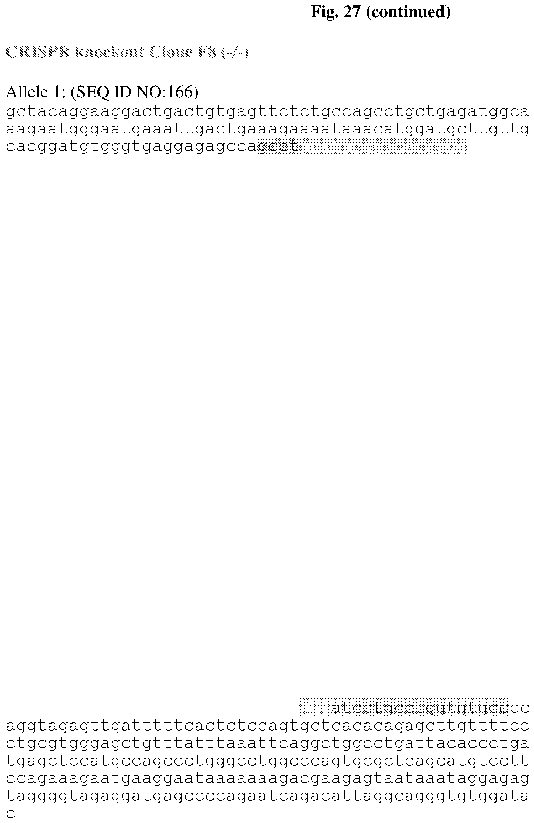

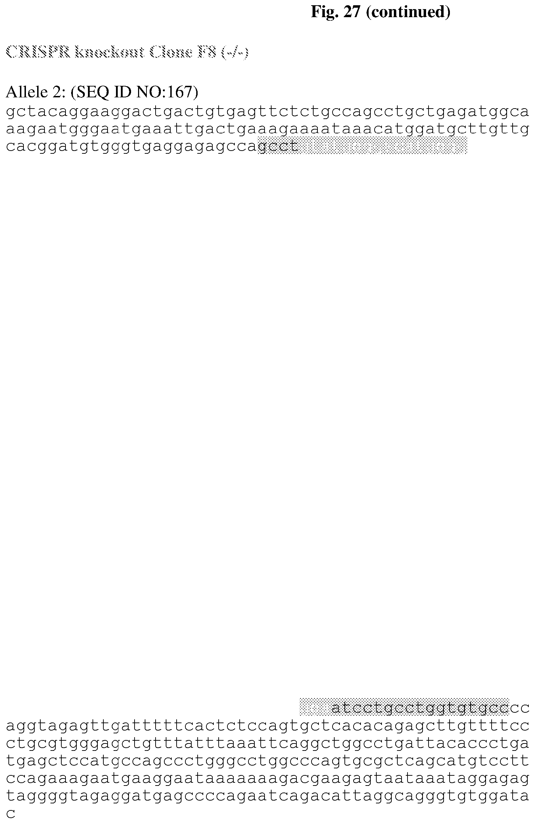

**Fig. 27** **Confirmation of homozygous e1-deletion in both alleles in H1 ES cells using Sanger sequencing**. The empty space indicates the deleted sub-sequence, the grey highlight indicates the sgRNA.

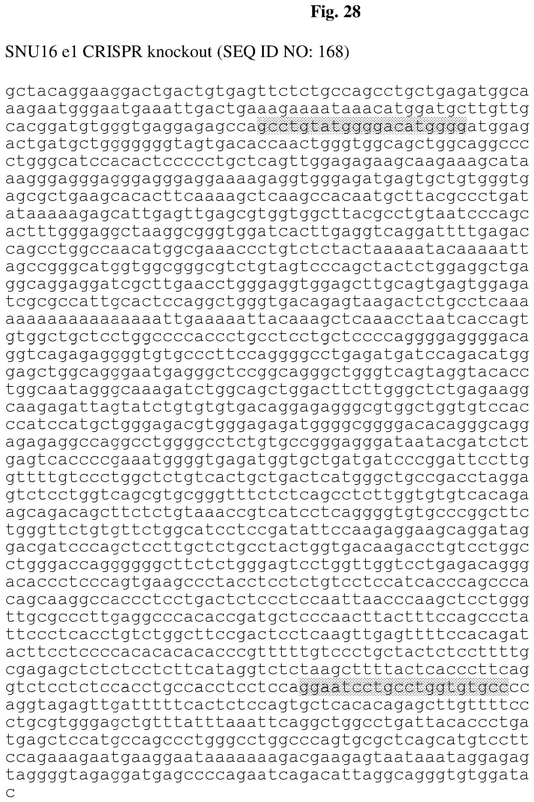

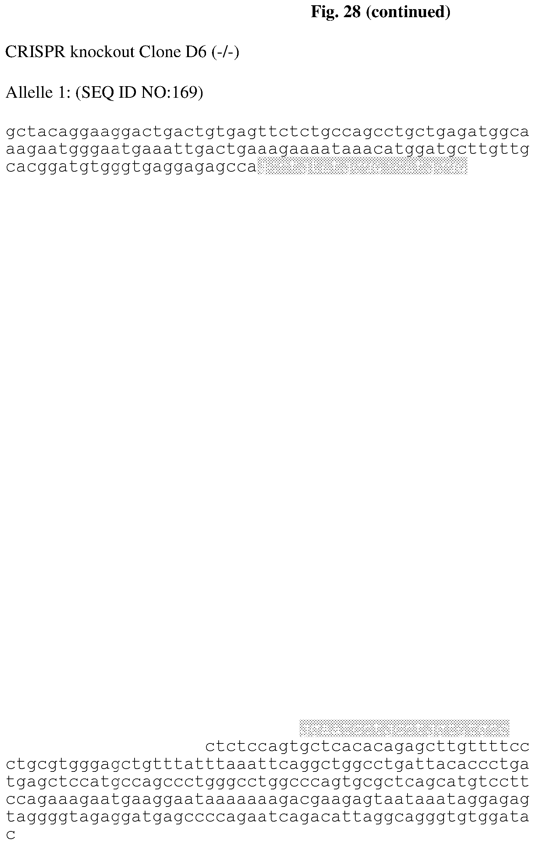

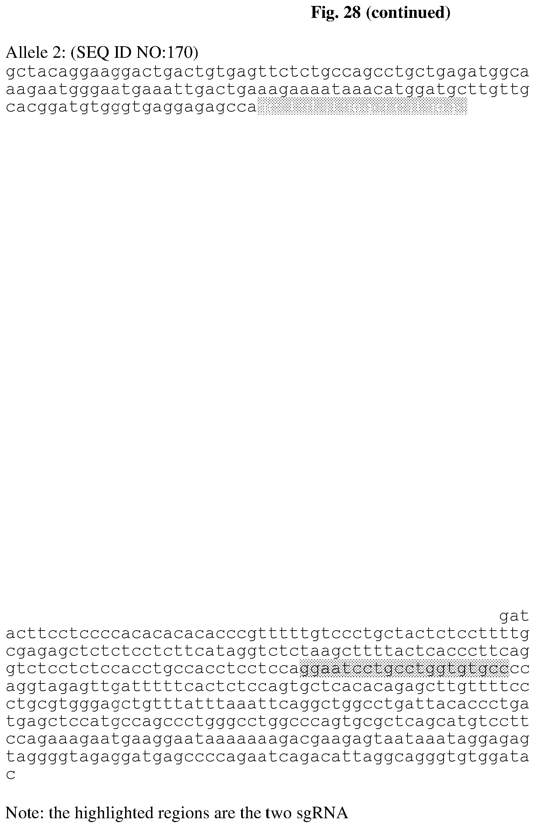

**Fig. 28** **Confirmation of homozygous e1-deletion in both alleles in SNU16 cells using Sanger sequencing.** The empty space indicates the deleted sub-sequence, the grey highlight indicates the sgRNA.

### DETAILED DESCRIPTION OF THE PRESENT INVENTION

In one aspect the present disclosure refers to a method for determining the presence or absence of at least one super-enhancer in a cancerous biological sample relative to a non-cancerous biological sample, comprising;
a) contacting a cancerous biological sample obtained from the subject with at least one antibody or antibodies specific for histone modification H3K27ac;
b) isolating nucleic acid from the cancerous biological sample, wherein the isolated nucleic acid comprises at least one region or regions specific to the histone modification H3K27ac;
c) mapping at least one enhancer using an annotated genome sequence based on a signal intensity of the histone modification H3K27ac, wherein the at least one enhancer is at least 2.5 kb from an annotated transcription start site;
d) mapping the at least one enhancer in the isolated nucleic acid against at least one enhancer in at least one reference nucleic acid sequence to identify at least one super-enhancer in the cancerous biological sample;
e) comparing the signal intensity of the at least one super-enhancer in the cancerous biological sample against a reference signal intensity of the at least one super-enhancer obtained from a non-cancerous biological sample; and
f) determining the presence or absence of the at least one super-enhancer in the cancerous biological sample based on the change in signal intensity of the at least one super-enhancer.

In one embodiment, the cancerous and non-cancerous biological sample may comprise a single cell, multiple cells, fragments of cells, body fluid or tissue. In one embodiment the cancerous and non-cancerous biological sample may be obtained from the same subject.

In one embodiment, the cancerous and non-cancerous biological sample are each obtained from different subjects.

The contacting step in accordance with the method as described herein may comprise at least one antibody specific for a histone modification. Examples of histone modification include but are not limited to H3K27ac, H3K4me3, H3K4me1 and H2BK20ac. In a preferred embodiment, the histone modification is H3K27ac.

The isolation step in accordance with the method as described herein may comprise isolating a nucleic acid from the cancerous biological sample by immunoprecipitation of chromatin. In one embodiment, the isolated nucleic acid comprises at least one region specific to histone modification. Examples of histone modification include but are not limited to H3K27ac, H3K4me3, H3K4me1 and H2BK20ac. In a preferred embodiment, the at least one regions specific to histone modification is a region specific to the histone modification H3K27ac.

The mapping step in accordance with the method as described herein may comprise using an annotated genome sequence based on a signal intensity of the histone modification. In one aspect of the disclosure, the histone modification is H327ac. In one aspect, the annotated genome sequence is a publicly available sequence. In one aspect, the annotated genome sequence is the Epigenome Roadmap. In another aspect, the annotated genome sequence is GENCODEv19.

The mapping step in accordance with the method as described herein may also comprise the at least one enhancer being at least 1 kb, at least 1.5 kb, at least 2 kb, at least 2.5 kb, at least 3 kb, at least 3.5kb, at least 4 kb, at least 4.5kb, at least 5 kb, at least 5.5 kb, at least 6 kb, at least 6.5 kb, at least 7 kb, at least 7.5 kb, at least 8 kb, at least 8.5 kb, at least 9 kb, at least 9.5 kb or at least 10 kb from an annotated transcription start site.

The method may further comprise mapping at least one enhancer in the isolated nucleic acid against at least one enhancer in at least one reference nucleic acid sequence to identify a to identify at least one super-enhancer in the cancerous biological sample.

In some aspects of the disclosure, the at least one reference nucleic acid sequence may comprise a nucleic acid sequence derived from: i) an annotated genome sequence; ii) a *de novo* transcriptome assembly; and/or iii) a non-cancerous nucleic acid sequence library or database.

In one embodiment, the at least one reference nucleic acid sequence is obtained from at least one cancer cell line.

In one embodiment, the signal intensity of the at least one super-enhancer is based on the Reads Per Kilobase of transcript per million (RPKM) value of the histone modification H3K27ac. In one embodiment, the signal intensity of the at least one super-enhancer is based on the Fragments Per Kilobase of transcript per Million (FPKM) value of the histone modification H3K27ac.

In one embodiment, the at least one super-enhancer in the cancerous biological sample is identified using the ROSE (Ranking of Super Enhancer) algorithm.

In some aspects of the disclosure, the at least one super-enhancer in the cancerous biological sample comprises at least one, at least two, at least three, at least four, at least five, at least six, at least seven, at least eight, at least nine or at least ten nucleic acid base pair overlapping with the at least one enhancer in the at least one reference nucleic acid sample.

In a preferred embodiment, the at least one super-enhancer in the cancerous biological sample comprises at least one nucleic acid base pair overlapping with the at least one enhancer in the at least one reference nucleic acid sample.

In one embodiment, the step of determining the presence or absence of the at least one super-enhancer may comprise determining that the RKPM value for the at least one super enhancer in the cancerous biological is: i) greater than 1.5-fold change, greater than a 2-fold change, greater than a 3-fold change, greater than a 4-fold change, greater than a 5-fold change, greater than a 6 fold change, greater than a 7-fold change, greater than an 8-fold change, greater than a 9-fold change or greater than a 10-fold change in RPKM value relative to the RPKM value of the at least one super-enhancer obtained from the non-cancerous biological sample; and ii) an absolute difference greater than a 0.5 RPKM, greater than a 1.0 RPKM, greater than a 1.5 RPKM, greater than a 2.0 RPKM, greater than a 2.5 RPKM, greater than a 3.0 RPKM, greater than a 3.5 RPKM, greater than a 4.0 RPKM, greater than a 4.5 RPKM or greater than a 5.0 RPKM relative to the RPKM value of the at least one super-enhancer obtained from the non-cancerous biological sample.

In a preferred embodiment, the step of determining the presence or absence of the at least one super-enhancer comprises determining that the RKPM value for the at least one super enhancer in the cancerous biological sample is: i) greater than a 2-fold change in RPKM value relative to the RPKM value of the at least one super-enhancer obtained from the non-cancerous biological sample; and ii) an absolute difference greater than a 0.5 RPKM relative to the RPKM value of the at least one super-enhancer obtained from the non-cancerous biological sample.

In one embodiment, an increase in RPKM value from the cancerous biological sample relative to the RPKM value of the non-cancerous biological sample is indicative of the presence of the at least one super-enhancer in the cancerous biological sample.

In one embodiment, a decrease in RPKM value from the cancerous biological sample relative to the RPKM value of the non-cancerous biological sample is indicative of the absence of the at least one super-enhancer in the cancerous biological sample.

In some aspects of the disclosure, the step of determining the presence or absence of the at least one super-enhancer may comprise determining that the FKPM value for the at least one super enhancer in the cancerous biological is: i) greater than a 1.5-fold change, greater than a 2-fold change, greater than a 3-fold change, greater than a 4-fold change, greater than a 5-fold change, greater than a 6 fold change, greater than a 7-fold change, greater than an 8-fold change, greater than a 9-fold change or greater than a 10-fold change in FPKM value relative to the FPKM value of the at least one super-enhancer obtained from the non-cancerous biological sample; and ii) an absolute difference greater than a 0.5 FPKM, greater than a 1.0 FPKM, greater than a 1.5 FPKM, greater than a 2.0 FPKM, greater than a 2.5 FPKM, greater than a 3.0 FPKM, greater than a 3.5 FPKM, greater than a 4.0 FPKM, greater than a 4.5 FPKM or greater than a 5.0 FPKM relative to the FPKM value of the at least one super-enhancer obtained from the non-cancerous biological sample.

In a preferred embodiment, the step of determining the presence or absence of the at least one super-enhancer comprises determining that the FKPM value for the at least one super enhancer in the cancerous biological sample is: i) greater than a 2-fold change in FPKM value relative to the FPKM value of the at least one super-enhancer obtained from the non-cancerous biological sample; and ii) an absolute difference greater than a 0.5 FPKM relative to the FPKM value of the at least one super-enhancer obtained from the non-cancerous biological sample.

In one embodiment, an increase in FPKM value from the cancerous biological sample relative to the FPKM value of the non-cancerous biological sample is indicative of the presence of the at least one super-enhancer in the cancerous biological sample.

In one embodiment, a decrease in FPKM value from the cancerous biological sample relative to the FPKM value of the non-cancerous biological sample is indicative of the absence of the at least one super-enhancer in the cancerous biological sample.

In some aspects of the disclosure, the at least one super-enhancer is positioned within 500 kb, 600 kb, 700 kb, 800 kb, 900 kb, 1000kb, 1100 kb, 1200 kb, 1300 kb, 1400 kb, 1500 kb or 2000 kb to a gene transcription start site. In a preferred embodiment, the at least one super-enhancer is positioned within 1000kb to a gene transcription start site.

In one embodiment, the gene is a cancer associated gene, an angiogenesis gene, a cell proliferation gene, a cell invasion gene, a gene associated with genome instability, a cell death resistance gene, a cellular energetics gene, a cell cycle gene or a tumour-promoting gene.

In some embodiments, the gene is selected from the group consisting of CLDN4, ABHD11, WBSCR28, ATAD2, KLH38, WDYHV1, CDH17, CCAT1, CLDN1, SMURF1, GDPD5, ADAMTS12, ASCL2, ASPM, ATP11A, AURKA, CAMK2N1, CBX2, CCNE1, CD9, CDC25B, CDCA7, CDK1, CXCL1, E2F7, ECT2, LAMC2, NID2, PMEPA1, RARRES1, RFC3, SLC39A10, TFAP2A, TMEM158, LINC00299 and a combination thereof.

In one embodiment, the cancerous biological sample is a gastric cancer.

In another aspect of the disclosure, there is provided a method for determining the presence of at least one cancer-associated super-enhancer in a subject, comprising:
a) contacting a cancerous biological sample obtained from the subject with at least one antibody or antibodies specific for histone modification H3K27ac;
b) isolating nucleic acid from the cancerous biological sample, wherein the isolated nucleic acid comprises at least one region or regions specific to the histone modification H3K27ac;
c) mapping at least one enhancer using an annotated genome sequence based on a signal intensity of the histone modification H3K27ac, wherein the at least one enhancer is at least 2.5 kb from an annotated transcription start site;
d) mapping the at least one enhancer in the isolated nucleic acid against at least one enhancer in at least one reference nucleic acid sequence to identify at least one super-enhancer in the cancerous biological sample;
e) comparing the signal intensity of the at least one super-enhancer in the cancerous biological sample against a reference signal intensity of the at least one super-enhancer obtained from a non-cancerous biological sample; and
f) determining the presence of at least one cancer-associated super-enhancer in a subject based on the change in signal intensity of the at least one super-enhancer,
wherein an increased signal intensity of the at least one super-enhancer in the cancerous biological sample relative to the non-cancerous biological sample is indicative of the presence of at least one cancer-associated super-enhancer.

In another aspect of the disclosure, there is provided a biomarker for detecting cancer in a subject, the biomarker comprising biomarker for detecting cancer in a subject, the biomarker comprising at least one super-enhancer having increased signal intensity of H3K27ac in a cancerous biological sample relative to a normal non-cancerous biological sample, or at least one super-enhancer associated with an increase in cancer-associated transcription factor binding sites relative to unaltered super-enhancers, or both. In some aspects, the cancer-associated transcription factor binding sites are gastric cancer-associated transcription factor binding sites.

In some embodiments, the gastric cancer-associated transcription factor is selected from the group consisting of CDX2, KLF5 and HNF4α. In some embodiments, the gastric cancer-associated transcription factor is selected from the group consisting of CDX2, KLF5, HNF4α and combinations thereof.

In another aspect of the disclosure, there is provided a method for determining the prognosis of cancer in a subject, comprising:
a) contacting a cancerous biological sample obtained from the subject with at least one antibody or antibodies specific for histone modification H3K27ac;
b) isolating nucleic acid from the cancerous biological sample, wherein the isolated nucleic acid comprises at least one region or regions specific to the histone modification H3K27ac;
c) mapping at least one enhancer using an annotated genome sequence based on a signal of the histone modification H3K27ac, wherein the at least one enhancer is at least 2.5 kb from an annotated transcription start site;
d) mapping the at least one enhancer in the isolated nucleic acid against at least one enhancer in at least one reference nucleic acid sequence to identify at least one super-enhancer in the cancerous biological sample;
e) comparing the signal intensity of the at least one super-enhancer in the cancerous biological sample against a reference signal intensity of the at least one super-enhancer obtained from a non-cancerous biological sample; and
f) determining the presence or absence of at least one cancer-associated super-enhancer in a subject based on the change in signal intensity of the at least one super-enhancer in the cancerous biological sample relative to the non-cancerous biological sample,
wherein the presence or absence of at least one cancer-associated super-enhancer is indicative of the prognosis of the cancer in the subject.

In one embodiment, the presence of the at least one cancer-associated super-enhancer in the cancerous biological sample is indicative of a poor prognosis of cancer survival in a subject.

In one embodiment, the absence of the at least one cancer-associated super-enhancer in the cancerous biological sample is indicative of an improved prognosis of cancer survival in a subject.

In one embodiment, the at least one cancer-associated super-enhancer is associated with one or more of a cell invasion gene, an angiogenesis gene or a cell death resistance gene, a cancer associated gene, a cell proliferation gene, a gene associated with genome instability, a cellular energetics gene, a cell cycle gene or a tumour-promoting gene.

In one embodiment, the at least one cancer-associated super-enhancer is associated with a gene selected from the group consisting of CLDN4, ABHD11, WBSCR28, ATAD2, KLH38, WDYHV1, CDH17, CCAT1, CLDN1, SMURF1, GDPD5, ADAMTS12, ASCL2, ASPM, ATP11A, AURKA, CAMK2N1, CBX2, CCNE1, CD9, CDC25B, CDCA7, CDK1, CXCL1, E2F7, ECT2, LAMC2, NID2, PMEPA1, RARRES1, RFC3, SLC39A10, TFAP2A, TMEM158, LINC00299 and a combination thereof.

In another aspect of the disclosure, there is provided method of determining the susceptibility of a subject to cancer or a gastrointestinal disease, comprising:
a) contacting a biological sample obtained from the subject with at least one antibody or antibodies specific for histone modification H3K27ac;
b) isolating nucleic acid from the biological sample, wherein the isolated nucleic acid comprises at least one region or regions specific to the histone modification H3K27ac;
c) mapping at least one enhancer using an annotated genome sequence based on a signal intensity of the histone modification H3K27ac, wherein the at least one enhancer is at least 2.5 kb from an annotated transcription start site;
d) mapping the at least one enhancer in the isolated nucleic acid against at least one enhancer in at least one reference nucleic acid sequence to identify at least one super-enhancer in the biological sample;
e) comparing the signal intensity of the at least one super-enhancer in the biological sample against a reference signal of the at least one super-enhancer obtained from a control biological sample; and
f) determining the presence or absence of the at least one super-enhancer based on the change in signal intensity of the at least one super-enhancer;
g) mapping the presence or absence of the at least one super-enhancer against a reference genome sequence comprising cancer or gastrointestinal disease associated SNPs,
wherein the presence or absence of the at least one super-enhancer associated with one or more cancer or gastrointestinal disease associated SNPs is indicative of the subjects susceptibility to cancer or a gastrointestinal disease.

In one aspect of the disclosure, the gastrointestinal disease is selected from one or more of achalasia, Barrett's oesophagus, liver cirrhosis, biliary cirrhosis, coeliac disease, colorectal polyps, Crohn's disease, diverticulosis, diverticulitis, fatty liver, gallstones, gastritis, Helicobacter pylori, hemochromatosis, hepatitis, irritable bowel syndrome, microscopic colitis, oesophageal cancer, pancreatitis, peptic ulcers, reflux oesophagitis, ulcerative colitis, colorectal cancer and constipation.

In one aspect of the disclosure, the cancer is selected from one or more of gastric cancer, oesophageal cancer, colorectal cancer, breast cancer and prostate cancer.

In another aspect of the present invention, there is provided a method for modulating the activity of at least one cancer-associated super-enhancer in a cell, comprising administering an inhibitor of CDX2 and/or HNF4α to the cell.

In one embodiment, the inhibitor is a small interfering RNA (siRNA). In another aspect of the disclosure, the inhibitor is short hairpin RNA (shRNA).

In one embodiment, the inhibitor is a small molecule or antibody.

In one embodiment, the inhibitor is metformin.

In one aspect of the disclosure, the activity of the at least one cancer-associated super-enhancer in a cell may be modulated by the CRISPR genome editing system. In another aspect, the CRISPR genome editing system is CRISPR/Cas9.

In one aspect of the disclosure, the activity of the at least one cancer-associated super-enhancer in a cell may be inhibited by the CRISPR genome editing system. In another aspect, the CRISPR genome editing system is CRISPR/Cas9.

In another aspect of the disclosure, there is provided a biomarker comprising at least one super-enhancer having increased signal intensity of H3K27ac in a cancerous biological sample relative to a normal non-cancerous biological sample, or at least one super-enhancer associated with an increase in cancer-associated transcription factor binding sites relative to unaltered super-enhancers, or both, for use in detecting cancer in a subject.

In another aspect of the disclosure, there is provided a use of a biomarker comprising at least one super-enhancer having increased signal intensity of H3K27ac in a cancerous biological sample relative to a normal non-cancerous biological sample, or at least one super-enhancer associated with an increase in cancer-associated transcription factor binding sites relative to unaltered super-enhancers, or both in the manufacture of a medicament for detecting cancer in a subject.

In another aspect of the invention, there is provided an inhibitor of CDX2 and/or HNF4α for use in modulating the activity of at least one cancer-associated super-enhancer in a cell.

In another aspect of the disclosure, there is provided a use of an inhibitor of CDX2 and/or HNF4α in the manufacture of a medicament for modulating the activity of at least one cancer-associated super-enhancer in a cell.

In one aspect, there is provided a method of predicting cancer cell survival or cancer cell viability in a cancerous biological sample obtained from a subject comprising:
a) contacting the cancerous biological sample with at least one antibody specific for histone modification H3K27ac;
b) isolating nucleic acid from the cancerous biological sample, wherein the isolated nucleic acid comprises at least one region specific to the histone modification H3K27ac;
c) mapping at least one enhancer using an annotated genome sequence based on a signal intensity of the histone modification H3K27ac, wherein the at least one enhancer is at least 2.5 kb from an annotated transcription start site;
d) mapping the at least one enhancer in the isolated nucleic acid against at least one enhancer in at least one reference nucleic acid sequence to identify at least one super-enhancer in the cancerous biological sample;
e) comparing the signal intensity of the at least one super-enhancer in the cancerous biological sample against a reference signal intensity of the at least one super-enhancer obtained from a non-cancerous biological sample; and
f) determining the presence of at least one cancer-associated super-enhancer in a subject based on the change in signal intensity of the at least one super-enhancer,
wherein an increased signal intensity of the at least one super-enhancer in the cancerous biological sample relative to the non-cancerous biological sample is predictive of cancer cell survival or cancer cell viability.

The invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

### EXPERIMENTAL SECTION

### Methods

### Primary Tissue Samples and Cell Lines

Primary patient samples were obtained from the SingHealth tissue repository with approvals from the SingHealth Centralised Institutional Review Board and signed patient informed consent. 'Normal' (i.e., non-malignant) samples used in this study refers to samples harvested from the stomach, from sites distant from the tumour and exhibiting no visible evidence of tumour or intestinal metaplasia/dysplasia upon surgical assessment. Tumor samples were confirmed by cryosectioning to contain >40% tumor cells. FU97, MKN7, OCUM-1 and RERF-GC-1B cell lines were obtained from the Japan Health Science Research Resource Bank. KATO-III and SNU16 cells were obtained from the American Type Culture Collection. NCC-59 was obtained from the Korean Cell Line Bank. YCC3, YCC7, YCC21, YCC22 were gifts from Yonsei Cancer Centre, South Korea. Cell line identities were confirmed by STR DNA profiling performed at the Centre for Translational Research and Diagnostics (Cancer Science Institute of Singapore, Singapore). STR profiles were assessed according to the standard ANSI/ATCC ASN-0002-2011 nomenclature, and the profiles of our cell lines showed >80% similarity to the reference databases. MKN7 cells - one commonly misidentified line by ICLAC (http://iclac.org/databases/cross-contaminations/) was confirmed by showing a perfect match (100%) with the MKN7 reference profile in the Japanese Collection of Research Bioresources Cell Bank. MycoAlert^{™} Mycoplasma Detection Kits (Lonza) and MycoSensor qPCR Assay Kits (Agilent Technologies) were used to detect mycoplasma contamination. All cell lines were negative for mycoplasma contamination. For this study, OCUM-1 and SNU16 cells were selected as main cell line models for two reasons. First, OCUM-1 and SNU16 cells were originally isolated from patients with poorly differentiated gastric adenocarcinoma, and the majority of primary GCs in this study are poorly differentiated (63%). Second, OCUM-1 and SNU16 have been previously used as gastric cancer (GC) models in many other published studies, and are thus regarded as accepted GC models in the field. Thus, OCUM-1 and SNU16 were used as consistent cell line models for several experiments, including Capture-C, 4C, enhancer CRISPR, transcription factor binding, and transcription factor knockdown.

### Nano ChIPseq

Nano-ChIPseq was performed as described with slight modifications. For primary tissues, fresh-frozen cancer and normal tissues were dissected using a razor blade in liquid nitrogen to obtain ~5mg sized piece for each ChIP. Tissue pieces were fixed in 1% formaldehyde/PBS buffer for 10 min at room temperature. Fixation was stopped by addition of glycine to a final concentration of 125 mM. Tissue pieces were washed 3 times with TBSE buffer. For cell lines, 1 million fresh harvested cells were fixed in 1% formaldehyde/medium buffer for 10 minutes (min) at room temperature. Fixation was stopped by addition of glycine to a final concentration of 125 mM. Fixed cells were washed 3 times with TBSE buffer, and centrifuged (5,000 r.p.m., 5 min). Pelleted cells and pulverized tissues were lysed in 100 µ1 1% SDS lysis buffer and sonicated to 300-500bp using a Bioruptor (Diagenode). ChIP was performed using the following antibodies: H3K4me3 (07-473, Millipore); H3K4me1 (ab8895, Abcam); H3K27ac (ab4729, Abcam).

After recovery of ChIP and input DNA, whole-genome-amplification was performed using the WGA4 kit (Sigma-Aldrich) and BpmI-WGA primers. Amplified DNAs were purified using PCR purification columns (QIAGEN) and digested with BpmI (New England Biolabs) to remove WGA adapters. 30ng of amplified DNA was used for each sequencing library preparation (New England Biolabs). 8 libraries were multiplexed (New England Biolabs) and sequenced on 2 lanes of Hiseq2500 (Illumina) to an average depth of 20-30 million reads per library.

### Sequence Mapping and ChIP-seq Density Analysis

Sequence reads were mapped against human reference genome (hg19) using Burrows-Wheeler Aligner (BWA-MEM, version 0.7.0), after trimming the first and the last 10 bases prior to alignment. Only high quality mapped reads (MAPQ≥10) were retained for downstream analyses. The MAPQ value (≥10) was chosen as this has i) been previously reported to be a good value to use for good/confident read mapping; ii) MAPQ≥10 has also been indicated by the developers of the BWA-algorithm to be a suitable threshold to use for confident mappings using their software; and iii) studies assessing various algorithms for read alignment have also shown that mapping quality scores do not correlate well with the likelihood of read mapping being true/accurate and have shown that the level of accuracy obtained for mapping accuracy plateaus between a 10-12 MAPQ threshold. This study focuses on *recurrent* predicted enhancers and super-enhancers that are reliably detected in multiple samples, which increases the robustness of the analysis. Sequencing coverage was computed using MEDIPS with a 50bp window size and read length extension to 200bp. Peaks with significant ChIP enrichment (FDR<5%) relative to input libraries were detected using CCAT (version 3). Peak densities within a region were computed by counting the total number of mapped reads normalized by the library and region size, a metric equivalent to reads per million mapped reads per kilobases (RPKM). This normalization method adjusts for biases due to the higher probability of reads falling into longer regions and has been applied in previous studies. This study elected to apply RPKM-based normalization to make the study comparable to these other studies. To account for background signals, read densities of each ChIP library were corrected against the corresponding input library. Read densities across samples were corrected for potential batch effects (eg date of ChIP assay) using COMBAT and to ensure equal sample variation. Of 17,360 recurrent predicted enhancers detected in two or more cell lines, 98% were present in at least one primary sample (normal or GC).

### Quality Control Assessment of Nano-ChIPseq Data

Qualities of the ChIP libraries (H3K27ac, H3K4me3 and H3K4me1) were assessed using two different methods. First, ChIP qualities, particularly H3K27ac and H3K4me3, were estimated by interrogating their enrichment levels at annotated promoters of protein-coding genes. Specifically, the study computed median read densities of input and input-corrected ChIP signals at 1,000 promoters associated with highly expressed protein-coding genes. For each sample, read density ratios of H3K27ac over input were compared as a surrogate of data quality, retaining only those samples where the H3K27ac/input ratio was greater than 4-fold. Using this criteria, 48 out of 50 H3K27ac samples (GC lines and primary samples) exhibited greater than 4-fold enrichment, indicating successful enrichment. A similar analysis was also performed for the H3K4me3 libraries (a promoter mark), and all 42 libraries satisfied this quality control criteria. Second, CHANCE (CHip-seq ANalytics and Confidence Estimation), a software for ChIP-seq quality control and protocol optimization that indicates whether a library shows successful or weak enrichment was used. It was found that the large majority (85%) of samples in the study exhibited successful enrichment as assessed by CHANCE. The assessment status for each library, as assessed by both methods, are reported in Table 1.

**Table 1: Mapping statistics and quality assessment of histone ChIP-seq libraries.**

| **Sample Name** | **LibraryID** | **Histone Modification** | **Total Mapped Reads (MAPQ>10)** | **Peak (FDR <5%, CCAT)** | **CHANCE** | **ChIP enrichment at promoters** (> **4 fold)** |
|---|---|---|---|---|---|---|
| T2000639 | CHG018 | H3K27ac | 68,132,545 | 21,614 | successful | yes |
| N2000639 | CHG022 | H3K27ac | 56,232,238 | 29,868 | successful | yes |
| T2000721 | CHG026 | H3K27ac | 63,718,156 | 43,271 | successful | yes |
| N2000721 | CHG030 | H3K27ac | 69,012,771 | 23,523 | successful | no |
| T2000986 | CHG034 | H3K27ac | 59,552,351 | 27,263 | successful | yes |
| N2000986 | CHG038 | H3K27ac | 63,262,652 | 25,606 | successful | yes |
| N980437 | CHG089 | H3K27ac | 24,841,454 | 14,717 | successful | yes |
| T980437 | CHG093 | H3K27ac | 21,924,449 | 43,624 | weak | yes |
| N980097 | CHG097 | H3K27ac | 20,969,770 | 5,437 | weak | no |
| T980097 | CHG101 | H3K27ac | 19,607,835 | 73,528 | successful | yes |
| T990489 | CHG279 | H3K27ac | 17,247,914 | 52,036 | successful | yes |
| N990489 | CHG284 | H3K27ac | 12,677,450 | 37,013 | weak | yes |
| T76629543 | CHG318 | H3K27ac | 12,912,838 | 12,636 | successful | yes |
| N76629543 | CHG324 | H3K27ac | 16,361,856 | 30,714 | weak | yes |
| T990068 | CHG439 | H3K27ac | 28,128,868 | 92,041 | successful | yes |
| N990068 | CHG443 | H3K27ac | 23,707,529 | 32,800 | successful | yes |
| T2000085 | CHG447 | H3K27ac | 28,975,716 | 67,464 | successful | yes |
| N2000085 | CHG451 | H3K27ac | 25,633,117 | 93,432 | successful | yes |
| T980401 | GCC003 | H3K27ac | 61,492,830 | 118,309 | successful | yes |
| N980401 | GCC007 | H3K27ac | 42,148,653 | 106,781 | successful | yes |
| T980447 | GCC011 | H3K27ac | 69,872,340 | 109,853 | successful | yes |
| N980447 | GCC015 | H3K27ac | 41,098,081 | 131,274 | successful | yes |
| T2001206 | GCC019 | H3K27ac | 47,707,006 | 103,980 | successful | yes |
| N2001206 | GCC023 | H3K27ac | 40,279,991 | 103,925 | successful | yes |
| T980436 | GCC027 | H3K27ac | 42,255,541 | 99,869 | successful | yes |
| N980436 | GCC031 | H3K27ac | 18,685,156 | 104,102 | successful | yes |
| T980417 | GCC035 | H3K27ac | 31,413,426 | 72,174 | successful | yes |
| N980417 | GCC039 | H3K27ac | 28,157,883 | 77,909 | successful | yes |
| T980319 | GCC073 | H3K27ac | 39,373,811 | 99,105 | successful | yes |
| N980319 | GCC077 | H3K27ac | 26,697,681 | 66,886 | successful | yes |
| T2000877 | GCC080 | H3K27ac | 36,521,392 | 40,955 | successful | yes |
| N2000877 | GCC083 | H3K27ac | 43,571,456 | 54,967 | weak | yes |
| T990275 | GCC086 | H3K27ac | 31,760,962 | 189,540 | successful | yes |
| N990275 | GCC089 | H3K27ac | 29,830,003 | 77,678 | successful | yes |
| T20021007 | GCC092 | H3K27ac | 24,455,102 | 128,614 | successful | yes |
| N20021007 | GCC095 | H3K27ac | 37,328,252 | 140,389 | successful | yes |
| T20020720 | GCC098 | H3K27ac | 33,143,174 | 75,297 | successful | yes |
| N20020720 | GCC101 | H3K27ac | 41,166,130 | 64,078 | successful | yes |
| T2000639 | CHG078 | H3K4me3 | 67,834,207 | 15,445 | successful | yes |
| N2000639 | CHG079 | H3K4me3 | 55,494,042 | 13,233 | successful | yes |
| T2000721 | CHG080 | H3K4me3 | 46,397,600 | 12,513 | successful | yes |
| N2000721 | CHG081 | H3K4me3 | 49,140,770 | 13,183 | successful | yes |
| T2000986 | CHG082 | H3K4me3 | 64,771,240 | 13,416 | successful | yes |
| N2000986 | CHG083 | H3K4me3 | 55,125,882 | 14,221 | successful | yes |
| T980437 | CHG091 | H3K4me3 | 15,109,877 | 7,775 | weak | yes |
| T980097 | CHG099 | H3K4me3 | 32,721,765 | 11,491 | weak | yes |
| T990068 | CHG437 | H3K4me3 | 17,114,644 | 23,311 | successful | yes |
| N990068 | CHG441 | H3K4me3 | 21,403,480 | 9,608 | successful | yes |
| T2000085 | CHG445 | H3K4me3 | 16,983,508 | 16,274 | successful | yes |
| N2000085 | CHG449 | H3K4me3 | 16,171,233 | 13,421 | weak | yes |
| T980401 | GCC001 | H3K4me3 | 23,909,424 | 12,005 | successful | yes |
| N980401 | GCC005 | H3K4me3 | 38,132,521 | 9,697 | successful | yes |
| T980447 | GCC009 | H3K4me3 | 34,216,551 | 9,474 | successful | yes |
| N980447 | GCC013 | H3K4me3 | 44,268,513 | 11,678 | successful | yes |
| T2001206 | GCC017 | H3K4me3 | 40,391,040 | 14,353 | weak | yes |
| N2001206 | GCC021 | H3K4me3 | 37,385,163 | 12,831 | successful | yes |
| T980436 | GCC025 | H3K4me3 | 27,293,345 | 8,735 | weak | yes |
| N980436 | GCC029 | H3K4me3 | 22,978,142 | 12,798 | successful | yes |
| T980417 | GCC033 | H3K4me3 | 34,433,924 | 10,222 | successful | yes |
| N980417 | GCC037 | H3K4me3 | 16,720,512 | 10,735 | successful | yes |
| T980319 | GCC071 | H3K4me3 | 26,211,223 | 14,712 | successful | yes |
| N980319 | GCC075 | H3K4me3 | 29,348,973 | 9,167 | successful | yes |
| T2000877 | GCC079 | H3K4me3 | 29,326,567 | 10,218 | successful | yes |
| N2000877 | GCC082 | H3K4me3 | 28,119,193 | 10,002 | successful | yes |
| T990275 | GCC085 | H3K4me3 | 18,145,486 | 25,313 | successful | yes |
| N990275 | GCC088 | H3K4me3 | 34,949,142 | 9,320 | successful | yes |
| T20021007 | GCC091 | H3K4me3 | 29,829,629 | 9,143 | weak | yes |
| N20021007 | GCC094 | H3K4me3 | 26,657,454 | 15,570 | successful | yes |
| T20020720 | GCC097 | H3K4me3 | 22,283,141 | 11,351 | successful | yes |
| N20020720 | GCC100 | H3K4me3 | 43,532,126 | 9,899 | successful | yes |
| T2000639 | CHG019 | H3K4me1 | 74,550,475 | 8,757 | successful | not applicable |
| N2000639 | CHG023 | H3K4me1 | 66,105,736 | 11,132 | successful | not applicable |
| T2000721 | CHG027 | H3K4me1 | 63,149,869 | 49,925 | successful | not applicable |
| N2000721 | CHG031 | H3K4me1 | 75,647,236 | 18,042 | successful | not applicable |
| T2000986 | CHG035 | H3K4me1 | 56,988,063 | 26,669 | successful | not applicable |
| N2000986 | CHG039 | H3K4me1 | 61,038,200 | 15,277 | successful | not applicable |
| N980401 | GCC006 | H3K4me1 | 44,116,192 | 34,319 | successful | not applicable |
| T980447 | GCC010 | H3K4me1 | 45,487,925 | 8,573 | successful | not applicable |
| N980447 | GCC014 | H3K4me1 | 47,830,291 | 21,889 | successful | not applicable |
| T2001206 | GCC018 | H3K4me1 | 39,039,599 | 38,861 | successful | not applicable |
| N2001206 | GCC022 | H3K4me1 | 40,849,598 | 56,599 | successful | not applicable |
| T980436 | GCC026 | H3K4me1 | 43,909,448 | 60,720 | successful | not applicable |

The study experimentally generated a second biological replicate of H3K27ac Nano-ChIP-seq using KATO-III cells, and also compared the results against independent H3K27ac KATO-III data generated from regular ChIP-seq protocols. The published sequencing reads were processed similarly to the NanoChIP-seq libraries, excluding sequence trimming. Peaks detected by CCAT at a FDR <5% were compared.

### Chromatin Accessibility, Conservation and Binding Enrichment

Chromatin accessibility profiles of Epigenome Roadmap normal gastric tissues were obtained from the Gene Expression Omnibus (GSM1027325, GSM1027320). Read densities of chromatin accessibility profiles were computed for predicted enhancer regions and compared against 100,000 randomly selected regions in RPKM units. The study also computed fractions of predicted enhancers overlapping open chromatin regions (.narrowPeak) and active regulatory elements (H3K27ac, .gappedPeak) from 25 Roadmap chromatin accessibility and H3K27ac profiles. For transcription factor binding enrichment analysis, P300 and other transcription factor binding coordinates curated by the ENCODE (wgEncodeRegTfbsClusteredV3.bed), were downloaded from the UCSC genome browser. Overlaps of at least 1bp were identified using BEDTools intersect. Levels of evolutionary sequence conservation were assessed using PhastConst scores (Castelo R. phastCons100way.UCSC.hg19: UCSC phastCons conservation scores for hg19. R package version 3.2.0). The maximum score within 500 bp from the enhancer midpoint was used as the enhancer conservation score. Conservation scores were also computed for 10,000 randomly selected regions, excluding pre-detected enhancer regions.

### Identification of Predicted Super Enhancers

Predicted enhancers were defined as enriched H3K27ac regions at least 2.5 kb from annotated transcription start sites (TSS) and also showing enrichment of H3K4me1 and depletion of H3K4me3. TSS annotations for this study were derived from GENCODE version 19. H3K4me3/H3K4me1 log ratios were computed using aggregated H3K4me3 and H3K4me1 signals from GC cell lines and primary samples. Distal predicted enhancers exhibiting high H3K27ac signals, but exhibiting high H3K4me3/H3K4me1 log ratios (> 2.4) were classified as mistaken predictions and thus excluded from analyses. Predicted enhancers were then further subdivided into predicted super-enhancers or typical enhancers using the ROSE algorithm. Predicted super-enhancer regions with at least one base overlap across multiple GC lines were merged using BEDTools, and predicted enhancers localizing to regions distinct from the predicted super-enhancer regions were termed predicted typical enhancers. The presence of predicted typical or predicted super-enhancers in individual samples was determined by the level of H3K27ac enrichment above background (P < 0.01, empirical test), the latter being the H3K27ac signal (in RPKM) from 100,000 randomly selected regions. To assign predicted enhancers/super-enhancers to genes, distances from the predicted enhancer/super-enhancer center to the nearest active transcription start site (TSS) were calculated, defined as a promoter (500bp flanking at TSS) with H3K27ac enrichment above randomly chosen regions. Genes associated with recurrent predicted super-enhancers were tested for oncogene enrichment using a one-sided Fisher's exact test. The top 500 oncogenes were used. To identify recurrent predicted enhancer and predicted super-enhancers, the regions in each GC line were ranked according to signal strength. The ranks of each predicted enhancer/super-enhancer across the lines were multiplied to compute the rank product. To determine the statistical significance of the rank product, the observed rank product against a null distribution were compared - ranks in each line were reshuffled and the rank products computed. The reshuffling procedure was repeated for 10,000 iterations. Observed rank products less than the null distribution were considered statistically significant.

### Validation of Predicted Interactions

Super-enhancer/gene assignments were validated using three orthogonal interaction data sets. These included:
i) Predetermined interactions detected by PreSTIGE from 12 cell lines. PreSTIGE interaction data was downloaded from the PreSTIGE web site (prestige.case.edu), involving *cis*-regulatory elements and target genes.
ii) *cis*-regulatory elements/gene assignment by GREAT using the default parameters
iii) Reference sets of enhancer-promoter interactions from RNAPII ChIA-PET studies in K562, HCT-116, NB4, MCF-7, HeLa-S3 and GM12878 cells. ChIA-PET interaction data was downloaded from encodeproject.org and GSE72816. All interactions identified in each biological replicate were considered for validation. These interactions involved two loci (anchors), one of which is within 2.5kb of a TSS and the other anchor overlapping predicted super-enhancer regions found in our study.

Besides i)-iii), additional validation was performed using Capture-C analysis on GC lines (see Figure 4).

### Functional Enrichment Analysis

GOrilla was used to identify biological processes (Gene Ontology annotations) enriched in recurrent predicted super-enhancer/gene promoter or predicted typical enhancer/gene promoter interactions. Default GOrilla parameters were used, and genes from GENCODE v19 were used as background. To ensure comparability, predicted typical enhancers with the highest H3K27ac across cell lines were selected to match the same number of recurrent predicted super-enhancers. To select the former, predicted typical enhancers were ranked in each line and were chosen based on the rank product score. The most significant terms (> 1.5 fold enrichment) associated with the recurrent predicted super-enhancers were then compared against enrichment levels associated with the top predicted typical enhancers. Besides GOrilla, functional enrichments associated with recurrent predicted super-enhancers and top predicted typical enhancers were also studied using GREAT using default parameters, as GREAT provides correction against genes flanked by larger intergenic regions. Significant terms (also with >1.5 fold enrichment) were ordered based on Binomial p-values.

### Cell line Derived Super Enhancers in Primary Samples

Regions showing H3K27ac enrichment or depletion of by two-fold or greater and with absolute differences of greater than 0.5 RPKM were considered differentially present between GCs and matched normal samples. For principal component analysis (PCA), signals from predicted super-enhancers were used showing somatic gain in two or more patients. PCA analysis was performed using R and plotted using the 'pca3d' package. The required sample size to achieve 80% power and 5% type I error (http://powerandsamplesize.com/) was estimated based on the average signals of 100 predicted super-enhancers (Table 2) from tumor and normal samples. This result yielded a recommended sample size of 13 (average), which is met in the study (19 N/T). Three classes of predicted super-enhancers were defined based on the primary samples: i) somatic gain, ii) somatic loss, and iii) unaltered. Genes associated with i), ii) and iii) were mapped to gene groups previously reported in Hnisz, 2013, where each group is a compilation of several gene ontology categories and used as a proxy for various cancerous hallmarks. Statistical significance was computed using one-sided Fisher's exact test in R. To assess lineage-specificities of the recurrently gained somatic predicted super-enhancers across different tissue types, overlaps between the gastric predicted super-enhancers were computed against other non-gastric tissues. An enrichment ratio with each non-gastric tissue was computed based on the total observed overlap versus the total overlap by chance.

**Table 2: Top 100 super-enhancers showing somatic gain in 10 or more patients and the assigned genes.**

| **contig** | **start** | **stop** | **total patient** | **assigned gene symbol** |
|---|---|---|---|---|
| chr1 | 233,242,450 | 233,253,100 | 14 | *PCNXL2* |
| chr3 | 148,314,000 | 148,323,900 | 14 | *GYG1* |
| chr7 | 575,600 | 583,900 | 14 | *PDGFA;AC147651.3* |
| chr10 | 95,142,550 | 95,149,000 | 13 | *MYOF* |
| chr12 | 93,703,300 | 93,716,550 | 13 | *RP11-486A14.1* |
| chr13 | 31,402,050 | 31,421,550 | 13 | *USPL1;LINC00398* |
| chr15 | 72,528,100 | 72,532,050 | 13 | *PKM* |
| chr20 | 46,597,900 | 46,609,000 | 13 | *RP11-347D21.4* |
| chr20 | 61,334,350 | 61,336,500 | 13 | *RP11-93F14.4* |
| chr4 | 143,467,400 | 143,475,500 | 13 | *INPP4B* |
| chr6 | 138,405,450 | 138,413,000 | 13 | *PERP* |
| chr10 | 33,420,550 | 33,448,450 | 12 | *NRP1* |
| chr10 | 124,022,000 | 124,073,650 | 12 | *BTBD16* |
| chr11 | 89,337,050 | 89,342,700 | 12 | *TRIM77* |
| chr2 | 8,766,400 | 8,785,950 | 12 | *AC011747.6;SNRPEP5* |
| chr2 | 30,886,400 | 30,890,950 | 12 | *CAPN13* |
| chr2 | 151,380,100 | 151,387,900 | 12 | *RND3* |
| chr20 | 36,722,600 | 36,785,850 | 12 | *TGM2* |
| chr20 | 50,302,150 | 50,379,300 | 12 | *RP5-827A12.2;ATP9A* |
| chr20 | 56,271,150 | 56,275,000 | 12 | *PMEPA1* |
| chr3 | 141,655,450 | 141,661,750 | 12 | *ATP1B3 RP11-434D9.1;RP11-* |
| chr5 | 67,062,650 | 67,073,700 | 12 | *83M16.6;PIK3R1* |
| chr6 | 86,108,600 | 86,129,950 | 12 | *NT5E;RP11-30P6.6* |
| chr7 | 17,242,450 | 17,252,950 | 12 | *AC003075.4* |
| chr7 | 27,713,900 | 27,721,650 | 12 | *HIBADH* |
| chr8 | 19,516,550 | 19,525,800 | 12 | *RP11-1105014.1* |
| chr8 | 94,880,850 | 94,899,700 | 12 | *RP3-388N13.3;PDP1;MIR378D2* |
| chr8 | 124,681,700 | 124,695,650 | 12 | *CTD-2552K11.2* |
| chrX | 132,807,600 | 132,814,350 | 12 | *RP3-417G15.1* |
| chr1 | 19,331,100 | 19,342,550 | 11 | *IFFO2;UBR4;RP5-1126H10.2* |
| chr1 | 149,987,150 | 149,996,100 | 11 | *OTUD7B* |
| chr1 | 162,316,150 | 162,324,600 | 11 | *C1orf226* |
| chr10 | 75,645,800 | 75,660,400 | 11 | *PLAU;VCL* |
| chr11 | 12,186,850 | 12,207,700 | 11 | *MICAL2* |
| chr11 | 35,357,500 | 35,360,800 | 11 | *AC090625.1* |
| chr12 | 2,268,950 | 2,280,050 | 11 | *CACNA1C-AS4* |
| chr12 | 14,360,100 | 14,362,500 | 11 | *RN7SL46P* |
| chr12 | 118,122,450 | 118,125,400 | 11 | *KSR2* |
| chr12 | 132,344,550 | 132,346,400 | 11 | *RP11-417L19.2* |
| chr14 | 61,643,050 | 61,647,400 | 11 | *PRKCH* |
| chr14 | 75,507,300 | 75,510,200 | 11 | *MLH3* |
| chr14 | 102,536,000 | 102,540,650 | 11 | *HSP90AA1* |
| chr15 | 101,259,950 | 101,276,450 | 11 | *RP11-66B24.5;RP11-66B24.2* |
| chr18 | 29,088,000 | 29,095,100 | 11 | *DSG2* |
| chr19 | 42,274,750 | 42,285,750 | 11 | *CEACAM6;AC011513.4;CEACAM3* |
| chr19 | 50,670,050 | 50,685,350 | 11 | *MYH14* |
| chr2 | 8,445,650 | 8,457,150 | 11 | *LINC00299* |
| chr20 | 56,841,250 | 56,845,250 | 11 | *PPP4R1L* |
| chr4 | 113,002,500 | 113,008,400 | 11 | *TUBB8P3* |
| chr7 | 46,017,900 | 46,020,700 | 11 | *RNU7-76P* |
| chr7 | 46,229,800 | 46,262,500 | 11 | *AC023669.1;AC023669.2* |
| chr7 | 73,204,750 | 73,314,500 | 11 | *WBSCR27* |
| chr7 | 100,396,800 | 100,402,200 | 11 | *EPHB4* |
| chr8 | 37,447,750 | 37,479,500 | 11 | *RP11-150012.3* |
| chr8 | 142,213,300 | 142,220,550 | 11 | *SLC45A4* |
| chr1 | 20,806,050 | 20,825,150 | 10 | *CAMK2N1* |
| chr1 | 59,037,200 | 59,059,450 | 10 | *TACSTD2* |
| chr1 | 186,806,100 | 186,820,000 | 10 | *PLA2G4A* |
| chr1 | 201,223,450 | 201,282,500 | 10 | *RP11-567E21.3;TMEM9;TNNT2* |
| chr1 | 235,804,100 | 235,807,100 | 10 | *GNG4* |
| chr10 | 97,877,700 | 97,886,350 | 10 | *ZNF518A* |
| chr11 | 12,145,900 | 12,173,050 | 10 | *MICAL2* |
| chr12 | 12,985,800 | 12,991,300 | 10 | *RP11-59H1.1;DDX47* |
| chr12 | 104,245,750 | 104,255,900 | 10 | *RP11-650K20.3* |
| chr13 | 74,243,950 | 74,259,650 | 10 | *LINC00392;KLF12* |
| chr13 | 80,655,150 | 80,663,050 | 10 | *SPRY2* |
| chr13 | 106,790,700 | 106,804,950 | 10 | *LINC00460;RNA5SP38;AL603632.1* |
| chr14 | 54,911,250 | 54,917,100 | 10 | *CNIH1* |
| chr16 | 52,483,700 | 52,506,000 | 10 | *RP11-297L17.2,TOX3* |
| chr16 | 86,695,500 | 86,700,800 | 10 | *MTHFSD;FOXL1* |
| chr18 | 3,814,650 | 3,818,650 | 10 | *snoU13* |
| chr18 | 9,823,650 | 9,840,800 | 10 | *RAB31;RN7SL862P* |
| chr19 | 42,233,300 | 42,251,850 | 10 | *CEACAM6* |
| chr2 | 8,548,250 | 8,558,050 | 10 | *LINC00299* |
| chr2 | 62,791,800 | 62,808,700 | 10 | *AC107083.1;EHBP1;TMEM17* |
| chr2 | 121,688,900 | 121,702,700 | 10 | *FLJ14816;AC016764.1* |
| chr20 | 19,858,600 | 19,864,650 | 10 | *RIN2* |
| chr20 | 48,802,850 | 48,878,800 | 10 | *CEBPB;RP11-290F20.3* |
| chr22 | 30,645,950 | 30,655,100 | 10 | *LIF;RP1-102K2.6* |
| chr3 | 14,098,050 | 14,100,800 | 10 | *TPRXL* |
| chr3 | 122,160,250 | 122,165,300 | 10 | *KPNA1* |
| chr3 | 158,485,100 | 158,509,400 | 10 | *RP11-379F4.1;RP11-379F4.8* |
| chr3 | 159,823,350 | 159,827,600 | 10 | *IL12A* |
| chr3 | 190,019,750 | 190,027,750 | 10 | *CLDN1* |
| chr3 | 197,313,350 | 197,328,100 | 10 | *BDH1;AC024560.3* |
| chr4 | 7,945,700 | 7,982,500 | 10 | *AC097381.1;AFAP1;ABLIM2* |
| chr4 | 57,079,600 | 57,085,100 | 10 | *KIAA1211* |
| chr6 | 137,279,250 | 137,292,400 | 10 | *RP11-204P2.3;RPL35AP3* |
| chr7 | 27,456,650 | 27,466,800 | 10 | *AC004009.3* |
| chr7 | 48,177,150 | 48,189,350 | 10 | *UPP1* |
| chr7 | 97,712,250 | 97,718,650 | 10 | *LMTK2* |
| chr7 | 97,738,750 | 97,766,850 | 10 | *LMTK2* |
| chr8 | 53,241,850 | 53,257,600 | 10 | *RPL34P17* |
| chr8 | 95,200,950 | 95,225,750 | 10 | *KB-1247B1.1;CDH17* |
| chr8 | 95,244,800 | 95,256,850 | 10 | *CDH17* |
| chr8 | 128,402,500 | 128,419,900 | 10 | *RP11-382A18.3;POU5F1B;CASC8* |
| chr9 | 111,234,600 | 111,241,950 | 10 | *RP11-240E2.2* |
| chr9 | 139,421,550 | 139,429,600 | 10 | *NOTCH1* |
| chr1 | 7,597,450 | 7,611,950 | 9 | *VAMP3* |
| chr1 | 20,171,500 | 20,206,000 | 9 | *OTUD3* |

### Capture-C and Data Analysis

Capture-C was performed as previously described Briefly, 1×10⁷ cells were crosslinked by 2% formaldehyde, followed by lysis, homogenization, DpnII digestion, ligation, and de-crosslinking. DNA was sonicated using a Covaris to 150-200bp to produce DNA suitable for oligo capture. 3µg of sheared DNA was used for sequencing library preparation (New England Biolabs). Predicted super-enhancer sequences were double captured by sequential hybridisation to customized biotinylated oligos (IDT, Table 3) and enrichment with Dynabeads (LifeTech). Captured DNA was sequenced on an Illumina MiSEQ using the 150 bp paired-end configuration.

**Table 3: Capture point coordinates and sequences used in Capture-C technology.**

| | | |
|---|---|---|
| #1 | chr1:202003 857-202003977 | ATCTCTTTCCTTCAGCCTGCCGTTCTTTCTGCAGCACCAGGGCCCTGGGACCAGCTG GTGGTTTCCACCAGAGCAGCCTCGGGGTGAATTTAGTCAGGAATGTGCCCTCAGCT CAAGAGA (SEQ ID NO:1) |
| #2 | chr1:202015 440-202015560 | GCTAAGTGAGGTGCAAACAAGAAACCTGGGTTGCCTTTGCCCTCTGTCCGCCCCTTG TCCTCTGTTTACATCCTCCCTTCCCGTAAATGAGTTGGGTGCTGGGCCCCACTGGCCC TGATC(SEQ ID NO:2) |
| #3 | chr1:202025 797-202025917 | ATCTGGAAGGCTTTTCCCAGCTTAGCGTGGTCAAGATAGGGATGGGCCGAGGCTGG CACTGATGCTAGACTTCCGTGCACAGGGCAAGTATGGACAAGCCCCAAGTGGCTTT GTGAGGCC (SEQ ID NO:3) |
| #4 | chr1:202054 225-202054345 | ATCCCGGAGATGGGGGGTGGCCCTGGGCCAAATCAGGCACCTCCCTTTCTCACCAG GTAGTGCCTCCCTGCACGTTCACACCCAATGCTGTGTTGTCAGGGGCTGTAACCTGA GCCCTGG (SEQ ID NO:4) |
| #5 | chr1:202077 797-202077917 | GCTAGCCATCTGTTGAACCACACCCCTGCCCCAACCATTCTAGAAAGAAATATAAAT CTCTTTTACAGCTGTAAATGGAGAGCTCTGTAACTCTAATATGGAGGGAGATACACG CTGATC (SEQ ID NO:5) |
| #6 | chr1:212656 104-212656224 | TTAAATCATTAGAGGGATTTATTCCTTTCCGGAAGAGTCACTCTTCTGCGGTCCTTC CACACCCAGCTTTGGACTGGGCCACCTGGCAAGGGTGTGAAGTGGACTTGTGGTTG ATGATC (SEQ ID NO:6) |
| #7 | chr1:212658 162-212658282 | ATCCACAGTCTGAAGGGCATTGCATTAGGGCCAGCCCAGGGCGAGTGGCCTTAGCT GGGCTGGCTATAGCGTGTAGCAGAGGTCAGTATGGAAAATGGCCCTAGGTGCATTC TGGGGCTC (SEQ ID NO:7) |
| #8 | chr1:212691 818-212691938 | ATCATTCTGAAATTGCTTTAGGGGGAAAGACGTGGGAACTTCACACTTCCACCCAGG GTGCCCCCTCAGCAATCTGGAATGATGGACTAACCATTAGCTGAGGGAGGAGGGG GCAGGACA (SEQ ID NO:8) |
| #9 | chr2:627969 50-62797070 | ATCACTTGGTCTGAATATAGGCTAGTAAGGCCCATATCATAAGGCCGGTAAGATTCA AAAAAGGTAAAAAAAAAACATCTAGTTTCGCAGACTGCAATCTTAAATACAGCAA GCCATTT (SEQ ID NO:9) |
| #10 | chr2:628052 11-62805331 | ATCTTTTTGCCAAATTGGATGTGAGCTGACTCACTGACATATTTCTCAAGTGACCCAT TGGTTCAATGAGTAACATCCTGGAAGAAACATGAGTTATTGTTAATCATAATTATTC CTTCA (SEQ ID NO:10) |
| #11 | chr2:106020 684-106020804 | ACAAGCTGTTCCTCCCACTCAAACCTTGGCCAGGAAACTGGTGGATGATTTGCCCTT GATTCAGAGGCAATCATTCTTAATTGCCTCACATGGTTGGAAGGTGAGTAAGTGTCT AAGATC (SEQ ID NO:11) |
| #12 | chr2:106065 222-106065342 | ATCAGTAAAGCGACGCTTTGAGAAGGGGAATTCCTTAACCAGCCTAAATCAGTGAA TAGGATTTTGCAGAGGGAATTAGCTAAATACATTCCAAATTAGGGAAGAAGGGATT TTGACAGC (SEQ ID NO:12) |
| #13 | chr2:106071 749-106071869 | AACACAGATGCTTCAAGTGCCAACAGCCAATAACCTATAACCCGAATGACATTAGGC TGGGACTGAAAGAAGTCAGGCAGCAGGCAGGCAAGCCTTTTAAAGAAAACTGAAT CCAAGATC (SEQ ID NO:13) |
| #14 | chr2:114039 690-114039810 | ATCTGCTGAGCCTTCAAAAGATGTTCTTTCTTTTCTGGACTCAGCTGTAATGCACTGG GCTGGTGGTAGGGTAATAAAGTGCCCTGGTTTGCCCTGGACGAAAACCAACAGTGT TTTCTA (SEQ ID NO:14) |
| #15 | chr2:114046 487-114046607 | CAGACCCTTTTGGGGCCCTGATTTACAGGTGCCCTGAAGGGGGAGGTATTGTTCTA ATGGCCCTGCGGGAGGATGAGGTCACTTCTGTGGGACTGTCTTACTCTGGCCTGCG CTGAGATC (SEQ ID NO:15) |
| #16 | chr3:149057 460-149057580 | ATCAATTGTTATTTGGAAGATGGTTCCAGAAGAATGACAGAAGTGAATGAAGAGGA TATTCCTGGCTGGAAAACTTGATAAAATTGTTGAAAAGGGAGTTGAGTAATTTATTT GTCTTTG (SEQ ID NO:16) |
| #17 | chr3:149086 491-149086611 | CTGTCACTTGAAAGAGCCTAACCCTGTACAGTAAGGAGAAAAATGCCTGTTACCCTT CCAGGGAGGCTGATACTTGCAGCACCTGGTAGAAAGGACCAGTGCCTAACTGGGG TGATGATC (SEQ ID NO:17) |
| #18 | chr3:149105 264-149105384 | CTCTGATGAGACCCTTCAGAATAGCTGTCCCTAAGAGGAACTAAATCAGGAATTGG GGATAGCTGGCAAGAAGACATCAAAGAAAGCTCAGGATGTGGAATCTCTACATTGC CCTGGATC (SEQ ID NO:18) |
| #19 | chr3:149119 076-149119196 | ATCTCCAGTTGCCTGTCACTACCCTCTGTCAAGACCCTTGGAGTCATGACTAACAGG AAGGGAGCAGGTGGCAGCGTGGCCACCTGCCATGCAGAAAGACTGGGTCACTTCC TGTTGGTA (SEQ ID NO:19) |
| #20 | chr3:193544 590-193544710 | TTGTGAAACTGAGTTGAATGGAGAGGGTTGGCTGGAGACCTGAAAGAGGATTTTTA AAGGCCCAGGTCTCATCATCAACGTGGCCACTCACTAGGTGGTGGAAGGAATGTAG AAAGGATC (SEQ ID NO:20) |
| #21 | chr3:193570 162-193570282 | TCTGCAGAGCGGCTCTCCCACCTGCTGGGATTCTCCAGAGGAATCCTTTTCTTCCGT CTGAGTTCAGCAAACTTCCTGCTTCCTCTACCCAGCGCAGCGAGCCCCTCTCTGTACT GATC (SEQ ID NO:21) |
| #22 | chr3:193591 177-193591297 | CTGCCACCTCAGCTTTGCAGCGCCTGGTGGGTAAACTCTTGTCCCCTCTCCGTGGCTC TGGTCAAAGGTACCTTCATTTGTGAGGTCTTCTCAGAACCCTCAGGCACAGTTAAAT TGATC (SEQ ID NO:22) |
| #23 | chr6:681284 6-6812966 | ATCCCAAGCCTGTGGGTTCACCTGCTCTAAGAAATCAATAAGTCAAGGGAAACATCA AAGGGCATTACACATATGGGCTTTGACGCCAGGCCGACCAACTTCAATCCAGGTCTA AATGAG (SEQ ID NO:23) |
| #24 | chr6:682114 9-6821269 | GGACAGGTGAGGCAGGCCAGAACCGGTGACTCATGGGCTCCCCTTGGTCAGGAGG GCTGGAGCAGGTAAAGCCCGCCCACAGCCGGGGAACCCACACCCAGCACACGTTCT CTCCTGATC (SEQ ID NO:24) |
| #25 | chr6:685771 4-6857834 | GGAATGAGGTGGGGCAGGACCTGAGAGCAAAGTGTGAGCTGGTGTGCAGAACCA CCCGGAGGTGGAAGGAAGCTAGAATCTAGTGTAGGGTGCCTCTGACACTTGTCCCA CACATAGATC (SEQ ID NO:25) |
| #26 | chr6:137283 834-137283954 | ATCAACTCCTTTCGGACCCACAACCTCTTCTTTTTAAGGCTGCTTGAACTATTTATTAG TCTGTAATTAGAGTCCCAAGCGTTTCCTTCTGTTTCCTAAAGGGTTGGAAAAATGCCC CGA (SEQ ID NO:26) |
| #27 | chr6:137285 302-137285422 | ATCCATGTTCCCTATTTAACATGCTATTCCTGTCCCCAGAAAAATCCTAAGACACATA CACGCGTGCTCTCTCTCTCACCTCTCACATTGCTTAAATAAGAGACCACAACATACTG TGAA (SEQ ID NO:27) |
| #28 | chr6:137291 056-137291176 | CATAGGGCTTTGCTCTTGTCTCCATCCCTGAAAAATCCTTCCTAAGCACTGTATGGTA TAAATATTTTAGTATCTGTCCATGGATTGGCTTGTTGTCTTTGTTGAGTTGCACGCAT GATC (SEQ ID NO:28) |
| #29 | chr6:137322 790-137322910 | ATAGAGTCTAGATAGAAGACCCTCCTCTCCGAGCCCATCCCCCTCAGAAGGCTCGCA GCCCTCTGAATCCTGGTCGAAGCTGGACAGCGAAGGAATACACAGCCTGCCAGTTT GGGGATC (SEQ ID NO:29) |
| #30 | chr6:137347 579-137347699 | ATCTGCACAGTCAGAAGATACTCAACAGCTGCGTTTTAATGAAGGCACAGTAACCCA TGGCATGGCAAGTGGTTGCTACATATTTTATGTGTATTTTTAAATAGGAAAATACCTT CATAG (SEQ ID NO:30) |
| #31 | chr6:137358 698-137358818 | TCCTTTTAGACATCAGAGGCCTGTGTTCATCAGGAACCTGATGCTGAATCATTGGAG GGTAAATGAACCTTCCAAGGTTCAGTGTTTAGAATGTTGTAGACCAGCAGTCTCATC ATGATC (SEQ ID NO:31) |
| #32 | chr7:579567 -579687 | AGCCCGCCCTCCTAGGAGAAGCCTGGCCAGGTTCCAGTGGGGTGGTGGCCGGCCC ATAAACAGGAGGGGTTTATGGCCCAGTGACAGGCAAAACTGGTGGGGCAAGCCCA GGCTAGATC (SEQ ID NO:32) |
| #33 | chr7:732368 65-73236985 | ATCTGGCAGCTGGACTTCTTGGGCTCTGAGAAGGCAAGATTAGTATCTGTGTGT GACAGGAGAGGGCGTGGCTGGTGTCCACCCATCCATGCTGGGAGACGTGGGAGAG ATGGGGCGG (SEQ ID NO:33) |
| #34 | chr7:732658 73-73265993 | AAACCAGAAGGGCACTACTGAATCAGGGTACAGGCAGTGTCTGAGACTCTGGTTAG CCTACAGAGTCATCAACGCACGTGTGCTGTAGACTTTTTTGTTTTTGCAAATGAGGG TGAGATC (SEQ ID NO:34) |
| #35 | chr7:771089 16-77109036 | CATTTCATGAAAGGAGTCTGATGCTTGTAAACTAGCTCAAATTACCTACTGGATGAC CAGAGATGCAAGGCTAGAGAAAGAGGAGCTCTATTGCATCAGGAGCTGAGGCAGG AGAGGATC (SEQ ID NO:35) |
| #36 | chr7:771109 71-77111091 | ATCAAGAACAACCCCATTCACTCCTAATCAAATGACCAACCTGGCCTTTGGCCTTAAT AGGAAGTAAAAGTGTCTCTTCCGGCATTGTATCAGTGGTATGTGCCGCACCTACCAC ACCTG (SEQ ID NO:36) |
| #37 | chr8:423347 16-42334836 | GTGAGGAACAAACTTAATTGGGTAGAAGTGTTTCGCCTCAAGCAACTTGTAATTACT GGCATCGCTGTAGTCACAGGAAGAATAACAAATGAGAGGTTCCAGAATCCTTCTGG AAGGATC (SEQ ID NO:37) |
| #38 | chr8:423498 95-42350015 | ATCTGCCCATGCAAGGTGTGTCTTCACTTCCTAAGGAAGTAATACTGCAGAGAGGA ATGTCATGACTACTCCTCTCATATAATTGCAGTAGAAAGACACGAGATGATGAAGAA AGGAAGG (SEQ ID NO:38) |
| #39 | chr8:423561 94-42356314 | GCTGAAGGCATCCCTCCTGCTCACTGCTCCTTCCACTTTAGATGAACAGCTGGAACTC ACATAACACAGCCTCTTCCGACAAGATTTCCTTTAGAGAGAGAACATTCTAGGGATG TGATC (SEQ ID NO:39) |
| #40 | chr8:102017 079-102017199 | ACTAGGATGAAAGGCAGCTAAAAAAGAAATATATGGCCAGGCCAGTTTACCTGGA GTAAGATACAAGTAGAATAACAGGAGTTGTAATTACAAAGCTTGGTGGGAAGGCTA TGTTAGATC (SEQ ID NO:40) |
| #41 | chr8:102039 348-102039468 | ATCTCCTTGACCCTGCAGCCAATGCCTCGGTCAGCCAGTGCACCTGTACTGTCTCTCC TCTTGGGATAGGGTCCCTCTCCATCAGGTACAATATATGGGAAATCGAGGGGTGGC CTTTGT (SEQ ID NO:41) |
| #42 | chr9:363168 00-36316920 | GGAGGGCAGCTGGCAGGGGCAGGCTCTGAAAGCACAGCTGTGTGAAGGTCCGGTT CAATATCCGCTTCAGAAGACACACAGCCCTTTGTTGCTCATGTCTGTTGCTGTCTAAA GTTGATC (SEQ ID NO:42) |
| #43 | chr9:363178 45-36317965 | ATCCCTGAGGGAATGAGCCACGGTTCAGCACCCAACCCCCACTTGAACTCTGCAGTT TCCCAGTTTCATTAAGAAGCCCATTGTTGAGTCTGGCCATGCGTCAAGGACACGTGG ACTCTT (SEQ ID NO:43) |
| #44 | chr9:363206 62-36320782 | GAGGCCTCAGCACTCCACTGACTCATCAACCCTTCTGTCCTTTGATGGGTAGGATGG GGTGAACGCTAATGCCAGCAGACCTGGTTTCATAATATCTTAGTGTGTTCTGCATGT GTGATC (SEQ ID NO:44) |
| #48 | chr12:63252 27-6325347 | ATCCTGTTTGTTCTTGCTGGCACTGCCTGGCCCGGCTTCCTGAGGAGTGAATCAGCC CATCCAAGGCTTGGCATGCAGTAGTGAGCCAAGGGTTGCCATGGAGATGGGCGAG GCCCAGAG (SEQ ID NO:45) |
| #49 | chr12:63877 78-6387898 | AGGGCCCGCGGGAACGGCCTGGCCCAGGCCCGCGCGCCCTGCCCTGTGTTCCCGG GAGGCGCGGTCTCCTGGCGGCAGGGGAGTCCCGGGAGGGCGCAGGGCGGCGCAG AGCCTGGGATC (SEQ ID NO:46) |
| #50 | chr12:63891 88-6389308 | ATCATTTGCATAGCTCTTAGTTATTTGAGCAACACATTCATTTAATTCCACAGCAATG TGGGGAATGAGGGACTCTTCCCTGCTTTCCAGGTCAGCAACAGGAAAAACAAATGA TTTGCT (SEQ ID NO:47) |
| #51 | chr12:64045 29-6404649 | GGTTGCCTGGGGCAACCCTGGGAAGGTGAGGCCCTGGATTTCCTGGTCTGGCCTCT GAGAACCTCCTAGCACCCCAGAACTCCCAGACAGAATGTTAGGTAATGCCAATGAG CACAGATC (SEQ ID NO:48) |
| #52 | chr12:12988 275-12988395 | ATCCAAGTTATCAAAAGTGGGTAACAAAGCAACATCAAGAAACTGTGTCCTTAAAAC TAAGAGAATATACAAGACATTCTAATCCACATGGTTCTTAGGGTGAAATCTAGCCTC TGGGCA (SEQ ID NO:49) |
| #53 | chr12:12989 691-12989811 | CTTCCTGTGTTTCAACCTTCTTATCTGAAGACACACAGTTATAGTACAGCATTGTGAA GTTAAATGAACTATACTGTGGCTCATGATAACAATCAGTAAGTGTTAGTCATTATAA TGATC (SEQ ID NO:50) |
| #54 | chr12:53364 077-53364197 | GCTGCATGCCTGGCAGCTAGTAGGTACTGCATAAATACAAGTTCATTGGCTCTCCTT GAGTTGTCTGGCTGGGGTCCCAGAGACTGAAACTTTGCTCTTGGCTGCAGCAGGGG CAAGATC (SEQ ID NO:51) |
| #55 | chr12:53386 828-53386948 | ATCTGGTAACGTTGGGCCCTATTTCAGTTGACAATGAGAGTCAGGGTTGAGTTGTG ACTGTCACCTTTAAAGAAAGTACATAGAGTAGGCTAGGCGACAATGACAAAATATC AGCAACTT (SEQ ID NO:52) |
| #56 | chr12:12507 3621-125073741 | CTCTTTAGAACGTCTCTTCCTCCCGTGGGATTGGAAAGAACATTTGTCCTCAGCGGC AAAGGCAGGGCTTCATCAAAGACGAGTGACTCAGTGCTTTTCCTGCTGTCTGGCCAC ATGATC (SEQ ID NO:53) |
| #57 | chr12:12508 8201-125088321 | ATCCCCCCAACAATCGTCTTGTATTTTTTCAGCTCAGTTTCCTGAGCCTGCCACACC GAGGGCCCCACAGTCGGAAACCACGTTTGGTTCGAAGCTCTCCTGTTCTCATCTGGA AATTC (SEQ ID NO:54) |
| #58 | chr12:12512 2284-125122404 | GAAGATAAAAATCTATTAGCTGCCAAGAGGATTGCTGGAAAAGCCAAGCCGTGAT TCATAGGGTAATAAATAGAAAGACAGTCCCGGCCCCGGGGCTGCATCTTCCTTGCAT GCTGATC (SEQ ID NO:55) |
| #59 | chr12:12516 8228-125168348 | TATTTCCCTTCAGAAATAGCATCCTTAACTTTCTTTTTTTTTCCTTATTAAAAATGTA CTCATGTAATCCACATGCACTGGCTGTGAGAAATTCCAAAATGTCTTTGGAGAGAG ATC (SEQ ID NO:56) |
| #60 | chr13:73900 379-73900499 | TTGTGAAGTGGGATATGTTTTTAAATTTCAGAGAACAGGAAGATGAATTGTTTTTAA TGGATTTTTTTTAATAGGCAAAGCTGTGTATGCACAAATGCTGGCCAGTGTAGGGCT ATGATC (SEQ ID NO:57) |
| #61 | chr13:73909 247-73909367 | ATCTATTTCTCTTATTTTTTTTAAATCTAGTGTTTCATAATGTCTAAAAGAAAGTGTT TGCAAGTCATTGTGGTTTTTTTTTTCATCAAAGTATTCCTTGTTTCACTCTCTGTCGC AT (SEQ ID NO:58) |
| #62 | chr13:73910 858-73910978 | ATCCTGTCTCATCTTGGATGTAATTCCTACAGTTAGACTTCTATCAAAGGGTCATTGT GCCAACTGGAATTCTTTCCAATTCGAGAAATAAGAATTTGAGGAATCTCTAAGGGTA GAAAT (SEQ ID NO:59) |
| #63 | chr13:74002 592-74002712 | ATCTCCTTACATAAGGAGAAATTTTCAGAAATTAATAAAATGAAGTTCAGCCTTAAG GAATGTGACTAATACATCTGAGATAAGTGACTCAAACACTAGAAGAGGGAGATACT TGCAGTT (SEQ ID NO:60) |
| #64 | chr13:74025 384-74025504 | ATCTTGACAAACACTAGAGACCCATCAGCCAGAGTGAATTCCCTTAGTGAAATCACT ATTCTCTGCCAAGAATCAAAGGTCATCCCAGTGGAGTGCCGTCTTCGTGTTCAGCAG CTACGG (SEQ ID NO:61) |
| #65 | chr13:74039 936-74040056 | CTCTGTGAGTCAGCGAAATGGATTGAATTATAAACCAGAAACCAGCACTTCAATCTG ATGAAACTCATCCTATAGAGGAAAGTGTCACTACATGTTGGCAGTTTGTAAAATGTG ATGATC (SEQ ID NO:62) |
| #66 | chr13:76278 885-76279005 | ATCAGAACTGGAACTGAGCGATAGAGCTTCAGAGAAACTTGTTGTTTACATTGGCA AGAAATCTTATCCAGCAAGAGGATTAAAATAAAACCATGACCAAAACAGTAAAGGG TGTGAAGA (SEQ ID NO:63) |
| #67 | chr13:76298 560-76298680 | ATCCTAGTACATAGAGGTATCTGGAAGGTTATTATGTTATCTGAATCTTGAATAGAA TCAGTTACCTGAAGCCAGTGTTACCTTAGGAATTCCATATGTGTGATTGAAGATTGC TTATTA (SEQ ID NO:64) |
| #71 | chr14:93510 901-93511021 | GCCACAGGGCCCCCCAGGTAAAGGACTGACAGCCAGCCATGCAAGCCCCTGGGACT GACCCAGATGCAGAACTGACCAATGTGGCGACAACCATCACCCAACCCTGTGGGGC TGTAGATC (SEQ ID NO:65) |
| #72 | chr14:93516 507-93516627 | TACACAAGGAGGACTGGGTTGCACTCGGTGCATTTTAAAGAAAGCCCGGCGGACAC ACGGGTTCTCCTGCTGGTGCTGCCAGCCTGGAAAATGACAGCTGAGACTCAAGCAG GCAGGATC (SEQ ID NO:66) |
| #73 | chr15: 10126 2583-101262703 | ATCCAGTGACTATACCTGGAATTTGGCCTCTAAGCGAGTGTTTCTTTTTGCACTTGGA CTTCTTGAGAGATTACTCATTACTCAGGAGATAACACCAAAGCATTCTCAAAACCAA TCTTT (SEQ ID NO:67) |
| #74 | chr15:10127 2951-101273071 | AATATAGGAGAAAGGTCACAGTGGCTTGGAGATGAATTCCAGGACACAGCACTGC AGGAGGGACCATGCCCCTCGAGGCTTCTTATATGTGGTTCCTCAGTTCTTGGCTAAG TTAGGATC (SEQ ID NO:68) |
| #75 | chr16: 17369 195-17369315 | TGTGGGCTGTTAACAACTACGCTTGGCTCAGTGCCCCACAGGTTTCTAAAGTCCCCC CCAGGAGGAAACACCACTTCCACCAAAGGCCCTGGAAGAAAGCAATCACTAAGTGA GCTGATC (SEQ ID NO:69) |
| #76 | chr16: 19010 165-19010285 | GCTGTTTGCTCATCTGTTTGTAAAGCCTTAGACCCAGCCAGTCACCGGCCCACCCA GGTGCCAGTTGACGTGAGTCATGCAGTTTCTCCTGACACTACTGTTACAAAGTCTAG CAGATC (SEQ ID NO:70) |
| #77 | chr16: 19016 835-19016955 | ATCCTCCATTCCCGAGATGAGAAAAATGTGACTCTGAGGCAGCTGTGGTCTCTCCTG GGGTGGCTTGGCTGGTGGGTGACAGGCCAGTATTTTAAACCAACCCAGGCTCCAGC CTGGGGC (SEQ ID NO:71) |
| #78 | chr16: 19440 596-19440716 | ATCTCAATACTAACAAATGTTTATTTACAGGAAGGCTTTGTTAATTGAGTGATGGAA GCTATTGCTCTCACTTGCTATTTAGAGACTATTGACATTACCTTTATTTCCTGGTCCTA AACA (SEQ ID NO:72) |
| #79 | chr16: 19443 483-19443603 | ATCCCTAGAAGATTTCTGAATTAGCTGAATGACAAACACAAGATACTTAGATGACCC ATTCGAACCAGGAAGCCTTGCAACATCAAGTAAGGAGTCCTCAATTAACAGCCTTAG AAGACA (SEQ ID NO:73) |
| #80 | chr16:69430 565-69430685 | ATCAAAGAATACTTTTTTCAGGGGGGAGGATGTTGGTATTTACTTAACTCACTGAT GGAGAAAAAGAACGCTCCCACTCTATAAATGTTTAGCATGTGTGTAAAGGTCGTAG GTCTGGT (SEQ ID NO:74) |
| #81 | chr16:69438 576-69438696 | TAGGTTTGAGTCCAGAGCATGACCTTTGGGCTTATCAGACAGTGAAGGATGGGTC AGAACTTGTCACCTGTGTTCACGGGATGCTGATTCACTCAGCAAGGATGAAGTCTTT TCAGATC (SEQ ID NO:75) |
| #82 | chr16:69441 798-69441918 | ATCAAGAAGGGAGCACAAATTCTCTCCCTCCACATGGGAGGGTGCTCCGGTCCACC GCTGCTTGTGACTGATGGGACAGTCTGTCTTATTAGGATACAAGGACACACTCAGCC ACATTCT (SEQ ID NO:76) |
| #83 | chr17:39058 295-39058415 | ATCAATAATGCATTAGATAATGGTGTTCTGAGGCAGGATTTCAATGAAAGGCTAAG TGCTGTAACTATGTAACAAAACACCAGCACAGGACAAAGAGTAGGTTTCTCAATCAA CAAGGAT (SEQ ID NO:77) |
| #84 | chr17:39059 208-39059328 | AGTCTTTCCAGTGCTTCGGATGCATGGTAAAGTTGGAGAACCATGGCTGTAAAGTAT CAGTAGTCGAGGAGCAGGAAGATATTTCCACTGGTTGTACATTTTAGTTTAGAATGC TTGATC (SEQ ID NO:78) |
| #85 | chr17:39061 487-39061607 | GTAATGATTCGGAGTGAGACCCCTAACACCTCCCGAATGGGGGCAAGAAGCTCAGG ACTCTGGTAGCCCAGGCAGAGTCTGAGTAACTGGCGTTGCCTCTCTTCATGCATCTA CCTGATC (SEQ ID NO:79) |
| #86 | chr17:39807 749-39807869 | ATCCTGAAGAAGCAGTGCCAGCTCCTCCCAGACAGCCCGCCCCAGCTGGCCCGCTG GCTCCAGCCCAGCCAGGAGCGAGGGCAGTGGGGGAGCCCCACAGGAGGGAACGG GGGCTCAGGA (SEQ ID NO:80) |
| #87 | chr17:39810 377-39810497 | ATCTCCCCCAGCCTGCACCCCACCCAGCAAGGACATGCAAGCCAAGCAGTTCCCACA CCCCGTTCCTGGTGGCTGCCAGAAGCTTCCTTACAAGGCCCCAGCGCTGAGCTGGCT CAGCTG (SEQ ID NO:81) |
| #88 | chr17:39811 992-39812112 | ATGTTTGGGCCCCGGGCTGAAGGTGTGGGTGGCAGGGAAGGTGGAGGATTTGAG GTGGGGAGGCTCACTTCCCGCTCCCAGGGCTCGAGGGCAGGGCAAGCCAGCTGGG ACTGCCAGATC (SEQ ID NO:82) |
| #89 | chr17:39822 111-39822231 | ATCGTGCTAGTTAAATGAATGAACATGTGGTGGTTACAGGCAAACCCACAATGCCA CACAGGACACCGACCAACAACTGAAAACGGTGACAGCTGCCCTTACCCGCAGCTCC CCAGCGTA (SEQ ID NO:83) |
| #90 | chr17:70614 530-70614650 | AGCTGAGCGAAACTCTTCCTCCTCCACGAGGCCTCTCCCAGCCCTTCAGTTCATGAC GCCTTGGTGAGAGAGGTCTGTATAGCAACACGGCTGCACTGAAGCTTCCTTTGTAG GTCGATC (SEQ ID NO:84) |
| #91 | chr17:70628 016-70628136 | ATCCGTGCCAGGGGGCCACTGAGCTCTCATTCCCAGAAGCCAACCAGAGCCAGTGA AGGGGCCGCAGAGACTCCGACACATCATTTTCATAAAAACAGCCCAAGGAGAGCAT TGCATTAG (SEQ ID NO:85) |
| #92 | chr18:36181 63-3618283 | ATCCTGTAAGTCAAAGACACAACCTGTTCCAGGAAGGACTTTGCAACAGCAATTACT CTAACAAGTTACAATTCGCAGAGTCCGGCCTTTAAGGGGCTTTGAAGAGAGAGAAA CCCATGA (SEQ ID NO:86) |
| #93 | chr18:36247 85-3624905 | ATCATATCACATCTGAGTTAGGCCATATGCACCCAGGGGGAATCTCAAGAGCAAAC TAATCTAAACTCCAAGAAATAGACCACTAAGACCCACCCAGGTAGTACTCACTGAAC GCTCTTC (SEQ ID NO:87) |
| #94 | chr18:20680 115-20680235 | AGATTCACTTAAATACATCATGAATGATAGACTGGAATATTTTTGAATCATATTCACC AAAACATTAGAAGTGAATCAGTAGTAGTGTGGTCTCTGAAGTCAACTTTAAAAGAA TTGATC (SEQ ID NO:88) |
| #95 | chr18:20682 099-20682219 | CCAGAGACACAAAGCAGTTGATGCTGTTCATGTTCTGGGGTTCACTCGCAAGCACTT GAAGGGAAACGACTGGACAGTTCCTGTGAAGACAGGGTTTTCTTGAGAGTTTCTAG GAAGATC (SEQ ID NO:89) |
| #96 | chr18:20686 589-20686709 | GTCCCCACCTTAGCTCCATTCTCACTCCCAGCTGTCAACTCTATGAGGTCAAGGTCTC TAACACAACTGGGTCCCTGGCGGCTCACAGCCGTGGCTGCTGGCACGGGAAAGATG CCGATC (SEQ ID NO:90) |
| #97 | chr19:42245 027-42245147 | TGATGCTGTCTGACTGAGGTCAAAAGGATGGGTGTGGAAGGCATCACACCTTTCTC CCATTTAGAAATCCATTGTCCCTTCCTTCTCCCTTATTGGGTTACATTCCTCTGTCCAT AGATC (SEQ ID NO:91) |
| #98 | chr19:42251 031-42251151 | CAACATCAGCCTCAGCCTGAACTCTGCCAGTAAACTTGTAACTTTCCAAGGAAACTT ACTCTACTGTAACAGTTCTTTTTCATCCGGAGACAAAATGTATCTGATTCGCAGTCAC CGATC (SEQ ID NO:92) |

Preprocessing of raw reads was performed to remove adaptor sequences (trim_galore, http://www.bioinformatics.babraham.ac.uk/projects/trim_galore/) and overlapping reads were merged using FLASH. In order to achieve short read mapping to the hg19 reference genome, the resulting preprocessed reads were then *in-silico* digested with DpnII and aligned using Bowtie (using p1, m2, best and strata settings). Aligned reads were processed using Capture-C analyser to (i) remove PCR duplicates, and (ii) classify sub fragments as 'capture' if they were contained within the capture fragment; 'proximity exclusion' if they were within 1kb on either side of the capture fragment; or 'reporter' if they were outside of the 'capture' and 'proximity exclusion' regions. Additionally, this study used the r3Cseq package on the capture and reporter fragments to identify significant interactions of the viewpoint against a scaled background (Q<0.05, FDR) and also to compare interaction profiles between different cell lines.

### 4C-seq and Data Analysis

4C templates were prepared using previously-published protocols with slight modifications. In brief, cultured cells were diluted into single-cell suspensions, and chromatin was cross-linked with 1% formaldehyde for 10 min at room temperature. Cells were lysed and cross-linked DNA was digested with the primary restriction enzyme HindIII-HF [R3104L, New England Biolabs (NEB)]. Next, HindIII-digested DNA was subjected to proximity ligation using T4 DNA ligase (EL0013, Thermo Scientific), followed by cross-link removal using Proteinase K (AM2546, Ambion), yielding 3C libraries. The 3C libraries were then subjected to a second restriction enzyme digestion using DpnII (R0543L, NEB), followed by a circularization reaction using T4 DNA ligase. For each viewpoint, 3.2 µg of the resulting 4C templates was used to perform a scale-up inverse, nested PCR (Table 4) of which 32 reactions (100 ng in each) were pooled and purified using the MinElute PCR Purification kit (Qiagen). 10 µg of the PCR products were then run on 4-20% TBE PAGE gels (5 µg per well). On the gel, smears from 200bp to 600bp were excised and unwanted PCR product bands were removed. DNA was then extracted from the cut-out gel pieces for next-generation sequencing on an Illumina Miseq (2x250 bp).

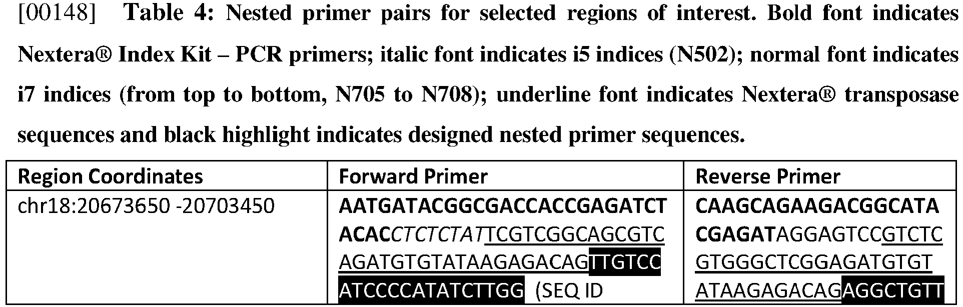

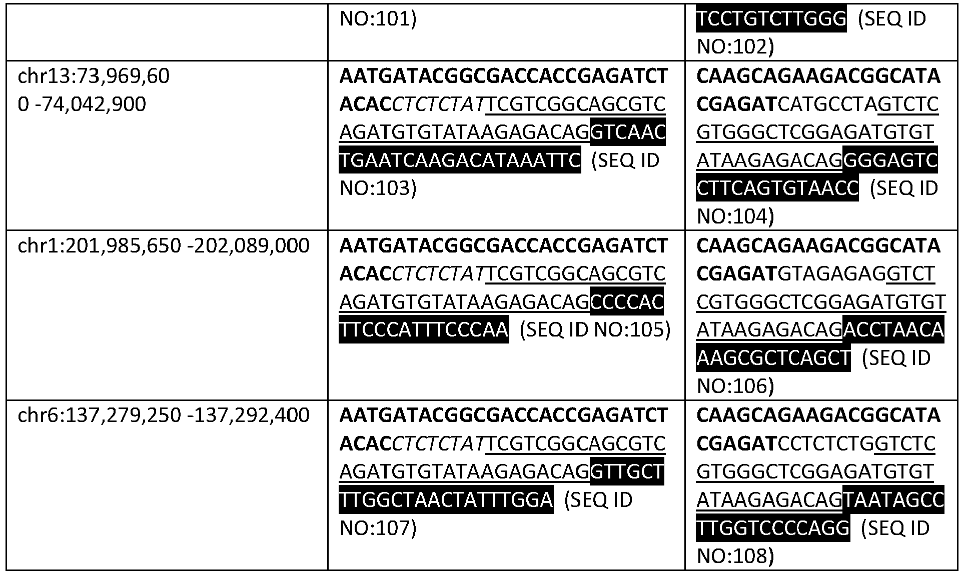

Inverse primers were designed based on a viewpoint concept. The UCSC Genome Browser [assembly: Feb. 2009 (GRCh37/hg19)] was used to locate the region of interest. Upon addition of HindIII and DpnII tracks, two HindIII restriction sites flanking the region of interest were identified and the sequence between the nearest HindIII and DpnII restriction sites were selected as the viewpoint region. Based on this region, two pairs of primers (outer and nested) were designed using the Primer-BLAST program [National Center for Biotechnology Information (NCBI)] with the following adaptations to the default settings: optimal primer melting temperature of 58 °C, with a minimum of 55 °C and maximum of 60 °C; GC content between 39 and 60%. Appropriate adaptors (Nextera^{®} Index Kit - PCR primer, Nextera^{®} transposase sequence) and index sequences were then added to the nested primer pair. Outer and nested primers used in this study are presented in Table 5 and Table 4 respectively.

**Table 5: Outer primer pairs (4C-seq) for selected regions of interest.**

| **Region Coordinates** | **Forward Primer** | **Reverse Primer** |
|---|---|---|
| chr18:20673650-20703450 | GTCTGCGCCTCAGGAAAAT (SEQ ID NO:93) | AAGGCTGTTTCCTGTCTTGG (SEQ ID NO:94) |
| chr13:73,969,600-74,042,900 | CCCTACCACTTTCCCTTTTC (SEQ ID NO:95) | TATGCAAGGGCATCAATTAGG (SEQ ID NO:96) |
| chr1:201,985,650-202,089,000 | CTTGAGGAACACAGAAGGGC (SEQ ID NO:97) | CAGCACTCTGCAAACAGACT (SEQ ID NO:98) |
| chr6:137,279,250-137,292,400 | AAAGTCTCTGCCATCTCCCAG (SEQ ID NO:99) | AAATCAAAGCTCAGAGGACT GG (SEQ ID NO:100) |

Primer sequences at the 5' ends of sequencing reads were trimmed using TagDust2, and mapped to the reference genome (hg19) using Bowtie2 (2.2.6). Unaligned reads were trimmed at the first 50 base pairs before realigning them to the reference genome. Only uniquely mapped reads with MAPQ≥30 were used in downstream analyses. Statistical significant interactions (Q<0.05, FDR) were detected using r3Cseq using a non-overlapping window approach (window size = 5kb). Signal plots of 4C data were generated using Basic4CSeq. Detected interactions within DNA amplified regions were excluded. Interactions were then mapped to genes, using promoters (+/- 2.5kb from annotated transcription start sites from GENCODE v19) overlapping with the interactions.

### CRISPR/Cas9 Enhancer Deletions

CRISPR sgRNA target search was performed using online software created by the Feng Zhang laboratory (http://tools.genome-engineering.org). sgRNA pairs were designed to target sequences flanking enhancers identified for deletion. Briefly, sequences corresponding to 100 bp upstream/20 bp downstream of the 5' end of the enhancer, and sequences corresponding to 20 bp upstream/ 100 bp downstream of the 3' end of the enhancer, were used for the search. Top hits with the lowest level of coding region off-target predictions were chosen. sgRNAs were cloned into the pSpCas9(BB)-2A-GFP or -Puro vectors (Addgene). Briefly, pairs of oligonucleotides were designed and procured from Integrated DNA Technologies, Inc. for each CRISPR target. Oligonucleotide pairs were then annealed to form DNA duplexes containing overhangs on both sides for ease of cloning. Guide RNAs used to target 5' ends of individual enhancers were cloned into Bbs I-digested pSpCas9(BB)-2A-GFP vectors, while sgRNAs targeting 3' ends of each enhancer were cloned into Bbs I-digested pSpCas9(BB)-2A-Puro vectors. The inserts and vectors were ligated using T4 DNA Ligase (New England Biolabs). DH5α cells were transformed with the ligation product and plated on LB agar supplemented with ampicillin. Colonies were picked and cultured, and plasmids extracted using the Wizard Plus SV Minipreps DNA Purification System (Promega). Sequences of plasmids were confirmed by performing Sanger sequencing. Oligonucleotides used for these experiments are listed in Table 6.

**Table 6: Primers used in enhancer deletion using CRISPR/Cas9 genome editing technology.**

| | | |
|---|---|---|
| Construct CRISPR sgRNA plasmids | e1-5'-1 | CACCgCCCCATGTCCCCATACAGGC (SEQ ID NO:109) |
| | e1-5'-2 | AAACGCCTGTATGGGGACATGGGGc (SEQ ID NO:110) |
| | e1-3'-1 | CACCGGCACACCAGGCAGGATTCC (SEQ ID NO:111) |
| | e1-3'-2 | AAACGGAATCCTGCCTGGTGTGCC (SEQ ID NO:112) |
| | e2-5'-1 | CACCgCGGGACTCAGACCTTAGTCA (SEQ ID NO:113) |
| | e2-5'-2 | AAACTGACTAAGGTCTGAGTCCCGc (SEQ ID NO:114) |
| | e2-3'-1 | CACCGAGGATTTCTTAAGCCCAGA (SEQ ID NO:115) |
| | e2-3'-2 | AAACTCTGGGCTTAAGAAATCCTC (SEQ ID NO:116) |
| | e3-5'-1 | CACCgTGAGGGAGGATAGGCGGGCC (SEQ ID NO:117) |
| | e3-5'-2 | AAACGGCCCGCCTATCCTCCCTCAc (SEQ ID NO:118) |
| | e3-3'-1 | CACCgCACCTAGAGGCCTGCTTTAG (SEQ ID NO:119) |
| | e3-3'-2 | AAACCTAAAGCAGGCCTCTAGGTGc (SEQ ID NO:120) |
| | e4-5'-1 | CACCGAAGAGAACTCCACCGGGTG (SEQ ID NO:121) |
| | e4-5'-2 | AAACCACCCGGTGGAGTTCTCTTC (SEQ ID NO:122) |
| | e4-3'-1 | CACCgCAGACATGACCTAGGTTCCC (SEQ ID NO:123) |
| | e4-3'-2 | AAACGGGAACCTAGGTCATGTCTGc (SEQ ID NO:124) |
| Determine deletion of enhancer (PCR) | e1-5'-F | GCCTTCCCTTCCTGATGTC (SEQ ID NO:125) |
| | e1-3'-R | TAATGGCAAGACTGGTATCCAC (SEQ ID NO:126) |
| | e2-5'-F | CTTGTGGTACTGTTCCCAGAC (SEQ ID NO:127) |
| | e2-3'-R | CAGCCTGGGAAGCATATTGA (SEQ ID NO:128) |
| | e3-5'-F | CTGGGTTCCCACCTGATAAT (SEQ ID NO:129) |
| | e3-3'-R | GATGAAATCCAAGTCATTGTGTCC (SEQ ID NO:130) |
| | e4-5'-F | CTTCTGGGTTCAAGTGAGTCT (SEQ ID NO:131) |
| | e4-3'-R | CATGAGCAAAGGTCCTCCTAC (SEQ ID NO:132) |
| Determine retention of enhancer after targeting (qPCR) | e1-int-F | CAGTAGGTACACCTGGCAATAG (SEQ 10 NO:133) |
| | e1-int-R | ATCCTGCTTCCTCTTGGAATATC (SEQ ID NO:134) |
| | e2-int-F | CCAGCTTCTTTCCTCTCCTTATC (SEQ ID NO:135) |
| | e2-int-R | GGTGAAATCCCATCTCCACTAAA (SEQ ID NO:136) |
| | e3-int-F | ATCCAGACACACCTGTAGGA (SEQ 10 NO:137) |
| | e3-int-R | CAGAACAAAGTCCAGAGAGAGG (SEQ 10 NO:138) |
| | e4-int-F | CCTGCCTCTCTTCTGCTTTC (SEQ ID NO:139) |
| | e4-int-R | GTTCATGCCCTGCCTTATCT (SEQ 10 NO:140) |

**Table 7: CDX2 candidate binding partners using PScanChIP and their expression correlations with CDX2 expression.**

| **Predicted Partner** | **Spearman Correlation** | **P-value** |
|---|---|---|
| HNF4A | 0.797 | 1.14E-07 |
| ISX | 0.796 | 2.29E-09 |
| ONECUT2 | 0.735 | 6.48E-07 |
| CDX1 | 0.699 | 2.40E-06 |
| HES2 | 0.698 | 2.54E-06 |
| FOXD2 | 0.687 | 3.78E-06 |
| HNF4G | 0.682 | 4.62E-06 |
| MYB | 0.682 | 4.78E-06 |
| CEBPG | 0.681 | 4.86E-06 |
| HNF1A | 0.660 | 1.12E-05 |
| MNX1 | 0.647 | 1.79E-05 |
| DMBX1 | 0.619 | 3.37E-05 |
| EVX1 | 0.616 | 3.80E-05 |
| SP8 | 0.589 | 9.92E-05 |
| VDR | 0.586 | 1.50E-04 |
| LEF1 | 0.584 | 1.56E-04 |
| HOXB13 | 0.582 | 1.25E-04 |
| KLF5 | 0.580 | 1.76E-04 |
| PDX1 | 0.576 | 2.03E-04 |
| HOXC11 | 0.570 | 1.85E-04 |
| HOXA9 | 0.565 | 2.78E-04 |
| KLF16 | 0.547 | 4.62E-04 |
| E2F2 | 0.542 | 5.38E-04 |
| HOXA11 | 0.524 | 7.41E-04 |
| HOXD13 | 0.523 | 7.54E-04 |
| TGIF1 | 0.503 | 1.49E-03 |
| ELF3 | 0.495 | 1.78E-03 |
| HOXA10 | 0.494 | 1.85E-03 |
| SREBF1 | 0.491 | 1.96E-03 |
| SP3 | 0.471 | 3.17E-03 |

SNU16 and OCUM-1 cells were grown to 80-90% confluence in RPMI supplemented with 10% FBS, 1xP/S and 0.5XNEAA. Cells were harvested and spun down, treated with Typsin for 5 min at 37 degrees, and re-suspended by pipetting to achieve single cell suspensions. Cell numbers were counted, and cells were washed once with 1xPBS before resuspension in Resuspension buffer (R) at 1×10⁷ cells/ml. For every 1×10⁷ cells in 1 ml of Resuspension buffer, 25 ug of pCas9-GFP-sgRNA and 25 ug of pCas9-Puro-sgRNA plasmid were mixed with SNU16 or OCUM-1 cells. 100 ul of each cell suspension was electroporated using a 100 ul Neon pipette in a Neon tube containing 3 ml of Electrolytic Buffer (E2). Electroporation conditions were: Pulse, V 1050, MS 30, Number 2. After electroporation, cells were plated onto 8ml of RPMI supplemented with 10% FBS, 1xP/S and 0.5XNEAA. At 24 hour after initial transfection, the cells were treated with 10 ug of Puromycin for 48 hours, and the remaining GFP-positive cells were sorted using FACS. The remaining surviving cells (both GFP-positive and Puromycin-resistant) were then subsequently analysed using qPCR to estimate knockout efficiencies.

Quantitative PCR (qPCR) was performed to determine the efficiency of deletion of individual enhancers in CRISPR/Cas9-targeted cells. Genomic DNA of targeted and untargeted cells (pooled) was extracted using the AllPrep DNA Micro Kit (QIAGEN) and subjected to qPCR in technical triplicates using KAPA SYBR FAST qPCR Master Mix (Kapa Biosystems) on a CFX96 Touch Real-Time PCR Detection System (Bio-Rad Laboratories, Inc.). Primers used in these reactions are listed in Table 6 (primers with "Int" in their names were used for this purpose). Relative amount of the specific targeted region present in the genomic DNA samples was calculated using the comparative C_{T} (ΔΔC_{T}) method, normalized to the *GAPDH* gene and relative to untargeted cells.

Genomic DNA was extracted from the sorted cells using a previously described protocol. Briefly, cells were triturated in 0.5x Direct-Lyse buffer (10 mM Tris pH 8.0, 2.5 mM EDTA, 0.2 M NaCl, 0.15% SDS, 0.3% Tween-20) and subjected to the following heating and cooling program: 65 °C for 30s, 8 °C for 30s, 65 °C for 1.5 min, 97 °C for 3 min, 8 °C for 1 min, 65 °C for 3 min, 97 °C for 1 min, 65 °C for 1 min, and 80 °C for 10 min. Subsequently, the lysates were diluted approximately 4x in water and 3 µl of the diluted lysates were used to perform 20-µl PCR reactions using Taq DNA Polymerase (Life Technologies). Primers used are in Table 6 (primer pairs of "5' F" and "3' R" for each enhancer).

### RT-qPCR to measure gene expression levels

Cells were FACS sorted for GFP positive cells and total RNA was extracted from cells using AllPrep DNA/RNA Micro Kit (QIAGEN). Reverse transcription was performed on pooled cells using iScript Select cDNA Synthesis Kit (Bio-Rad) with random primers. qPCR was conducted using TaqMan Gene Expression Master Mix and TaqMan probes (Applied Biosystems) on a CFX384 Touch Real-Time PCR Detection System (Bio-Rad). All qPCR experiments were run in triplicate, and mean values were used to determine mRNA levels. Relative quantification was performed using the comparative CT method with *GAPDH* as the reference gene and with the formula 2-ΔΔC_{T}.

### Copy Number Alterations and DNA Methylation

Genomic DNAs from gastric tumors and matched normal gastric tissues were hybridized on Affymetrix SNP6.0 arrays. (Affymetrix, Santa Clara, California, USA). Data in .CEL format was processed in the following order: (1) Normalization: Raw .CEL files were processed using Affymetrix Genotyping Console 4.2. Reference models were created from SNP6.0 profiles of normal gastric tissues according to the hybridization batch. Copy number changes in cell lines and primary tumor samples were determined by using the reference model from primary normal samples. (2) Segmentation: Copy number segmentation data was produced using the circular binary segmentation (CBS) algorithm implemented in the DNAcopy R package. The p-value cutoff for detecting a change-point was 0.01, with a permutation number of 10,000. Copy number gain and loss regions were defined for showing average log ratios of > 0.6 and < -1.0, respectively. Illumina HumanMethylation450 (HM450) Infinium DNA methylation arrays were also used to assay DNA methylation levels. Methylation β-values were calculated and background corrected using the methylumi R BioConductor package. Normalization was performed using the BMIQ method (watermelon package in R).

### RNAseq and Analysis

Total RNA was extracted using the Qiagen RNeasy Mini kit. RNA-seq libraries were constructed according to manufacturer's instructions using Illumina Stranded Total RNA Sample Prep Kit v2 (Illumina, San Diego, California, USA) Ribo-Zero Gold option (Epicentre, Madison, Wisconsin, USA) and 1 ug total RNA. Completed libraries were validated with an Agilent Bioanalyzer (Agilent Technologies, Palo Alto, CA) and applied to an Illumina flow cell via the Illumina Cluster Station. Sequencing was performed using the paired-end 101bp read option. RNA-seq reads were aligned to the human genome (hg19) using TopHat2-2.0.12 (default parameter and --library-type fr-firststrand). The per base sequence quality and per sequence quality scores of the mapped reads was assessed using FastQC version 0.10.1 (http://www.bioinformatics.babraham.ac.uk/projects/fastqc/). Transcript abundances at the gene level were estimated by cufflinks. Gene expression from primary samples showing variation greater than zero were corrected for potential batch effects using ComBat. Gene expression values were measured in FPKM units. Differential expression between groups was identified as genes showing altered expression by at least two-fold and absolute differences of 0.5 FPKM.

### Survival analysis

GC samples from 7 independent studies were clustered using a K-medoids approach. Only genes with expression values in all 7 studies were used in analyses. Kaplan-Meier survival analysis was employed with overall survival as the outcome metric. Log-rank tests were used to assess the significance of Kaplan-Meier curves. Multivariate anaysis involving additional variables, such as age, tumor stage, Lauren's histological subtypes and locality (Asian vs Non-Asian) was performed using Cox regression.

### Disease-associated SNP Analysis

Trait-associated SNPs were downloaded from the UCSC browser of genome-wide association studies (27th August 2015). For this study, we focused on SNPs occurring in noncoding regions and excluded SNPs within coding regions. Overlaps between SNPs from each trait/disease and somatic predicted super-enhancers were computed using BEDtools 'intersect' (nGWAS), and compared nGWAS against the total number of disease-associated SNPs outside the predicted super-enhancers (nGWAS'). As an additional control, a "SNP background" model was created using a set of all SNPs from two commonly-used SNP arrays (Illumina HumanHap550 and Affymetrix SNP6). The number of SNPs from the SNP background overlapping the predicted super-enhancers was calculated (nBackground) and compared against the total number of background SNPs outside the predicted super-enhancers (nBackground'). The ratio of normal SNPs in predicted super-enhancers was computed as nBackground/nBackground'. Expecting that the increased number of disease-associated SNPs in predicted super-enhancers is associated with a high prevalence of SNPs in these regions, our null hypothesis is therefore that there is no difference between the ratio of disease-associated SNPs and the ratio of normal SNPs (enrichment ratio). Chi-square tests were conducted, with enrichment p-values of < 0.01 considered statistically significant. To understand the relationship between risk-associated SNPs and histone modification, the study identified validated SNPs in gastrointestinal diseases (eg ulcerative colitis and colorectal cancer) found to be associated with disease in at least two independent studies. Samples were classified into two groups based on the presence of the disease-associated SNPs, using GATK Unified Genotyper. Differences of H3K27ac signals between tumor and matched normal in samples with or without disease-associated SNPs were compared.

### Transcription Factor Binding Motif Analysis

The study interrogated enrichments of transcription factors in somatic gain predicted super-enhancers and unaltered predicted super-enhancers using the ReMap database. Transcription factor binding sites with at least 60% of overlap with predicted super-enhancers were counted, and the ranks of the top 10 most enriched transcription factors compared. Binding densities of transcription factors were computed as the total binding sites detected in the regions divided by the total size of the regions in unit of million base pairs (Mbp). For CDX2, CDX2 binding sites were examined in recurrently gained somatic predicted super-enhancers to predict nearby binding of other transcription factors, using HOMER with default parameters. The top 20 transcription factor identified from the HOMER outputs were used for expression correlation analysis. Additionally, CDX2 co-binding motifs were also identified using PScanChIP with JASPAR 2016. Expression correlations (Spearman's correlation) between CDX2 and potential co-binding partners were evaluated.

### siRNA Transfection

ON-TARGETplus Human siRNA SMARTpools (*HNF4α* and *CDX2*)*,* individual ON-TARGETplus Human individual siRNAs (*HNF4α*) and ON-TARGETplus Non-targeting siRNA controls (Dharmacon/Thermo Fisher Scientific) were used to transfect cells (2×10⁵) at 50nM in 6-well plate, using Dharmafect 1 transfect reagent, according to the manufacturer instructions. Knockdown efficiency after 72hrs' RNAi treatment was examined using quantitative RT-PCR and/or Western Blot analysis (Fig.23).

### Western Blotting

Cells (2×10⁵) were harvested in RIPA buffer (Sigma) and lysed for 10mins on ice. Concentration of supernatants were measured using Pierce BCA protein assay (Thermo Scientific). CDX2 (1:500; MU392A-UC, Biogenex), HNF4α (1:1000; sc-8987, Santa Cruz Biotechnology) and GAPDH (1:3000; 60004-1-Ig, Proteintech Group) antibodies were used to probe the lysate.

### Quantitative RT-PCR

Total RNA was isolated using RNeasy Mini Kit (Qiagen), and DNA was removed using RNase-Free DNase Set (Qiagen). 2ug RNA was reverse transcribed using Superscript III First Strand Synthesis System (Invitrogen), and complementary DNA was amplified using SYBRGreen PCR Master Mix (Applied Biosystems). Fold changes were normalized to *GAPDH.* Primer sequences are as follows: HNF4α: F1-5' GTGCGGAAGAACCACATGTACTC 3' (SEQ ID NO:143), R1- 5' CGGAAGCATTTCTTGAGCCTG 3' (SEQ ID NO:144), F2-5' CTGCAGGCTCAAGAAATGCTT 3' (SEQ ID NO:145), R2- 5' TCATTCTGGACGGCTTCCTT 3' (SEQ ID NO:146), F3- 5' TGTCCCGACAGATCACCTC 3' (SEQ ID NO:147), R3- 5' CACTCAACGAGAACCAGCAG 3' (SEQ ID NO:148); CDX2: F1- 5' GCAGCCAAGTGAAAACCAGG 3' (SEQ ID NO:149), R1- 5' CCTCCGGATGGTGATGTAGC 3' (SEQ ID NO:150), F2- 5' AGTCGCTACATCACCATCCG 3' (SEQ ID NO:151), R2- 5' TTCCTCTCCTTTGCTCTGCG 3' (SEQ ID NO:152); GAPDH: F - 5' CCAGGGCTGCTTTTAACTC 3' (SEQ ID NO:153), R - 5' GCTCCCCCCTGCAAATGA 3' (SEQ ID NO:154).

### CDX2 and HNF4α ChIP-seq and Analysis

Cells were cross-linked with 1% formaldehyde for 10 minutes at room temperature, and stopped by adding glycine to a final concentration of 0.2M. Chromatin was extracted and sonicated to 500bp. CDX2 (MU392A-UC, Biogenex) and HNF4α (sc-8987, Santa Cruz Biotechnology) antibodies were used for chromatin immunoprecipitation (ChIP). ChIPed DNA (10ng) was used for ChIP with DNA sequencing (ChIP-seq) library construction following manufacturer protocols (New England Biolabs). Input DNA from cells prior to immunoprecipitation was used to normalize ChIP-seq peak calling. Prior to sequencing, qPCR was used to verify that positive and negative control ChIP regions amplified in the linear range. Size distributions of the library samples were checked using a Bio-analyzer (Agilent Technologies). In an initial analysis comparing recurrently gained predicted super-enhancers specific to intestinal and diffuse-type GCs (10 intestinal, 6 diffuse), there was no observed significant differences in CDX2 binding between the two subtypes. A deeper analysis revealed, however, high within-subtype variability in *CDX2* expression between individual tumors of the same subtype, consistent with previous reports that CDX2 expression is not absolutely associated with intestinal subtype GC. This study thus performed a complementary analysis where the GCs were ordered by their individual *CDX2* expression levels and examined. CDX2 binding density were then computed at recurrent somatic gain predicted super-enhancers identified in GC samples showing high (n=8) and low (n=8) *CDX2* expression. In differential binding signal analysis, binding signals for CDX2 and HNF4α were computed for 200 bins spanning those predicted super-enhancers showing somatic gain or no alteration in primary samples and also detected in OCUM-1 or SNU16 cell lines. Signals were measured in RPKM units. To estimate the effect of transcription factor (TF) knockdown on H3K27ac strength, an internal control was defined comprising observed variation of H3K27ac signals between independent wild type (WT) samples. The differences between WT samples against TF-silenced (siCDX2, siHNF4α, or double TF) samples were measured, which were then compared against this background variation. Sub-regions with differences > 99% of background variation were termed as H3K27ac depletion; while differences < 1% of the background variation were termed as H3K27ac gain. Statistical enrichments of H3K27ac-depleted sub-regions corresponding to predicted super-enhancers were conducted using one-sided Fisher's exact test. To study relationships between the differential regions and their distances to nearby CDX2/HNF4α binding sites, the regions were also segregated into three categories (near, moderate, distal) based on their distance distribution. Mid-point locations between the CDX2 and HNF4α summits were used to analyse distances between H3K27ac-depleted sub-regions and CDX2-HNF4α cobinding sites. To study associations between gene expression and somatic gain predicted super-enhancers in TF-silenced cells, we selected genes linked to predicted super-enhancers exhibiting significant positive expression correlations with H3K27ac predicted super-enhancer signals in primary samples (r > 0.4; P < 0.05, two-sided t-test) and also observed in the GC cell lines. To assess the significance of transcription factor knockdown on predicted super-enhancer target gene expression, a permutation approach was used. Specifically, focusing on predicted super-enhancers exhibiting H3K27ac depletion after TF silencing, we permuted the actual super-enhancer to gene assignments 10,000 times. An empirical P-value was then derived by counting the number of times the number of down-regulated genes in the permuted gene/super-enhancer set exceeded the experimentally observed number of down-regulated genes in the actual gene/super-enhancer set.

### Data availability

Histone NanoChIP-seq (GSE76153 and GSE75898), SNP array (GSE85466), RNA-seq (GSE85465) and DNA methylation data (GSE85464) generated during this study have been deposited in Gene Expression Omnibus. Previously deposited histone ChIP-seq (GSE51776 and GSE75595) and SNP array (GSE31168 and GSE36138) data that are used in this study are available in Gene Expression Omnibus. Chromatin accessibility profiles of normal gastric tissues from Epigenome Roadmap were obtained from the Gene Expression Omnibus (GSM1027325, GSM1027320). RNAPII ChIA-PET data analyzed in this study was obtained from *encodeproject.org* and Gene Expression Omnibus (GSE72816).

### Results

### Distal Predicted Enhancer Landscapes of GC Cell Lines

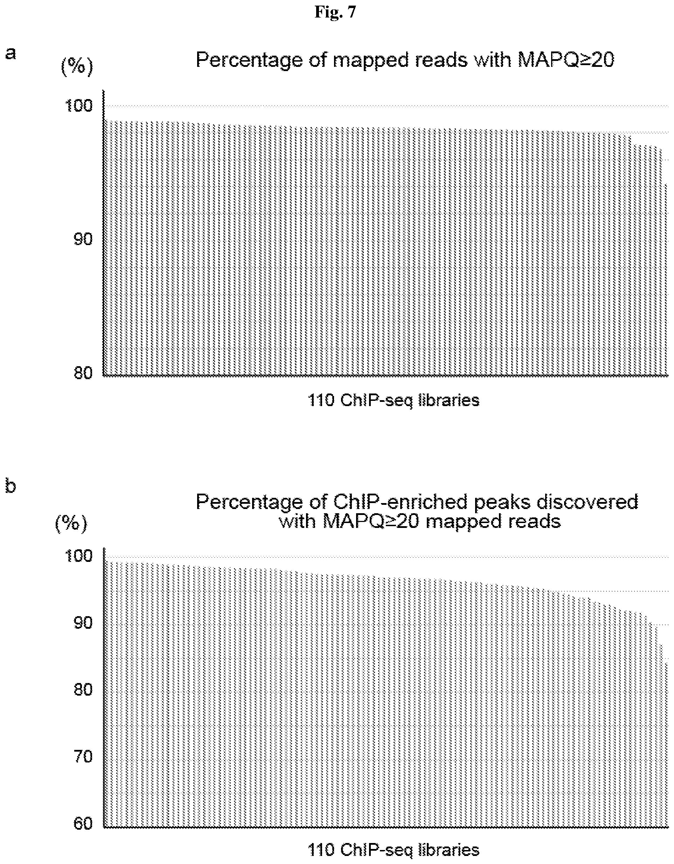

Using Nano-ChIPseq, 110 chromatin profiles from 19 primary GCs, 19 matched normal gastric tissues, and 11 GC cell lines covering multiple histone H3 modifications (H3K27ac, H3K4me3, H3K4mel) (average -3.3 × 10⁷ reads per profile) were generated. Clinical information and molecular classification of the primary GCs is presented in Table 8, sequencing statistics in Table 1, and clinico-pathological details for the GC lines in Table 9. The series included 10 gland forming adenocarcinomas (53%, intestinal type), 6 samples with highly infiltrating isolated cells (32%, diffuse type) and 3 GC samples (15%) of mixed histology. More than 60% of the tumors (n=12) were Stage 3 or above (AJCC 7^{th} edition). Extensive quality control analysis of the Nano-ChIPseq data was performed, including variations in mapping quality filters, analysis of biological replicates and promoter ChIP-enrichment, and assessment by the quality control software CHANCE (CHip-seq ANalytics and Confidence Estimation). Increasing mapping threshold stringencies (from MAPQ ≥10 to 20) did not appreciably alter mapping statistics - >90% of the total mapped reads were retained, and 85% of ChIP-enriched peaks and 98% of predicted enhancers were rediscovered respectively (Fig. 7). Histone peak concordance between biological replicates of KATO-III cells generated by Nano-ChIPseq, and also against independent KATO-III H3K27ac data generated by conventional ChIP-seq, confirmed high reproducibility (overlaps of -85% and ~90%) (Fig. 8). Comparisons of input and input-corrected H3K27ac and H3K4me3 signals at 1,000 promoters associated with highly expressed protein-coding genes revealed successful enrichment in 48 out of 50 (96%) H3K27ac and 42 out of 42 (100%) H3K4me3 libraries respectively. CHANCE analysis of ChIP enrichment, particularly for H3K4mel (which is depleted at promoters), revealed that the large majority (85%) of samples exhibited successful enrichment (Methods). These results demonstrate the good technical quality of the Nano-ChIPseq cohort. Besides Nano-ChIPseq, the samples were also processed for DNA methylation analysis (Infinium HumanMethylation 450K BeadChip arrays), copy number analysis (Affymetrix SNP arrays) and Illumina RNA-sequencing.

**Table 8: Clinical information of patients used in histone ChIP-seq, RNA-seq, Affymetrix SNP6.0 arrays and Infinium HumanMethylation 450K BeadChip arrays.**

| **Patient ID** | **Tumor content** | **Molecular Subtype** | **Lauren's classification** | **AJCC7** |
|---|---|---|---|---|
| **2000085** | 95% | GS | intestinal | 1B |
| **2000639** | 60% | GS | intestinal | 4 |
| **2000721** | 70% | GS | diffuse | 4 |
| **2000877** | >90% | CIN | intestinal | 2A |
| **2000986** | 80% | GS | diffuse | 4 |
| **2001206** | 90% | CIN | diffuse | 4 |
| **20020720** | 80% | CIN | intestinal | 2A |
| **20021007** | 85% | GS | intestinal | 2A |
| **76629543** | 80% | CIN | intestinal | 3A |
| **980097** | 70% | EBV | mixed/OTHER S | 2A |
| **980319** | 70% | GS | mixed/OTHER S | 3A |
| **980401** | 90% | GS | diffuse | 3A |
| **980417** | 80% | GS | diffuse | 3C |
| **980436** | 80% | GS | intestinal | 3A |
| **980437** | 90% | CIN | intestinal | 3C |
| **980447** | 40% | CIN | intestinal | 4 |
| **990068** | 95% | GS | diffuse | 3B |
| **990275** | 50% | CIN | intestinal | 2B |
| **990489** | 90% | | CIN | mixed/OTHER 1B S |

**Table 9: Gastric cancer cell line information**

| **No.** | **Cell-line** | **Histological sub-type of GC** | **Derivation** |
|---|---|---|---|
| 1 | FU97 | Diffuse adenocarcinoma;poorly differentiated; Lymph node metastasis and pancreatic metastasis were observed | primary stomach |
| 2 | KATOIII | SRCC | pleural effusion |
| 3 | MKN7 | Well-differentiated tubular adenocarcinoma | lymph node |
| 4 | NCC-59 | Moderately differentiated tubular adenocarcinoma | primary stomach |
| 5 | OCUM-1 | poorly differentiated adenocarcinoma containing signet ring cells; | pleural effusion |
| 6 | RERF-GC-1B | Adenocarcinoma; | pyloric lymph nodes |
| 7 | SNU16 | gastric carcinoma; poorly differentiated | metastatic ascites |
| 8 | YCC-21 | Adenocarcinoma | ascites |
| 9 | YCC-22 | Adenocarcinoma | ascites |
| 10 | YCC-3 | Poorly differentiated | ascites |
| 11 | YCC-7 | Adenocarcinoma | ascites |

GC cell lines were chosen as a discovery cohort to discover cancer-associated distal enhancers in GC, as cell lines are purely epithelial in nature, have the highest data quality, and because previous studies have shown that stromal contamination in primary tissues can influence genomic results. The study also focused on recurrent epigenetic alterations present in multiple GC samples, which reduces the introduction of "private" epigenetic alterations associated with individualized cell line features. First, genome-wide *cis*-regulatory elements were mapped based on H3K27ac signals, previously shown to mark active promoters and enhancers. To enrich for enhancer elements, the study focused on H3K27ac signals located distant from known annotated transcription start sites (TSSs; >2.5 kb) (Fig. 1a). The study then further refined the enhancer predictions using aggregated H3K4mel and H3K4me3 data, excluding from analysis predicted enhancers exhibiting high H3K4me3/H3K4me1 log ratios (>2.4). Using this approach, 3,017 to 14,338 putative distal enhancers were identified in the GC lines (Fig. 1b), with an average genomic footprint of 25 Mb/line.

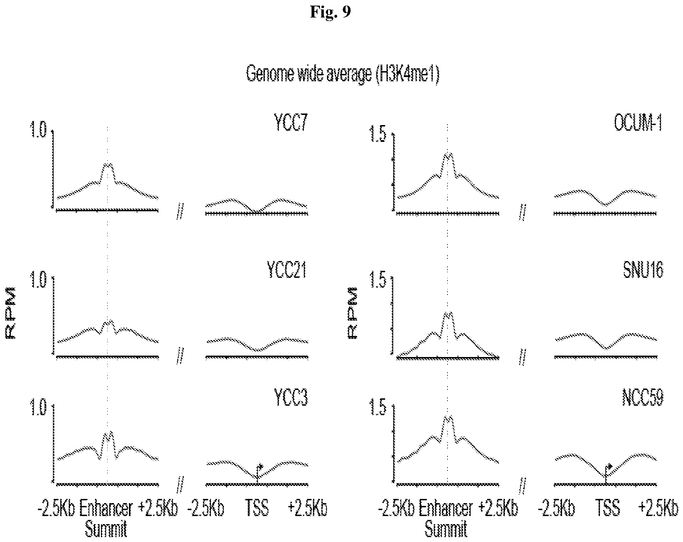

In total, the study detected 36,973 predicted distal enhancer regions, spanning ~140Mb or approximately 5% of the human genome. The predicted enhancers exhibited a bimodal H3K27ac signal distribution (Fig. 1b), were depleted of H3K4me3, and were enriched in H3K4mel signals (Fig. 1c and Fig. 9). Visual comparison of these H3K27ac-enriched regions revealed that some regions were active in multiple lines ("recurrent") while other regions were active in only 1 line ("private"). Approximately 47% of the predicted enhancers were recurrent, exhibiting activity in at least two GC cell lines (Fig. 1d). The percentage of recurrent enhancers was significantly lower compared to promoters (67% vs 47%, P < 2.2×10⁻¹⁶, one-sided proportion test), indicating that enhancer activity is highly variable across GC cell lines.

The predicted enhancers were validated by integrating publicly available epigenomic datasets. Using DNase I hypersensitivity (DHS) data of normal gastric tissues from the Epigenome Roadmap, it was found that DHS signal distributions (log-transformed RPKM) at predicted enhancers were significantly greater than randomly selected regions (P< 2.2×10⁻¹⁶, one-sided Welch's t-test; Fig. 1e, Methods), indicating that predicted enhancers are associated with open chromatin. When compared against DHS and H3K27ac data of 9 different tissue and cellular categories, predicted enhancers exhibited the highest overlap with DHS-positive and H3K27ac-positive regions from digestive and epithelial tissues (fetal intestine, gastric, and small intestine), and were distinct from non-epithelial tissue types such as blood and T-cells (Fig. 1f). Supporting their regulatory potential, 54% of the predicted enhancers (n=20,127) were associated with EP300 binding sites (Fig. 1g; P<0.001, empirical test), and 92% with transcription factor (TF) binding sites. At the DNA sequence level, 63% of the predicted enhancer sequences were evolutionarily conserved (Fig. 1h; P<0.0001, empirical test).

### Super Enhancers are Enriched in Cancer Signatures

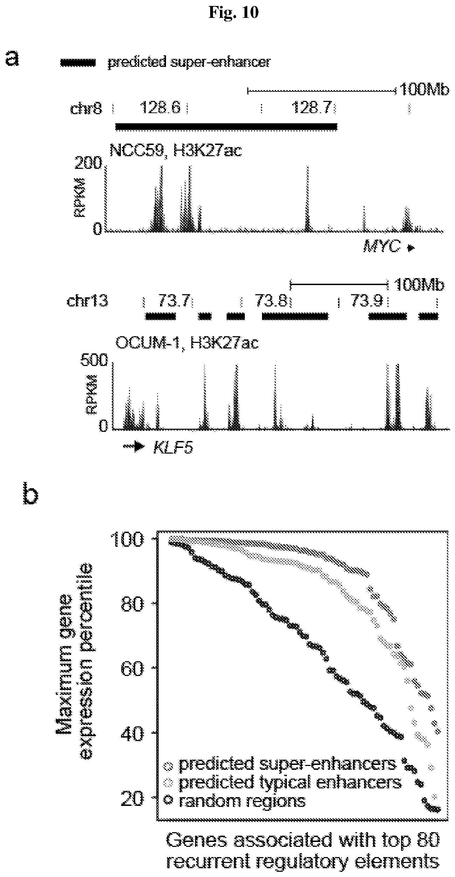

Using the ROSE algorithm, 133 to 1,318 predicted super-enhancers per GC line were identified, collectively encompassing 3,759 non-redundant predicted super-enhancers (Fig. 2a). It is thus estimated that about 10% of GC cell line predicted enhancers are associated with predicted super-enhancer activity. Compared to predicted typical enhancers, predicted super-enhancers exhibited a significantly greater tendency to be recurrent (Fig. 2b; one-sided proportion test, P< 2×10⁻¹⁶), with 3,345 predicted super-enhancers being active in at least two GC cell lines. It was observed that predicted super-enhancers associated with known protein-coding GC oncogenes (eg *MYC* and *KLF5;* Fig. 10a) and also at non-protein-coding gene regions such as at the *MALAT1* locus (Fig. 2c), which encodes a long-noncoding RNA (IncRNA) recently shown to promote GC proliferation.

Predicted super-enhancers were assigned to target genes based on regions exhibiting the nearest active TSS (defined as H3K27ac enrichment at promoters, within 500 bp of an annotated TSS). Only 53% of the predicted super-enhancer/gene interactions involved the closest proximal gene (see Methods, mean distance 76 kb). The predicted super-enhancer/gene assignments were validated using three orthogonal interaction data sets: (i) pre-determined interactions predicted by PreSTIGE, (ii) GREAT, and (iii) published RNAPII ChIA-PET data (encodeproject.org, GSE72816). Of 2,677 predicted interactions with protein-coding genes, 88% were supported by at least one of these three data-sets (Fig. 11). This number is likely a lower limit as the biological samples for the latter validation data in i)-iii) did not involve gastric tissues (see subsequent sections). To understand biological themes associated with the predicted super-enhancers, the study applied GOrilla pathway analysis and found that biological processes plausibly related to cancer development, such as regulation of signal transduction, programmed cell death, and cell proliferation were strongly associated with predicted super-enhancer linked genes (p-values 6.7×10⁻²² to 2.3×10⁻¹³ , hypergeometric test by GOrilla) (Fig. 2d). Many of these processes (eg regulation of programmed cell death, cell proliferation) remained significantly associated when the recurrent predicted super-enhancers were analysed by GREAT, indicating that that these enrichments are not due to biases toward genes flanked by large intergenic regions (Fig. 12). Similar analyses employing genes linked to the top predicted typical enhancers yielded a lesser degree of enrichment (Fig. 2d). Predicted super-enhancer associated genes were also enriched for oncogenes (P=1.7×10⁻⁸, one-sided Fisher's exact test). When correlated to gene expression, genes associated with recurrent predicted super-enhancers and typical enhancers were both significantly correlated with RNA expression (Fig. 10b).

### Super Enhancer Heterogeneity in Primary Tumors

To determine which cell line predicted super-enhancers are also associated with somatic alterations *in vivo,* the study compared H3K27ac enrichment levels for these regions across 19 primary GCs and matched normal gastric tissues. While previous studies have suggested the presence of distinct molecular subtypes of GC, due to limited sample sizes the current study elected to focus on predicted enhancer differences conserved in multiple GC tissues relative to matched normal tissues (see Discussion). Prior to analysis, it was confirmed that the primary gastric normal samples were indeed reflective of gastric epithelia, by correlating against published profiles ( see section "Comparisons of primary gastric non-malignant samples to Epigenome Roadmap"). Of 3,759 cell line predicted super-enhancers, two-thirds exhibited differential enrichment between tumors and matched normal samples (Fig. 3a, Table 2, referred to as somatically altered thereafter). Close to half of the predicted super-enhancers, n=1,748; 47%) exhibited somatic gain in 2 or more primary GCs (> 2-fold enrichment in tumor, minimum 0.5 RPKM difference), and principal component analysis (PCA) using these gained predicted super-enhancers confirmed separations between GC and matched normal tissues (Fig. 3b). Supporting the consistency of these results, the vast majority of these recurrent somatic gain predicted super-enhancers (85%, >1.5 fold change threshold) were rediscovered when using only those normal/tumor (N/T) primary pairs passing all quality control criteria (14 pairs, see earlier). Unexpectedly, despite their activity in cancer cell lines, a substantial proportion of predicted super-enhancers (18%) were associated with somatic loss rather than gain in primary GCs (Fig. 3a). It is possible that these latter regions may represent regions epigenetically silenced in primary tumors but reactivated in cell lines during *in-vitro* culture (Fig. 13a). 11% of the predicted super-enhancers (n=416) exhibited unaltered H3K27ac levels between GCs and normal tissues (Fig. 3a, Fig. 13b), consistent with these regions not being cancer-associated but related to "housekeeping" or general tissue functions. Finally, 21% (n=808) of cell-line predicted super-enhancers did not exhibit sufficient H3K27ac enrichment (RPKM < 0.5) in primary samples for analysis (Fig. 13c). Interestingly, this class was also associated with low recurrence in GC lines (Fig. 3a - histogram in black). Taken collectively, these results demonstrate that predicted super-enhancers derived from cell lines can be further subclassified using histone modification data from primary tumors and matched normal controls into at least 3 categories - somatic gain, somatic loss, and unaltered. A list of the top 100 somatic predicted super-enhancers is presented in Table 2.

Supporting their biological distinctiveness, predicted super-enhancers belonging to the three categories also exhibited other epigenetic differences *in vivo.* For example, predicted super-enhancer alterations in H3K27ac were similarly correlated with H3K4mel enhancer mark alterations (Fig. 3c), and at the DNA methylation level somatic gain predicted super-enhancers exhibited significantly lower DNA methylation levels, while somatic loss super-enhancers exhibited increased DNA methylation (P = 3.8×10⁻²²⁹, one-sided Welch's t-test). Unaltered predicted super-enhancers occupied an intermediate range (Fig. 3d). As a visual example, decreased DNA methylation (indicated by a lower beta value) was observed in GC T2000721 compared to its matched normal (N2000721), mapping to a somatic gain predicted super-enhancer at the *ABLIM2* locus (Fig. 3e). In contrast, somatic loss of H3K27ac signals at a *SLC1A2* predicted super-enhancer in T2000639 showed increased DNA methylation compared to N2000639 (Fig. 3f). These results further support the biological and molecular heterogeneity of predicted super-enhancers in gastric tissues.

### Super Enhancers Exhibit Complex Chromatin Interactions

Integration with copy number data revealed that the majority of somatic predicted super-enhancers are localized to copy number neutral regions (Fig. 14a-c, section titled "Associations between copy number alterations and predicted super-enhancers in gastric cancer). To examine associations between predicted super-enhancers and gene expression, the study interrogated RNA-seq information from the same primary samples, using the same predicted super-enhancer/gene assignments as the previous pathway analyses (Fig. 2). Somatic gain predicted super-enhancers were associated with elevated gene expression relative to matched normal samples, while somatic loss predicted super-enhancers were associated with decreased expression (P<2.2×10⁻¹⁶, one-sided Welch t-test; Fig. 4a).

Previous research has also shown that enhancers are often involved in long-range chromatin interactions that may influence the expression of multiple genes. To identify long-range interactions associated with somatic predicted super-enhancers in GC, the study applied Capture-C technology to survey interactions for 36 predicted super-enhancers, selected from regions exhibiting both recurrent somatic gains in primary tumor samples and also demonstrating activity in GC lines. Analysing three GC cell lines (OCUM-1, SNU16, KATO-III), multiple genomic locations (n=92, referred to as "capture points") across the 36 predicted super-enhancers were probed, identifying 88 capture points with significant interactions (Q<0.05, r3Cseq package). Figure 4b depicts 12 representative predicted super-enhancers covering 20 capture points. On average, each predicted super-enhancer exhibited 20-26 and 5-7 interactions with other genomic locations and promoters respectively. The average distance between capture points and detected interactions was approximately 17.0 kb (standard deviation: 30.5 kb). The study also identified longer-range interactions, including a predicted super-enhancer interaction with the *TM4SF4* promoter at a distance of -100 kb in OCUM-1 cells (Fig. 15). Notably, for regions with informative interaction data, the availability of experimental Capture-C information also allowed further validation of 93% (n=62) of the original predicted super-enhancer/gene interactions. Integration of expression data from the cell lines revealed that -70% of the interacting promoters are associated with detectable gene expression (FPKM > 0).

As a representative example, Figure 4c depicts the long-range interaction landscape of the *CLDN4* genomic region in SNU16 cells (Fig. 16 for other examples). This region was selected as *CLDN4* expression has been previously associated with GC progression and prognosis, and recurrent gain of the *CLDN4* predicted super-enhancer was observed in multiple primary GCs (Fig. 14d). Specifically, the study sought to investigate interactions involving two predicted sub-super-enhancer regions exhibiting high H3K27ac signals and also CDX2 and HNF4α co-binding (see later). Besides interactions with the *CLDN4* promoter, interactions were also detected with other distal promoters (up to ~100kb) such as *WBSCR27, CLDN3, ABHD11 and ABHD11*-*AS1. ABHD11-AS1* is a long non-coding RNA was previously shown to be highly expressed in gastric cancer. To validate the Capture-C data, the study also performed circularized chromosome conformation capture assays (4C) on 4 selected predicted super-enhancers in two GC lines (OCUM-1, SNU16) (Fig. 17). A concordance between Capture-C and 4C data of 75% was observed, similar to concordance rates between 4C experimental replicates (Fig. 18). Due to the significantly greater depth of 4C sequencing, additional interactions were also identified, such as a long-range interaction between a predicted super-enhancer and the *KLF5* promoter at a distance of ~350kb (Fig. 17b).

Previous reports have suggested that certain long-range interactions are associated with super-enhancer activity, while other interactions are more invariant and reflective of cell lineage. In agreement with these findings, of 22 (out of 36) predicted super-enhancers displaying differential activity between the GC lines, 4 predicted super-enhancers exhibited a good correlation between predicted super-enhancer activity and the presence of long-range interactions (Fig. 4d and Fig. 19). For the remaining 18 predicted super-enhancers, long-range interactions were observed independent of predicted super-enhancer activity.

To investigate a causal role between the predicted super-enhancers and gene expression, the study used CRISPR/Cas9 genome editing to delete two enhancer regions (e1 and e2; see Fig. 4c) within the *CLDN4* predicted super-enhancer region. After confirming CRISPR deletion efficiencies in OCUM-1 and SNU16 cells (Fig. 20a-c), predicted target gene expression levels between enhancer-deleted and wild-type cells were compared by RT-qPCR. In both cell lines, e1 CRISPR-deletion caused down-regulation of multiple *CLDN4* locus genes, including *ABHD11, CLDN3,* and *CLDN4* (*CLDN4* in SNU16 cells, Fig. 20d). In a similar fashion, the study also observed *ABHD11, CLDN3,* and *CLDN4* downregulation after e2 deletion in OCUM-1 cells (e2-deleted SNU16 cells were not viable, hence precluding gene expression analysis; Fig. 20e). To extend these results, the study then CRISPR deleted two other predicted enhancer elements (e3 and e4) from the *ELF3* predicted super-enhancer in OCUM-1 cells (Fig. 17a, Fig. 20c), as *ELF3* has been reported as a cancer gene in several malignancies. Both e3 and e4 deletion resulted in down-regulation of multiple *ELF3* locus genes including *ARL8A, ELF3, RNPEP* and *TIMM17A* (Fig. 20f). Taken collectively, these results support a causal relationship between predicted super-enhancer activity and tumor gene expression.

### Somatic Super Enhancers and Clinical Outcome

To further explore the biological and clinical relevance of predicted super-enhancer heterogeneity, the study performed cancer hallmark analysis categorized by somatic modification status (gained, lost, unaltered). Of ten cancer hallmarks, somatic gain predicted super-enhancers were significantly enriched in genes related to invasion (P = 8.6×10⁻¹¹, one-sided Fisher's exact test), angiogenesis (P = 2.4×10⁻⁴, one-sided Fisher's exact test) and cell death resistance (P = 7.8×10⁻³, one-sided Fisher's exact test), exceeding somatic loss and predicted unaltered super-enhancers by an order of magnitude (Fig. 5a). These results suggest that somatic gain predicted super-enhancers may be involved in traits associated with aggressive GC. When compared against predicted super-enhancer profiles of 86 cell and tissue samples, >60% of somatic gain predicted super-enhancers in GC exhibited high tissue-specificity. Significant overlaps (P < 0.001, empirical test) with predicted super-enhancers previously described in other cancer types, such as colorectal, breast, cervical and pancreatic cancer (Fig. 21) were also observed, suggesting that certain GC-associated predicted super-enhancers may also be active in other cancer types.

The study next asked if gene expression patterns associated with the somatic gain predicted super-enhancers might be associated with GC patient survival. Genes associated with the top 50 predicted super-enhancers, from regions exhibiting both recurrent somatic gains of in multiple GC patients, and also exhibiting the highest correlations with target gene expression were selected. Supporting the validity of this approach, several genes selected in this manner were observed to have been previously shown to be overexpressed in GC, such as *CDH17* and *CCAT1.* The gene list also included potentially novel GC associated genes, such as *SMURF1* and *LINC00299* (Table 10).

**Table 10: Genes associated with top predicted super-enhancers (with asterisk). Genes that were used in evaluating patient survival were indicated as "Yes".**

| **Genes associated with top N super-enhancers** | **N=60** | **N=50** | **N=30** |
|---|---|---|---|
| *ABTB2* | Yes * | Yes * | Yes * |
| *AC104654.2* | * | | |
| *AGFG2* | * | * | * |
| *AP000344.3* | * | | |
| *ATP2C2* | * | * | * |
| *ATP6V1C2* | * | * | |
| *BAIAP2L1* | Yes* | Yes * | |
| *BFSP2* | * | * | |
| *BX470102.3* | * | | |
| *CAMK2N1* | Yes* | Yes* | |
| *CCAT1* | * | * | * |
| *CCDC88C* | * | * | |
| *CDH17* | Yes * | Yes * | Yes * |
| *CDKN2B* | Yes* | | |
| *CLDN1* | Yes * | Yes * | Yes * |
| *CLRN3* | Yes * | Yes * | Yes * |
| *CREB3L1* | Yes * | Yes * | Yes * |
| *DSG2* | Yes * | Yes * | |
| *EGFR* | Yes * | Yes * | Yes * |
| *EPHB2* | Yes * | Yes * | Yes * |
| *ETV4* | Yes * | Yes * | Yes * |
| *GDA* | Yes * | Yes * | Yes * |
| *GDPD5* | Yes * | Yes * | |
| *GLS* | Yes * | Yes * | Yes * |
| *HRH1* | * | * | * |
| *IGFL4* | * | * | |
| *IL22RA1* | * | * | |
| *ITPK1* | Yes * | Yes * | Yes * |
| *KB-1471A8.1* | * | * | * |
| *KIAA1211* | * | * | |
| *LAMC2* | Yes* | | |
| *LINC00299* | * | * | * |
| *MALL* | Yes* | | |
| *MMP20* | * | * | |
| *MYO16-AS1* | * | * | * |
| *NOTCH1* | Yes* | | |
| *PCSK5* | * | | |
| *POP1* | * | * | * |
| *PFKP* | Yes* | | |
| *RARRES1* | Yes * | Yes * | Yes * |
| *RBCK1* | Yes * | Yes * | Yes * |
| *RNF170* | Yes * | Yes * | * |
| *RP11-400N13.2* | * | * | * |
| *RP11-486A 14.1* | * | | |
| *SLC9A4* | * | * | |
| *SMURF1* | Yes * | Yes * | Yes * |
| *SOX13* | Yes * | Yes * | |
| *SSTR5* | * | * | * |
| *ST3GAL4* | Yes * | Yes * | |
| *TASP1* | Yes * | Yes * | Yes * |
| *TPCN2* | Yes * | Yes * | Yes * |
| *TPRXL* | * | * | * |
| *TTYH3* | Yes* | Yes* | |
| *UPP1* | Yes * | Yes * | |
| *VIPR1* | Yes * | Yes * | Yes * |
| *ZKSCAN1* | * | * | |
| *ZNRF3* | * | * | * |

Survival analysis was performed across 3 non-Asian GC and 4 Asian GC cohorts comprising of 848 GC patients. Patients with GCs exhibiting high expression of predicted super-enhancer associated genes showed poor overall survival compared with GC samples where these genes are relatively lowly expressed (Fig. 5b, P = 1.8×10⁻², log rank test). Supporting the robustness of this association, the relationship with patient survival remained significant even after varying the number of predicted super-enhancers (n=30, P = 0.02, log rank test; n=60, P=0.03, log rank test). In a multivariate analysis, the association with survival also remained statistically significant even after adjusting for other risk factors, such as age, stage, patient locality and histological subtype (P = 0.044, Wald test). This data indicates that genes driven by somatic gain predicted super-enhancers in GC may be clinically important.

To address the relationship between the different predicted super-enhancer categories and disease risk, previous genome-wide association studies (GWAS) studies showing that disease-associated single-nucleotide polymorphisms (SNPs) are enriched at regulatory elements were considered. The study mapped catalogues of disease-associated SNPs reported from 1,470 genome-wide association studies against those predicted super-enhancers exhibiting recurrent somatic alterations (gained or lost) or unaltered predicted super-enhancers. Somatic predicted super-enhancers were enriched for disease-risk SNPs associated with various cancers (prostate, colorectal, breast; enrichment ratio=3.0-7.2; P<4.4x10⁻³, chi-square test) and gastrointestinal diseases such as ulcerative colitis (enrichment ratio=3.3; P=5.2 ×10⁻⁴, chi-square test) (Fig. 5c). In contrast, unaltered predicted super-enhancers did not exhibit similar enrichments. Unexpectedly, we also observed enrichment of multiple sclerosis SNPs in somatic altered predicted super-enhancers (enrichment ratio = 4.3; P=1.8 ×10⁻⁷, chi-square test), suggestive of interconnections between cancer and autoimmune response. To explore if predicted super-enhancer disease SNPs might be associated with local changes in chromatin modification, the study then focused on SNPs associated with colorectal cancer reported in at least two studies and also exhibiting heterozygosity in at least 1/3 of the GC patients (see Discussion). Two SNPs fulfilled these criteria (rs10411210 and rs10505477). Samples with the rs10411210 SNP exhibited significantly higher H3K27ac signals in tumors versus matched normals (Fig. 5d; P = 0.01, one-sided Welch's t-test), and a similar trend was also observed in samples with the rs10505477 SNP (P=0.07, one-sided Welch's t-test). Such associations suggest a relationship between disease-associated risk SNPs and cancer-associated histone modification.

### Super Enhancers Exhibit Dense Transcription Factor Occupancy

Finally, *trans*-acting factors associated with somatic gain predicted super-enhancers were explored. GC predicted super-enhancers exhibited significantly enriched ENCODE TF binding profiles compared to other genomic regions, supporting the former as TF "hot-spots" (P<2.2 ×10⁻¹⁶, one-sided proportion test). Interrogating the ReMap database, the study then identified specific TFs associated with the different predicted super-enhancer categories. Both somatic gain and unaltered predicted super-enhancers exhibited enrichments in CEBPB, MYC, and FOXA1 binding. However, among the top 10 enriched TFs, CDX2 exhibited elevated enrichment in somatic gain predicted super-enhancers (rank #2), with an approximately 30% increased binding density compared to unaltered predicted super-enhancers (rank #8) (Fig. 6a and 6b).

As TFs often act in a cooperative manner, potential CDX2 partners were identified by using HOMER, a *de novo* motif discovery algorithm. HOMER analysis identified HNF4α, KLF5, and GATA4 binding motifs associated with CDX2 binding (Fig. 6c). The study also analysed CDX2 co-binding motifs using PScanChIP with JASPAR 2016. Using PScanChIP, the study predicted 367 proteins as potential CDX2 partners, once again including HNF4α, KLF5 and GATA4 (Table 7). Gene co-expression analysis revealed that *HNF4α* (Spearman correlation, r = 0.80) and *KLF5* (r = 0.58) are the most strongly correlated candidates with *CDX2* expression, suggesting that HNF4α and KLF5 may be likely CDX2 partners (Fig. 6d). Notably, CDX2 has been previously identified in GC as a driver of intestinal metaplasia, and KLF5 and GATA4/6 have been previously reported as oncogenic transcription factors in GC that cooperate to upregulate *HNF4a.*

To experimentally confirm genomic co-occupancy of CDX2 with HNF4α (the highest correlated factor), CDX2 and HNF4α ChIP-seq was performed on OCUM-1 gastric cells, and integrated the TF binding locations with predicted super-enhancer locations. In OCUM-1 cells, CDX2 and HNF4α binding summits (q<0.01, MACS2) exhibited high co-occurrence (500bp window), with 76% of CDX2 binding co-occurring with HNF4α (known as CDX2/HNF4α sites) (Fig. 6e). Comparing the top 50% of high CDX2-expressing GCs against the lowest 50%, we found that in the former samples, recurrent somatic gain predicted super-enhancers were indeed associated with higher CDX2 binding densities (123 bindings per million base pair, Mbp vs 92 Mbp; see Methods). CDX2/HNF4α sites were preferentially localized to somatic gain predicted super-enhancers relative to unaltered predicted super-enhancers (P = 2.4×10⁻⁴, chi-square test), and both CDX2 and HNF4α binding signals were increased at somatic gain predicted super-enhancers relative to unaltered predicted super-enhancers (Fig. 6f). Similar CDX2 and HNF4α ChIP-seq results were also obtained in SNU16 cells (Fig. 22a). This result indicates that somatic gain predicted super-enhancers in GC are associated with CDX2 and HNF4α occupancy.

To test if CDX2 and HNF4α might play a role in GC super-enhancer maintenance, silencing of each TF was performed, either singly or both factors simultaneously, followed by genome-wide H3K27ac profiling. Depletion of either factor, either individually or in combination, did not induce global changes in H3K27ac in OCUM-1 cells (Fig. 22b). However, *CDX2* and *HNF4α*-silencing led to specific H3K27ac alterations in 9.7Mb and 4.3Mb of the genome respectively, and double-TF knockdown induced significantly greater H3K27ac depletion (P=3.4×10⁻²⁹ and 1.2×10⁻⁸⁸ compared to *CDX2* and *HNF4α*-alone, one-sided Wilcoxon rank sum test) (Fig. 22c). For both single-TF and double-TF silencing, H3K27ac depletion occurred more prominently at somatic gain predicted super-enhancers compared to predicted typical enhancers, suggesting a heightened sensitivity of super-enhancer activity to TF depletion (Fig. 6g, Fig. 2d, Tables 11a to 11d; P=5.3×10⁻⁷; P=1.8×10⁻¹⁷;P=1.5×10⁻¹⁰ for *CDX2, HNF4α* and *CDX2*/*HNF4α* respectively , one-sided Wilcoxon rank sum test). Supporting the specificity of these effects, H3K27ac depletion at predicted super-enhancers was more pronounced at regions centered at CDX2 or HNF4α binding sites, particularly at sites co-occupied by both factors (Fig. 6h). Similar results were also obtained in SNU16 cells (Fig. 22e). Next, to assess relationships between predicted super-enhancers and gene expression, the study focused on predicted super-enhancers exhibiting H3K27ac depletion after TF silencing. It was observed that >60% of predicted super-enhancer target genes also exhibited reduced expression after TF silencing (si*CDX2*, P = 4×10⁻⁴ , empirical test; si*HNF4α,* P < 1×10⁻⁴ , empirical test; si(*CDX2*/*HNF4α*)*,* P < 1×10⁻⁴ , empirical test; Fig. 22f). This proportion significantly exceeded that expected by chance, as assessed by permutation analysis (Methods). Taken collectively, these results support a functional requirement for CDX2 and HNF4α in GC super-enhancer maintenance.

**Table 11a: Somatic gain predicted super-enhancers in OCUM1. a. Enrichment significance of sub-regions with depleted H3K27ac signals in predicted super-enhancers after CDX2 silencing.**

| **contig** | **start** | **stop** | **pvalues** | **adjusted pvalue (Benjamini-Hochberg)** |
|---|---|---|---|---|
| chr14 | 54,306,200 | 54,324,100 | 8.52E-148 | 6.10E-145 |
| chrX | 132,807,600 | 132,814,350 | 2.37E-146 | 8.48E-144 |
| chr8 | 128,189,200 | 128,325,350 | 1.34E-113 | 2.40E-111 |
| chr12 | 71,536,600 | 71,613,350 | 3.92E-109 | 5.61E-107 |
| chr8 | 128,402,500 | 128,419,900 | 2.28E-104 | 2.72E-102 |
| chr1 | 178,000,350 | 178,031,500 | 2.55E-100 | 2.61E-98 |
| chr18 | 28,776,200 | 28,859,750 | 3.75E-100 | 3.35E-98 |
| chr15 | 67,858,900 | 67,866,600 | 5.06E-82 | 4.02E-80 |
| chr7 | 20,377,550 | 20,396,900 | 1.83E-78 | 1.31E-76 |
| chr10 | 114,267,000 | 114,300,350 | 5.93E-75 | 3.86E-73 |
| chr3 | 146,219,350 | 146,232,300 | 5.77E-68 | 3.44E-66 |
| chr3 | 42,216,150 | 42,228,600 | 1.41E-62 | 7.75E-61 |
| chrX | 15,488,650 | 15,499,600 | 5.50E-60 | 2.81E-58 |
| chr13 | 110,237,400 | 110,247,950 | 7.18E-58 | 3.43E-56 |
| chr11 | 95,826,500 | 95,849,500 | 2.39E-54 | 1.07E-52 |
| chr19 | 42,233,300 | 42,251,850 | 4.99E-53 | 2.10E-51 |
| chr10 | 90,654,700 | 90,658,150 | 9.75E-52 | 3.88E-50 |
| chr10 | 4,702,550 | 4,779,800 | 2.39E-45 | 9.01E-44 |
| chr13 | 80,789,100 | 80,814,050 | 5.92E-43 | 2.02E-41 |
| chr15 | 60,650,550 | 60,673,750 | 5.73E-43 | 2.02E-41 |
| chr3 | 41,197,000 | 41,203,450 | 1.11E-41 | 3.61E-40 |
| chr17 | 56,446,050 | 56,474,250 | 3.25E-41 | 1.01E-39 |
| chr7 | 39,619,650 | 39,646,100 | 7.01E-40 | 2.09E-38 |
| chr9 | 71,342,150 | 71,367,650 | 3.00E-38 | 8.60E-37 |
| chr3 | 149,054,800 | 149,145,650 | 4.60E-38 | 1.27E-36 |
| chr11 | 67,862,850 | 67,878,700 | 2.67E-37 | 7.08E-36 |
| chr14 | 56,266,200 | 56,333,200 | 2.35E-35 | 6.02E-34 |
| chr2 | 135,148,200 | 135,172,450 | 4.42E-35 | 1.09E-33 |
| chr18 | 29,928,900 | 29,966,450 | 5.09E-35 | 1.21E-33 |
| chr4 | 74,868,300 | 74,900,100 | 1.88E-34 | 4.33E-33 |
| chr18 | 19,854,800 | 19,883,350 | 3.17E-34 | 7.10E-33 |
| chr10 | 97,877,700 | 97,886,350 | 2.22E-32 | 4.81E-31 |
| chr12 | 76,146,700 | 76,178,850 | 7.60E-32 | 1.60E-30 |
| chr12 | 76,102,000 | 76,111,200 | 1.88E-31 | 3.85E-30 |
| chr7 | 95,036,450 | 95,072,000 | 2.54E-31 | 5.05E-30 |
| chr10 | 105,868,350 | 105,879,200 | 2.73E-30 | 5.29E-29 |
| chr6 | 135,569,550 | 135,594,500 | 6.17E-29 | 1.16E-27 |
| chr11 | 102,463,500 | 102,484,550 | 7.21E-29 | 1.32E-27 |
| chr4 | 69,595,200 | 69,617,700 | 1.40E-28 | 2.50E-27 |
| chr7 | 47,814,250 | 47,829,750 | 2.00E-28 | 3.49E-27 |
| chr13 | 106,790,700 | 106,804,950 | 1.86E-27 | 3.17E-26 |
| chr8 | 75,161,350 | 75,208,350 | 2.26E-27 | 3.76E-26 |
| chr13 | 80,339,100 | 80,361,300 | 6.01E-27 | 9.77E-26 |
| chr3 | 153,448,250 | 153,455,100 | 2.54E-26 | 4.04E-25 |
| chr7 | 47,229,450 | 47,235,650 | 9.72E-26 | 1.51E-24 |
| chr2 | 169,041,050 | 169,079,800 | 1.77E-24 | 2.69E-23 |
| chr1 | 113,546,250 | 113,565,450 | 2.09E-24 | 3.11E-23 |
| chr20 | 10,518,500 | 10,603,250 | 3.07E-24 | 4.49E-23 |
| chr18 | 33,034,800 | 33,064,850 | 3.47E-24 | 4.97E-23 |
| chr15 | 58,715,850 | 58,762,950 | 1.80E-23 | 2.52E-22 |

**Table 11b: Enrichment significance of sub-regions with depleted H3K27ac signals in predicted super-enhancers after HNF4α silencing.**

| **contig** | **start** | **stop** | **pvalues** | **adjusted pvalue (Benjamini-Hochberg)** |
|---|---|---|---|---|
| chr8 | 122,730,100 | 122,764,650 | 5.32E-103 | 3.81E-100 |
| chr8 | 128,189,200 | 128,325,350 | 6.37E-80 | 2.28E-77 |
| chr14 | 54,306,200 | 54,324,100 | 4.31E-70 | 1.03E-67 |
| chr7 | 25,004,000 | 25,014,900 | 1.82E-66 | 3.26E-64 |
| chr11 | 89,337,050 | 89,342,700 | 1.19E-62 | 1.71E-60 |
| chr14 | 32,404,150 | 32,421,350 | 6.33E-55 | 7.56E-53 |
| chr2 | 160,926,650 | 160,935,400 | 8.73E-55 | 8.93E-53 |
| chr13 | 43,611,750 | 43,640,300 | 1.27E-52 | 1.14E-50 |
| chr14 | 65,535,250 | 65,542,000 | 2.03E-51 | 1.62E-49 |
| chr7 | 27,713,900 | 27,721,650 | 3.08E-51 | 2.20E-49 |
| chr13 | 110,237,400 | 110,247,950 | 7.68E-51 | 5.00E-49 |
| chr13 | 73,735,300 | 73,753,550 | 2.32E-49 | 1.39E-47 |
| chr8 | 18,984,200 | 19,002,750 | 6.30E-49 | 3.47E-47 |
| chr4 | 139,110,850 | 139,130,300 | 7.45E-48 | 3.81E-46 |
| chr13 | 95,737,350 | 95,810,400 | 9.67E-47 | 4.61E-45 |
| chrX | 46,169,350 | 46,194,300 | 1.51E-43 | 6.76E-42 |
| chr14 | 54,911,250 | 54,917,100 | 1.68E-43 | 7.08E-42 |
| chr8 | 126,648,100 | 126,715,050 | 1.27E-41 | 5.07E-40 |
| chr13 | 110,487,450 | 110,494,450 | 1.83E-40 | 6.90E-39 |
| chr14 | 56,266,200 | 56,333,200 | 2.42E-38 | 8.66E-37 |
| chr13 | 47,154,350 | 47,197,300 | 2.83E-37 | 9.64E-36 |
| chr13 | 73,880,450 | 73,918,700 | 4.48E-37 | 1.46E-35 |
| chr1 | 202,424,000 | 202,428,750 | 3.06E-34 | 9.52E-33 |
| chr12 | 47,479,050 | 47,499,650 | 4.73E-34 | 1.41E-32 |
| chr1 | 225,625,350 | 225,643,650 | 1.87E-33 | 5.36E-32 |
| chrX | 151,146,550 | 151,166,200 | 1.53E-32 | 4.22E-31 |
| chr13 | 21,119,400 | 21,137,600 | 7.03E-32 | 1.86E-30 |
| chr7 | 95,036,450 | 95,072,000 | 3.51E-31 | 8.98E-30 |
| chr11 | 128,290,000 | 128,296,850 | 4.20E-31 | 1.04E-29 |
| chr18 | 33,034,800 | 33,064,850 | 4.65E-31 | 1.07E-29 |
| chr7 | 47,814,250 | 47,829,750 | 4.56E-31 | 1.07E-29 |
| chr13 | 73,931,800 | 73,950,750 | 1.21E-30 | 2.72E-29 |
| chr4 | 75,393,650 | 75,426,350 | 9.80E-29 | 2.13E-27 |
| chr13 | 80,558,550 | 80,565,950 | 1.27E-28 | 2.67E-27 |
| chr10 | 97,877,700 | 97,886,350 | 3.30E-28 | 6.76E-27 |
| chrX | 122,934,750 | 122,987,250 | 4.67E-28 | 9.29E-27 |
| chr4 | 79,480,750 | 79,492,700 | 6.27E-27 | 1.21E-25 |
| chr2 | 103,272,000 | 103,286,900 | 7.09E-27 | 1.34E-25 |
| chr7 | 138,549,200 | 138,586,800 | 4.93E-26 | 9.06E-25 |
| chr8 | 29,577,500 | 29,588,500 | 2.57E-25 | 4.61E-24 |
| chr18 | 68,023,400 | 68,049,250 | 1.25E-24 | 2.19E-23 |
| chr13 | 76,267,850 | 76,301,900 | 1.77E-24 | 3.03E-23 |
| chr18 | 28,776,200 | 28,859,750 | 3.82E-24 | 6.36E-23 |
| chrX | 105,948,700 | 105,963,900 | 6.47E-24 | 1.05E-22 |
| chr8 | 116,779,400 | 116,793,600 | 1.27E-23 | 2.02E-22 |
| chr11 | 35,019,250 | 35,080,300 | 3.80E-23 | 5.92E-22 |
| chr3 | 149,054,800 | 149,145,650 | 5.00E-23 | 7.62E-22 |
| chr13 | 80,789,100 | 80,814,050 | 5.38E-23 | 8.02E-22 |
| chr5 | 112,351,650 | 112,360,050 | 6.59E-23 | 9.64E-22 |
| chr9 | 21,673,800 | 21,690,700 | 7.18E-23 | 1.03E-21 |

**Table 11c: Enrichment significance of sub-regions with depleted H3K27ac signals in predicted super-enhancers after silencing CDX2 and HNF4α simultaneously.**

| **contig** | **start** | **stop** | **pvalues** | **adjusted pvalue (Benjamini-Hochberg)** |
|---|---|---|---|---|
| chr15 | 67,858,900 | 67,866,600 | 1.42E-99 | 1.01E-96 |
| chr14 | 54,306,200 | 54,324,100 | 6.33E-88 | 2.26E-85 |
| chr2 | 169,041,050 | 169,079,800 | 1.75E-77 | 4.17E-75 |
| chr13 | 110,237,400 | 110,247,950 | 1.90E-70 | 3.40E-68 |
| chr6 | 135,569,550 | 135,594,500 | 2.57E-58 | 3.68E-56 |
| chr14 | 65,535,250 | 65,542,000 | 5.71E-56 | 5.84E-54 |
| chr7 | 48,177,150 | 48,189,350 | 3.64E-53 | 3.26E-51 |
| chr1 | 207,172,100 | 207,201,700 | 4.29E-53 | 3.41E-51 |
| chrX | 151,146,550 | 151,166,200 | 8.90E-49 | 6.37E-47 |
| chr8 | 128,189,200 | 128,325,350 | 1.22E-47 | 7.93E-46 |
| chr11 | 128,290,000 | 128,296,850 | 1.43E-47 | 8.52E-46 |
| chr14 | 93,506,200 | 93,523,700 | 2.18E-47 | 1.20E-45 |
| chr12 | 94,945,600 | 94,966,900 | 8.62E-46 | 4.41E-44 |
| chr2 | 103,272,000 | 103,286,900 | 3.06E-43 | 1.46E-41 |
| chr1 | 178,000,350 | 178,031,500 | 4.40E-41 | 1.97E-39 |
| chr12 | 71,536,600 | 71,613,350 | 3.10E-40 | 1.31E-38 |
| chr13 | 106,790,700 | 106,804,950 | 6.32E-39 | 2.51E-37 |
| chr1 | 172,311,850 | 172,332,600 | 6.76E-38 | 2.55E-36 |
| chr18 | 33,034,800 | 33,064,850 | 1.93E-37 | 6.90E-36 |
| chr2 | 160,926,650 | 160,935,400 | 7.64E-37 | 2.61E-35 |
| chr18 | 20,245,750 | 20,293,550 | 1.42E-35 | 4.62E-34 |
| chr14 | 56,266,200 | 56,333,200 | 7.23E-35 | 2.25E-33 |
| chr8 | 19,516,550 | 19,525,800 | 3.45E-33 | 1.03E-31 |
| chr13 | 80,558,550 | 80,565,950 | 8.26E-33 | 2.37E-31 |
| chr2 | 69,532,450 | 69,539,250 | 9.64E-33 | 2.65E-31 |
| chr15 | 74,815,000 | 74,825,100 | 1.19E-32 | 3.16E-31 |
| chr14 | 54,911,250 | 54,917,100 | 2.75E-32 | 7.04E-31 |
| chr2 | 106,192,000 | 106,202,700 | 1.03E-31 | 2.55E-30 |
| chr3 | 41,197,000 | 41,203,450 | 1.03E-30 | 2.47E-29 |
| chr12 | 53,354,350 | 53,395,250 | 1.61E-30 | 3.72E-29 |
| chr6 | 47,368,650 | 47,390,500 | 2.43E-29 | 5.44E-28 |
| chr15 | 60,650,550 | 60,673,750 | 2.68E-29 | 5.81E-28 |
| chr3 | 153,448,250 | 153,455,100 | 4.31E-29 | 9.08E-28 |
| chr13 | 76,267,850 | 76,301,900 | 4.93E-28 | 9.80E-27 |
| chr18 | 29,928,900 | 29,966,450 | 7.45E-28 | 1.44E-26 |
| chr7 | 39,619,650 | 39,646,100 | 1.15E-27 | 2.11E-26 |
| chr8 | 95,200,950 | 95,225,750 | 1.12E-27 | 2.11E-26 |
| chr6 | 137,279,250 | 137,292,400 | 2.04E-27 | 3.65E-26 |
| chr7 | 20,377,550 | 20,396,900 | 2.22E-27 | 3.88E-26 |
| chr11 | 86,166,500 | 86,238,150 | 1.27E-25 | 2.16E-24 |
| chr9 | 71,342,150 | 71,367,650 | 1.93E-25 | 3.21E-24 |
| chr3 | 189,617,400 | 189,667,950 | 4.01E-24 | 6.53E-23 |
| chr7 | 55,186,000 | 55,204,050 | 1.73E-23 | 2.75E-22 |
| chr7 | 27,713,900 | 27,721,650 | 1.93E-23 | 3.00E-22 |
| chr3 | 42,216,150 | 42,228,600 | 1.08E-22 | 1.64E-21 |
| chr15 | 66,192,250 | 66,199,550 | 1.99E-22 | 2.97E-21 |
| chr4 | 139,110,850 | 139,130,300 | 2.45E-22 | 3.58E-21 |
| chr4 | 79,480,750 | 79,492,700 | 3.72E-22 | 5.33E-21 |
| chr19 | 42,233,300 | 42,251,850 | 4.22E-22 | 5.92E-21 |
| chr8 | 122,730,100 | 122,764,650 | 1.00E-21 | 1.38E-20 |

**Table 11d: Fold changes of CDX2 and HNF4α after silencing either factor or both simultaneously in OCUM1 cells. Negative values indicate that expression in cells with the silenced TF(s) is lower compared to the control.**

| | | **siCDX2** | **Conditions siHNF4a** | **siBOTH** |
|---|---|---|---|---|
| **Transcription factor** | **CDX2** | -7.1 | 1.3 | -2.5 |
| | **HNF4a** | 1.2 | -4.1 | -4.5 |

### Lineage specific enhancer elements in cancer

As proof of concept that some enhancer sub-regions may display cancer-specific essentiality, this study has tested the extent to which a *CLDN4* sub-enhancer region (el) can be deleted in either GC cells or normal ES cells (Fig 15, 16; Table 12). As shown in Figure 24, homozygous deletion of the *CLDN4* e1 enhancer subregion was readily achieved in H1 ES cells (Fig. 26, 27), but not in SNU16 GC cells (Fig. 25, 28), suggesting that retention of 1 copy of *CLDN4* e1 may be essential for SNU16 cancer cell survival.

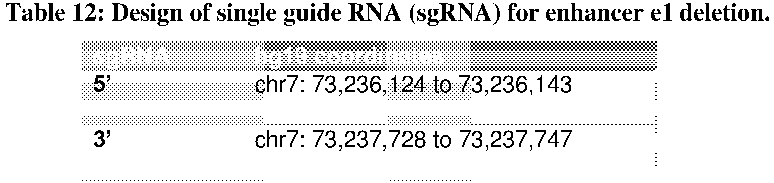

This allows the deletion around chr7:73,262,400-73,266,700. The size of deletion varies during actual experiment. Therefore, the aforementioned deleted region is just an estimate based on the sgRNA design. The sequences of the sgRNA are shown in Table 13.

**Table 13: Sequences of sgRNA**

| | |
|---|---|
| 5' sgRNA | CGGGACTCAGACCTTAGTCATGG (SEQ ID NO:141) |
| 3'sgRNA | GAGGATTTCTTAAGCCCAGAAGG (SEQ ID NO:142) |

### Correlation between gene expression and distal predicted regulatory elements

To correlate distal predicted regulatory elements defined by Nano-ChIPseq to gene expression, 80 predicted super-enhancers exhibiting high recurrence across multiple lines were identified (P < 0.0001, empirical test). The same approach was also used to identify highly recurrent predicted typical enhancers. For both predicted super-enhancers and predicted typical enhancers, genes associated with distal regulatory elements exhibited higher expression that randomly selected genes (Fig. 10b). Comparing the expression of predicted super-enhancer/typical enhancer associated genes revealed higher overall expression levels (in unit of percentile) for predicted super-enhancer associated genes (P = 5.2×10-3, one-sided Wilcoxon's rank sum test). These results suggest a positive association between H3K27ac enrichment in predicted super-enhancers and predicted typical enhancers with target gene expression.

### Comparisons of primary gastric non-malignant samples to Epigenome Roadmap

To confirm that the non-malignant gastric tissues in this study are indeed reflective of gastric epithelia and not muscle, immune cells etc, the non-malignant gastric H3K27ac profiles from this study were compared to previously published normal gastric profiles and also to stomach smooth muscle profiles. For each Nano-ChIPseq profile, 70% (average) of the H3K27ac signals overlapped with published normal gastric profiles, while only 34% (average) overlapped with stomach smooth muscle. The result suggests that the non-malignant gastric samples are indeed reflective of gastric epithelia and not stomach smooth muscle.

### Associations between copy number alterations and predicted super-enhancers in gastric cancer

The study investigated the extent to which recurrent somatic altered predicted super-enhancers might be associated with somatic copy number alterations (sCNAs). Overlaps between the predicted super-enhancers and copy number information from the cell lines and primary GCs were computed using in-house generated Affymetrix SNP6.0 array data. The analysis was restricted to regions covered by at least 6 SNP probes per 10 kb (2x higher than the mean genome-wide coverage), to allow regions of sCNA to be confidently identified. Confirming the reliability of the sCNA analysis, an average of 98% of copy number gains and 82% of copy number losses in the analysis were also reported in Cancer Cell Line Encyclopedia for GC cell lines found in the latter (FU97, KATO-III, MKN7, OCUM-1, RERFGC1B, SNU16).

In the cell lines, it was found that only 5-6% (± 6% standard deviation) of the predicted super-enhancers were associated with copy number gains (average log2 ratio > 0.6). For example, an *FGFR2*-associated predicted super-enhancer detected in KATO-III overlapped with copy number gain (Fig. 14b), suggesting that the observed higher H3K27ac read density at the locus is potentially driven by regional genomic amplification. On the other hand, the majority of the predicted super-enhancers detected in GC cell lines localized at copy number neutral regions, suggesting that the establishment of predicted super-enhancers is independent of somatic copy number events. This fraction is greater than by random chance (P< 0.01, empirical test).

Similarly, in primary GCs, this study was able to compute CNA/SE correlations for 1,748 recurrent somatic gain predicted super-enhancers in 19 primary T/N pairs. It was found that a only small fraction of somatic gain predicted super-enhancers (< 2% ± 3% s.d) overlapped with copy number gains (Fig. 14c), with >90% of somatic gain predicted super-enhancers found in individual T/N pairs are detected within copy number neutral regions (Fig. 14a). This result suggests that there is no strong association between somatic gain of H3K27ac in predicted super-enhancers and copy number changes, and that H3K27ac acquisition at predicted super-enhancers in the tumor samples are likely driven by mechanisms separate from copy number alteration.

### Discussion

GC is a clinically heterogeneous disease, and besides surgery and chemotherapy, only traztuzumab (anti-HER2) and ramucirumab (anti-VEGFR2) are approved clinically with other molecularly targeted agents proving unsuccessful to date. Epigenomic deregulation has emerged as an important pathway in gastric tumorigenesis, with chromatin modifier genes (eg *ARID1A*) being frequently mutated in GC and epigenetic alterations associated with gastric pre-malignancy. To date however, the vast majority of GC epigenomic studies have focused on promoter DNA methylation in the context of tumor suppressor gene silencing. In contrast, very little is currently known about distal regulatory elements (i.e. enhancers) in GC.

This study analyzed >35k predicted enhancer elements identified through micro-scale histone modification profiling of primary gastric tumors, matched non-malignant tissues, and GC cell lines. Small-scale ChIP protocols are known to be technically challenging and may sometimes result in significant between-sample variability. Reassuringly, the authors have previously demonstrated that Nano-ChIP signals between tumors and normal samples exhibit a good concordance with orthogonal ChIP-qPCR results and in the present study the authors have also performed extensive quality control analyses, including variations in mapping stringency, biological replicate analysis, promoter ChIP enrichment and CHANCE analysis, to confirm that the vast majority (85-100%) of Nano-ChIPseq libraries are of acceptable quality. The focus on recurrent epigenomic alterations present in multiple samples further ensured that the biological conclusions are likely to be robust, as shown by the observation that 84% of the recurrent somatic gain predicted super-enhancers were still rediscovered when analysis was confined to only those "high-quality" tumor/normal pairs passing both promoter-based and CHANCE quality analysis.

In this study, recurrent predicted super-enhancers largely manifested at known oncogenes and genes participating in oncogenic processes (Fig. 2d). High levels of enhancer variation between individual samples, exceeding proximal promoter elements (Fig. 1d) was also observed. When compared against other tissues and tumor-types, almost 60% of GC predicted super-enhancers were tissue-specific (Fig. 21). It is worth noting that in the current study, GCs was studied as a general category against matched non-malignant gastric tissues for maximal sensitivity. However distinct histopathological and molecular GC subtypes exist which suggest that there may exist distinct enhancer alterations in different histological subtypes of GC. Such findings reflect the exquisite tissue-specific nature of enhancer elements, and the consequent need for generating comprehensive enhancer catalogs in expanded patient cohorts and in many different tumor types.

The majority of samples analysed in the study were primary tissues derived directly from patients, rather than *in vitro* cultured cell lines. By comparing predicted enhancer activities (H3K27ac) between tumors and matched normals, it was possible to further sub-classify cell line predicted super-enhancers according to their somatic alteration status (somatic gain, somatic loss and unaltered). Supporting their biological distinctiveness, the subcategorized predicted super-enhancers also displayed specific differences in other orthogonal features, including epigenomic patterns (H3K4me1, DNA methylation), gene transcription, and cancer hallmarks. Notably, in our data only a small fraction of somatic gain predicted super-enhancers localized to regions of copy number amplification. The ability to sub-classify predicted super-enhancers according to *bona-fide* somatic gain or loss is likely to improve downstream attempts to pinpoint oncogenic mechanisms responsible for establishing super-enhancers in cancer. Such approaches are also possibly extendable to other disease states.

*A priori* consideration of predicted super-enhancer heterogeneity may also prove useful when analysing germline variants associated with disease risk. While previous findings have reported that disease-associated SNPs are generally over-represented in regulatory elements, it was found that somatic altered, but not unaltered predicted super-enhancers, were specifically enriched in SNPs associated with cancer and inflammatory gastrointestinal disease (a known risk factor for gastrointestinal cancer). SNPs in these regions may alter disease risk and cancer development through several non-exclusive mechanisms, including modification of TF binding motifs, regulation of long-range chromatin interactions, or alteration of H3K27ac levels. Indeed, in this study, it was observed that two SNPs associated with colorectal cancer (CRC) risk (rs10505477 and rs10411210) were also associated with local changes in chromatin modification in primary GCs. There are several reasons why it may be plausible to integrate CRC risk data with GC. First, at least one of these CRC risk SNPs (rs10505477) has been reported to also influence GC clinical outcome in both treatment response and patient survival. Second, the key transcription factors associated with the GC predicted super-enhancers (*CDX2, HNF4α)* are also known to regulate colonic development. Third, the role of intestinal metaplasia (IM) as a pre-malignant risk factor for GC is well-established, and in IM the gastric epithelial cells adopt a cellular architecture and appearance similar to colonic epithelium. The observation that these genetic variants, while present in germline DNA, may influence chromatin structure and gene expression in the tumor, has also been observed in CRC. These results further highlight the importance of studying aberrant epigenetic states to refine our understanding of germline processes underlying disease predisposition.

The results of the study suggest certain general principles regarding how individual super-enhancers in GC might interact with the *cis-* and trans-acting transcriptional machinery. Using two distinct long-range chromatin interaction assays (Capture-C and 4C), several examples of somatic gain predicted super-enhancers engaging both proximal and distal genes exhibiting elevated tumor expression were observed. It has been proposed that genes linked to somatic gain predicted super-enhancers are likely to occupy similar topological associating domains, established through cohesin-mediated enhancer-promoter loops. The ability of somatic gain predicted super-enhancers to influence both proximal and distal gene expression implicates predicted super-enhancers as pivotal regulators of aberrant gene expression in gastric tumors, which can contribute to disease progression and chemoresponse (Fig. 5b). At the *trans-* level, the data revealed that somatic gain predicted super-enhancers in GC are associated with CDX2 and HNF4α occupancy. Previous studies have shown that aberrant CDX2 expression in the stomach is associated with intestinal metaplasia of the mucosal epithelial cells, an important early event in gastric tumor formation and that CDX2 has the potential to function as a GC oncogene. HNF4α has also been recently implicated in GC, as a target of both the lineage-specific oncogenes KLF5 and GATA factors, and the AMPK signaling pathway. The results in primary human tumors are supported by recent findings in the mouse small intestine, where CDX2 has been found to regulate HNF4α occupancy to control intestinal gene expression. Echoing these studies, it was also found that CDX2/HNF4α depletion effected chromatin alterations at local regions concentrated at CDX2 and/or HNF4α binding sites.

In conclusion, this study demonstrates a role for heterogeneity in predicted super-enhancers and the utility of intersecting chromatin profiles from primary tissues and cell lines to dissect regulatory biology. This first-generation roadmap of GC distal enhancers now renders possible future integrative studies involving transcriptional features associated with GC predicted enhancers (eRNAs), and identifying somatic regulatory mutations perturbing predicted super-enhancer activity.

## Claims

1. A method for determining the presence or absence of at least one cancer-associated super-enhancer in a primary cancerous biological sample obtained from a subject relative to a matched primary non-cancerous biological sample, wherein the primary cancerous biological sample is gastric cancer, comprising;
a) contacting the primary cancerous biological sample obtained from the subject with at least one antibody specific for histone modification H3K27ac;
b) isolating nucleic acid from the primary cancerous biological sample, wherein the isolated nucleic acid comprises at least one region specific to the histone modification H3K27ac, wherein the nucleic acid is isolated from the cancerous biological sample by immunoprecipitation of chromatin specific to the histone modification H3K27ac;
c) mapping at least one enhancer using an annotated genome sequence based on a signal intensity of the histone modification H3K27ac, wherein the at least one enhancer is at least 2.5 kb from an annotated transcription start site;
d) mapping the at least one enhancer in the isolated nucleic acid against at least one enhancer in at least one reference nucleic acid sequence to identify at least one cancer-associated super-enhancer in the primary cancerous biological sample;
e) comparing the signal intensity of the at least one cancer-associated super-enhancer in the primary cancerous biological sample against a reference signal intensity of the at least one cancer-associated super-enhancer obtained from the matched primary non-cancerous biological sample, wherein the signal intensity of the at least one cancer-associated super-enhancer is based on the Reads Per Kilobase of transcript per Million (RPKM) value of the histone modification H3K27ac; and
f) determining the presence or absence of the at least one cancer-associated super-enhancer in the primary cancerous biological sample based on the change in signal intensity of the at least one cancer-associated super-enhancer, comprising determining that the RPKM value for the at least one cancer-associated super-enhancer in the primary cancerous biological sample is:
i) greater than a 2-fold change in RPKM value relative to the RPKM value of the at least one cancer-associated super-enhancer obtained from the matched primary non-cancerous biological sample; and
ii) an absolute difference greater than a 0.5 RPKM relative to the RPKM value of the at least one cancer-associated super-enhancer obtained from the matched primary non-cancerous biological sample.

2. The method of claim 1, wherein the primary cancerous and matched primary non-cancerous biological sample comprises a single cell, multiple cells, fragments of cells, body fluid or tissue; optionally
wherein the primary cancerous and matched primary non-cancerous biological sample is obtained from the same subject; optionally
wherein the cancerous and non-cancerous biological sample are each obtained from different subjects; optionally
wherein the nucleic acid is isolated from said primary cancerous biological sample by immunoprecipitation of chromatin.

3. The method of claims 1 or 2, wherein the signal intensity of the at least one super-enhancer is based on the Reads Per Kilobase of transcript per Million (RPKM) value of the histone modification H3K27ac; optionally
wherein the at least one reference nucleic acid sequence are obtained from at least one cancer cell line; optionally
wherein the at least one super-enhancer in the primary cancerous biological sample is identified using the ROSE (Ranking of Super Enhancer) algorithm; optionally
wherein the at least one super-enhancer in the primary cancerous biological sample comprises at least one nucleic acid base pair overlapping with the at least one enhancer in the at least one reference nucleic acid sequence.

4. The method of claim 3,
wherein an increase in RPKM value from the primary cancerous biological sample relative to the RPKM value of the matched primary non-cancerous biological sample is indicative of the presence of the at least one super-enhancer in the primary cancerous biological sample; optionally wherein a decrease in RPKM value from the primary cancerous biological sample relative to the RPKM value of the matched primary non-cancerous biological sample is indicative of the absence of the at least one super-enhancer in the primary cancerous biological sample.

5. The method of any one of claims 1-4, wherein the at least one super-enhancer is positioned within 1000kb to a gene transcription start site; optionally
wherein the gene is one or more of a cancer associated gene, an angiogenesis gene, a cell proliferation gene, a cell invasion gene, a gene associated with genome instability, a cell death resistance gene, a cellular energetics gene, a cell cycle gene or a tumour-promoting gene; optionally
wherein the gene is selected from the group consisting of CLDN4, ABHD11, WBSCR28, ATAD2, KLH38, WDYHV1, CDH17, CCAT1, CLDN1, SMURF1, GDPD5, ADAMTS12, ASCL2, ASPM, ATP11A, AURKA, CAMK2N1, CBX2, CCNE1, CD9, CDC25B, CDCA7, CDK1, CXCL1, E2F7, ECT2, LAMC2, NID2, PMEPA1, RARRES1, RFC3, SLC39A10, TFAP2A, TMEM158, LINC00299 and a combination thereof.

6. The method of claim 1,
wherein an increased signal intensity of the at least one super-enhancer in the primary cancerous biological sample relative to the matched primary non-cancerous biological sample is indicative of the presence of at least one cancer-associated super-enhancer.

7. The method of claim 6,
wherein the presence or absence of at least one cancer-associated super-enhancer is indicative of the prognosis of the cancer in the subject; optionally
wherein the presence of the at least one cancer-associated super-enhancer in the primary cancerous biological sample is indicative of a poor prognosis of cancer survival in a subject; optionally
wherein the absence of the at least one cancer-associated super-enhancer in the primary cancerous biological sample is indicative of an improved prognosis of cancer survival in a subject; optionally
wherein the at least one cancer-associated super-enhancer is associated with one or more of a cell invasion gene, an angiogenesis gene or a cell death resistance gene, a cancer associated gene, a cell proliferation gene, a gene associated with genome instability, a cellular energetics gene, a cell cycle gene or a tumour-promoting gene; optionally
wherein the at least one cancer-associated super-enhancer is associated with a gene selected from the group consisting of CLDN4, ABHD11, WBSCR28, ATAD2, KLH38, WDYHV1, CDH17, CCAT1, CLDN1, SMURF1, GDPD5, ADAMTS12, ASCL2, ASPM, ATP11A, AURKA, CAMK2N1, CBX2, CCNE1, CD9, CDC25B, CDCA7, CDK1, CXCL1, E2F7, ECT2, LAMC2, NID2, PMEPA1, RARRES1, RFC3, SLC39A10, TFAP2A, TMEM158, LINC00299 and a combination thereof.

8. The method of claim 6, wherein the subject has been administered an inhibitor of CDX2 and HNF4α to modulate the activity of the least one cancer-associated super-enhancer; optionally
wherein the inhibitor of CDX2 and HNF4α is a siRNA; optionally
wherein the inhibitor is metformin.

9. Use of an inhibitor of CDX2 and HNF4α in modulating in vitro the activity of at least one cancer-associated super-enhancer in a cell, wherein the at least one cancer-associated super-enhancer is detected by the method of claim 6.

10. The method of claim 1,
wherein an increased signal intensity of the at least one super-enhancer in the primary cancerous biological sample relative to the matched primary non-cancerous biological sample is predictive of cancer cell survival or cancer cell viability.

## Patentansprüche

1. Verfahren zum Bestimmen der Gegenwart oder Abwesenheit von zumindest einem krebsassoziierten Super-Enhancer in einer primären kanzerösen biologischen Probe, die von einem Individuum erhalten wurde, im Vergleich zu einer abgestimmten primären nichtkanzerösen biologischen Probe, wobei die primäre kanzeröse biologische Probe Magenkrebs ist, umfassend:
a) Inkontaktbringen der primären kanzerösen biologischen Probe, die von dem Individuum erhalten wurde, mit zumindest einem Antikörper, der für die Histonmodifikation H3K27ac spezifisch ist;
b) Isolieren von Nucleinsäure aus der primären kanzerösen biologischen Probe, wobei die isolierte Nucleinsäure zumindest eine Region umfasst, die für die Histonmodifikation H3K27ac spezifisch ist, wobei die Nucleinsäure durch Immunpräzipitation von Chromatin, das spezifisch für die Histonmodifikation H3K27ac ist, aus der kanzerösen biologischen Probe isoliert wird;
c) Kartieren des zumindest einen Enhancers unter Verwendung einer annotierten Genomsequenz basierend auf der Signalintensität der Histonmodifikation H3K27ac, wobei sich der zumindest eine Enhancer zumindest 2,5 kb von einer annotierten Transkriptionsstartstelle entfernt befindet;
d) Kartieren des zumindest einen Enhancers in der isolierten Nucleinsäure gegenüber zumindest einem Enhancer in zumindest einer Bezugsnucleinsäuresequenz, um zumindest einen krebsassoziierten Super-Enhancer in der primären kanzerösen biologischen Probe zu identifizieren;
e) Vergleichen der Signalintensität des zumindest einen krebsassoziierten Super-Enhancers in der primären kanzerösen biologischen Probe mit einer Bezugssignalintensität des zumindest einen krebsassoziierten Super-Enhancers, die von der abgestimmten primären nichtkanzerösen biologischen Probe erhalten wurde, wobei die Signalintensität des zumindest einen krebsassoziierten Super-Enhancers auf dem Wert der Ablesungen pro Kilobase Transkript pro Million (RPKM) der Histonmodifikation H3K27ac basiert; und
f) Bestimmen der Gegenwart oder Abwesenheit des zumindest einen krebsassoziierten Super-Enhancers in der primären kanzerösen biologischen Probe basierend auf der Veränderung der Signalintensität des zumindest einen krebsassoziierten Super-Enhancers, was das Bestimmen umfasst, dass der RPKM-Wert für den zumindest einen krebsassoziierten Super-Enhancer in der primären kanzerösen Probe:
i) mehr als eine 2-fache Änderung des RPKM-Werts bezogen auf den RPKM-Wert des zumindest einen krebsassoziierten Super-Enhancers ist, der von der abgestimmten primären nichtkanzerösen biologischen Probe erhalten wurde; und
ii) eine absolute Differenz von mehr als 0,5 RPKM bezogen auf den RPKM-Wert des zumindest einen krebsassoziierten Super-Enhancers aufweist, der von der abgestimmten primären nichtkanzerösen biologischen Probe erhalten wurde.

2. Verfahren nach Anspruch 1, wobei die primäre kanzeröse und abgestimmte primäre nichtkanzeröse biologische Probe eine einzelne Zelle, mehrere Zellen, Fragmente von Zellen, Körperflüssigkeit oder Gewebe umfasst;
wobei gegebenenfalls die primäre kanzeröse und abgestimmte primäre nichtkanzeröse biologische Probe vom selben Individuum erhalten werden;
wobei gegebenenfalls die kanzeröse und nichtkanzeröse biologische Probe jeweils von unterschiedlichen Individuen erhalten werden;
wobei gegebenenfalls die Nucleinsäure durch Immunpräzipitation von Chromatin aus der primären kanzerösen biologischen Probe isoliert wird.

3. Verfahren nach Anspruch 1 oder 2, wobei die Signalintensität des zumindest einen Super-Enhancers auf dem Wert der Ablesungen pro Kilobase Transkript pro Million (RPKM) der Histonmodifikation H3K27ac basiert;
wobei gegebenenfalls die zumindest eine Bezugsnucleinsäuresequenz von zumindest einer Krebszelllinie erhalten wird;
wobei gegebenenfalls der zumindest eine Super-Enhancer in der primären kanzerösen biologischen Probe unter Verwendung eines ROSE-Algorithmus (Ranking of Super Enhancer) identifiziert wird;
wobei gegebenenfalls der zumindest eine Super-Enhancer in der primären kanzerösen biologischen Probe zumindest ein Nucleinsäure-Basenpaar umfasst, das mit dem zumindest einen Enhancer in der zumindest einen Bezugsnucleinsäuresequenz überlappt.

4. Verfahren nach Anspruch 3, wobei eine Erhöhung des RPKM-Werts von der primären kanzerösen biologischen Probe in Bezug auf den RPKM-Wert der abgestimmten primären nichtkanzerösen biologischen Probe die Gegenwart von zumindest einem Super-Enhancer in der primären kanzerösen biologischen Probe anzeigt; wobei gegebenenfalls eine Verringerung des RPKM-Werts von der primären kanzerösen biologischen Probe in Bezug auf den RPKM-Wert der abgestimmten primären nichtkanzerösen biologischen Probe die Abwesenheit des zumindest einen Super-Enhancers in der primären kanzerösen biologischen Probe anzeigt.

5. Verfahren nach einem der Ansprüche 1-4, wobei der zumindest eine Super-Enhancer innerhalb von 1000 kb zu einer Gentranskriptionsstartstelle positioniert ist;
wobei das Gen gegebenenfalls eines oder mehrere aus einem krebsassoziierten Gen, einem Angiogenesegen, einem Zellproliferationsgen, einem Zellinvasionsgen, einem mit Genominstabilität assoziierten Gen, einem Zelltodresistenzgen, einem Gen der zellulären Energetik, einem Zellzyklusgen oder einem Tumor-fördernden Gen ist;
wobei gegebenenfalls das Gen aus der aus CLDN4, ABHD11, WBSCR28, ATAD2, KLH38, WDYHV1, CDH17, CCAT1, CLDN1, SMURF1, GDPD5, ADAMTS12, ASCL2, ASPM, ATP11A, AURKA, CAMK2N1, CBX2, CCNE1, CD9, CDC25B, CDCA7, CDK1, CXCL1, E2F7, ECT2, LAMC2, NID2, PMEPA1, RARRES 1, RFC3, SLC39A10, TFAP2A, TMEM158, LINC00299 und einer Kombination davon bestehenden Gruppe ausgewählt ist.

6. Verfahren nach Anspruch 1, wobei eine erhöhte Signalintensität des zumindest einen Super-Enhancers in der primären kanzerösen biologischen Probe in Bezug auf die abgestimmte primäre nichtkanzeröse biologische Probe die Gegenwart zumindest eines krebsassoziierten Super-Enhancers anzeigt.

7. Verfahren nach Anspruch 6,
wobei die Gegenwart oder Abwesenheit des zumindest einen krebsassoziierten Super-Enhancers die Prognose für den Krebs im Individuum anzeigt;
wobei gegebenenfalls die Gegenwart des zumindest einen krebsassoziierten Super-Enhancers in der primären kanzerösen biologischen Probe eine schlechte Prognose für das Überleben des Krebses durch das Individuum anzeigt;
wobei gegebenenfalls die Abwesenheit des zumindest einen krebsassoziierten Super-Enhancers in der primären kanzerösen biologischen Probe eine verbesserte Prognose für das Überleben des Krebses durch das Individuum anzeigt;
wobei gegebenenfalls der zumindest eine krebsassoziierte Super-Enhancer mit einem oder mehreren aus einem Zellinvasionsgen, einem Angiogenesegen oder einem Zelltodresistenzgen, einem krebsassoziierten Gen, einem Zellproliferationsgen, einem mit Genominstabilität assoziierten Gen, einem Gen der zellulären Energetik, einem Zellzyklusgen oder einem Tumor-fördernden Gen assoziiert ist;
wobei gegebenenfalls der zumindest eine krebsassoziierte Super-Enhancer mit einem Gen assoziiert ist, das aus der aus CLDN4, ABHD11, WBSCR28, ATAD2, KLH38, WDYHV1, CDH17, CCAT1, CLDN1, SMURF1, GDPD5, ADAMTS12, ASCL2, ASPM, ATP11A, AURKA, CAMK2N1, CBX2, CCNE1, CD9, CDC25B, CDCA7, CDK1, CXCL1, E2F7, ECT2, LAMC2, NID2, PMEPA1, RARRES 1, RFC3, SLC39A10, TFAP2A, TMEM158, LINC00299 und einer Kombination davon bestehenden Gruppe ausgewählt ist.

8. Verfahren nach Anspruch 6, wobei dem Individuum ein Inhibitor von CDX2 und HNF4α verabreicht wurde, um die Aktivität des zumindest einen krebsassoziierten Super-Enhancers zu modulieren;
wobei gegebenenfalls der Inhibitor von CDX2 und HNF4α eine siRNA ist;
wobei der Inhibitor gegebenenfalls Metformin ist.

9. Verwendung eines Inhibitors von CDX2 und HNF4α bei der In-vitro-Modulation zumindest eines krebsassoziierten Super-Enhancers in einer Zelle, wobei der zumindest eine krebsassoziierte Super-Enhancer durch ein Verfahren nach Anspruch 6 detektiert wird.

10. Verfahren nach Anspruch 1, wobei eine erhöhte Signalintensität des zumindest einen Super-Enhancers in der primären kanzerösen biologischen Probe in Bezug auf die abgestimmte primäre nichtkanzeröse biologische Probe das Krebszellüberleben oder die Krebszelllebensfähigkeit vorhersagt.

## Revendications

1. Procédé pour déterminer la présence ou l'absence d'au moins un super amplificateur associé au cancer dans un échantillon biologique cancéreux primaire obtenu auprès d'un sujet par rapport à un échantillon biologique non cancéreux primaire correspondant, dans lequel l'échantillon biologique cancéreux primaire est un cancer gastrique, comprenant les étapes consistant à :
a) mettre en contact l'échantillon biologique cancéreux primaire obtenu auprès du sujet avec au moins un anticorps spécifique pour une modification d'histone H3K27ac ;
b) isoler un acide nucléique à partir de l'échantillon biologique cancéreux primaire, dans lequel l'acide nucléique isolé comprend au moins une région spécifique à la modification d'histone H3K27ac, dans lequel l'acide nucléique est isolé de l'échantillon biologique cancéreux par immunoprécipitation de chromatine spécifique à la modification d'histone H3K27ac ;
c) cartographier au moins un amplificateur en utilisant une séquence de génome annotée sur la base d'une intensité de signal de la modification d'histone H3K27ac, dans lequel le au moins un amplificateur est au moins 2,5 kb à partir d'un site de début de transcription annotée ;
d) mettre en correspondance le au moins un amplificateur dans l'acide nucléique isolé par rapport à au moins un amplificateur dans au moins une séquence d'acide nucléique de référence pour identifier au moins un super amplificateur associé au cancer dans l'échantillon biologique cancéreux primaire ;
e) comparer l'intensité de signal du au moins un super amplificateur associé au cancer dans l'échantillon biologique cancéreux primaire à une intensité de signal de référence du au moins un super amplificateur associé au cancer obtenu à partir de l'échantillon biologique non cancéreux primaire correspondant, dans lequel l'intensité de signal du au moins un super amplificateur associé au cancer est basé sur la valeur de lectures par kilobase de transcription par million (RPKM) de la modification d'histone H3K27ac ; et
f) déterminer la présence ou l'absence du au moins un super amplificateur associé au cancer dans l'échantillon biologique cancéreux primaire sur la base du changement d'intensité de signal du au moins un super amplificateur associé au cancer, comprenant une détermination que la valeur RPKM pour le au moins un super amplificateur associé au cancer dans l'échantillon biologique cancéreux primaire est :
i) supérieure à deux fois un changement de la valeur RPKM par rapport à la valeur RPKM du au moins un super amplificateur associé au cancer obtenu à partir de l'échantillon biologique primaire non cancéreux correspondant ; et
ii) une différence absolue supérieure à 0,5 RPKM par rapport à la valeur RPKM du au moins un super amplificateur associé au cancer obtenu à partir de l'échantillon biologique primaire non cancéreux correspondant.

2. Procédé selon la revendication 1, dans lequel l'échantillon biologique cancéreux primaire et non cancéreux primaire correspondant comprend une cellule unique, des cellules multiples, des fragments de cellules, un fluide corporel ou un tissu ; facultativement
dans lequel l'échantillon biologique cancéreux primaire et non cancéreux primaire correspondant est obtenu à partir du même sujet ; facultativement
dans lequel les échantillons biologiques cancéreux et non cancéreux sont chacun obtenus auprès de différents sujets ; facultativement
dans lequel l'acide nucléique est isolé à partir dudit échantillon biologique cancéreux primaire par immunoprécipitation de chromatine.

3. Procédé selon les revendications 1 ou 2, dans lequel l'intensité de signal du au moins un super amplificateur est basée sur la valeur de lectures par kilobase de transcription par million (RPKM) de la modification d'histone H3K27ac ; facultativement
dans lequel la au moins une séquence d'acide nucléique de référence est obtenue à partir d'au moins une lignée cellulaire cancéreuse ; facultativement
dans lequel le au moins un super amplificateur dans l'échantillon biologique cancéreux primaire est identifié en utilisant l'acronyme ROSE (classement de super amplificateur) ; facultativement
dans lequel le au moins un super amplificateur dans l'échantillon biologique cancéreux primaire comprend au moins une paire de bases d'acide nucléique chevauchant le au moins un amplificateur dans la au moins une séquence d'acide nucléique de référence.

4. Procédé selon la revendication 3,
dans lequel une augmentation de la valeur RPKM à partir de l'échantillon biologique cancéreux primaire par rapport à la valeur RPKM de l'échantillon biologique non cancéreux primaire correspondant indique la présence du au moins un super amplificateur dans l'échantillon biologique cancéreux primaire ; facultativement dans lequel une diminution de la valeur RPKM à partir de l'échantillon biologique cancéreux primaire par rapport à la valeur RPKM de l'échantillon biologique non cancéreux primaire correspondant indique l'absence du au moins un super amplificateur dans l'échantillon biologique cancéreux primaire.

5. Procédé selon l'une quelconque des revendications 1 à 4, dans lequel le au moins un super amplificateur est positionné à l'intérieur de 1 000 kb sur un site de début de transcription de gène ; facultativement
dans lequel le gène est un ou plusieurs parmi un gène associé au cancer, un gène d'angiogenèse, un gène de prolifération cellulaire, un gène d'invasion cellulaire, un gène associé à l'instabilité génomique, un gène de résistance à la mort cellulaire, un gène énergétique cellulaire, un gène de cycle cellulaire ou un gène promoteur de tumeur ; facultativement
dans lequel le gène est sélectionné dans le groupe comprenant CLDN4, ABHD11, WBSCR28, ATAD2, KLH38, WDYHV1, CDH17, CCAT1, CLDN1, SMURF1, GDPD5, ADAMTS12, ASCL2, ASPM, ATP11A, AURKA, CAMK2N1, CBX2, CCNE1, CD9, CDC25B, CDCA7, CDK1, CXCL1, E2F7, ECT2, LAMC2, NID2, PMEPA1, RARRES1, RFC3, SLC39A10, TFAP2A, TMEM158, LINC00299 et une combinaison de ceux-ci.

6. Procédé selon la revendication 1,
dans lequel une intensité de signal accrue de l'au moins un super amplificateur dans l'échantillon biologique cancéreux primaire par rapport à l'échantillon biologique non cancéreux primaire correspondant indique la présence d'au moins un super amplificateur associé au cancer.

7. Procédé selon la revendication 6,
dans lequel la présence ou l'absence d'au moins un super amplificateur associé au cancer indique le pronostic du cancer chez le sujet ; facultativement
dans lequel la présence d'au moins un super amplificateur associé au cancer dans l'échantillon biologique cancéreux primaire indique un mauvais pronostic de survie au cancer chez un sujet ; facultativement
dans lequel l'absence du au moins un super amplificateur associé au cancer dans l'échantillon biologique cancéreux primaire indique un pronostic amélioré de survie au cancer chez un sujet ; facultativement ;
dans lequel ledit au moins un super amplificateur associé au cancer est associé à un ou plusieurs parmi un gène d'invasion cellulaire, un gène d'angiogenèse ou un gène de résistance à la mort cellulaire, un gène associé au cancer, un gène de prolifération cellulaire, un gène associé à l'instabilité génomique, un gène énergétique cellulaire, un gène de cycle cellulaire ou un gène promoteur de tumeur ; facultativement
dans lequel ledit au moins un super amplificateur associé au cancer est associé à un gène choisi dans le groupe comprenant CLDN4, ABHD11, WBSCR28, ATAD2, KLH38, WDYHV1, CDH17, CCAT1, CLDN1, SMURF1, GDPD5, ADAMTS12, ASCL2, ASPM, ATP11A, AURKA, CAMK2N1, CBX2, CCNE1, CD9, CDC25B, CDCA7, CDK1, CXCL1, E2F7, ECT2, LAMC2, NID2, PMEPA1, RARRES1, RFC3, SLC39A10, TFAP2A, TMEM158, LINC00299 et une combinaison de ceux-ci.

8. Procédé selon la revendication 6, dans lequel le sujet s'est vu administrer un inhibiteur de CDX2 et de HNF4α pour moduler l'activité du au moins un super amplificateur associé au cancer ; facultativement
dans lequel l'inhibiteur de CDX2 et de HNF4α est un ARNsi ; facultativement
dans lequel l'inhibiteur est la metformine.

9. Utilisation d'un inhibiteur de CDX2 et de HNF4α dans la modulation in vitro de l'activité d'au moins un super amplificateur associé au cancer dans une cellule, dans laquelle le au moins un super amplificateur associé au cancer est détecté par le procédé selon la revendication 6.

10. Procédé selon la revendication 1,
dans lequel une intensité de signal accrue du au moins un super amplificateur dans l'échantillon biologique cancéreux primaire par rapport à l'échantillon biologique non cancéreux primaire correspondant est prédictif de la survie des cellules cancéreuses ou de la viabilité des cellules cancéreuses.
